# EUROPEAN PATENT APPLICATION

(11) **EP 1 247 864 A2**
(43) Date of publication of application: **09.10.2002**
(21) Application number: 02250331.2
(22) Date of filing: 17.01.2002
(51) Int. Cl.: C12N 15/12, C12N 15/63, C12N 15/62, C12N 1/21, C12N 5/10, C07K 14/47, C07K 16/18, G01N 33/68, G01N 33/53, C12Q 1/68

(54) **58297, an amino acid transporter and uses therefor**

(30) Priority: 18.01.2001 US 262515 P
(71) Applicant: MILLENIUM PHARMACEUTICALS, INC., Cambridge, MA 02139 (US)
(72) Inventor: Curtis, Rory A. J., Southborough, MA 01772 (US)
(74) Representative: Lunt, Mark George Francis

(57) **Abstract**

The invention provides isolated nucleic acids molecules, designated 58297 nucleic acid molecules, which encode amino acid transporter family members. The invention also provides antisense nucleic acid molecules, recombinant expression vectors containing 58297 nucleic acid molecules, host cells into which the expression vectors have been introduced, and nonhuman transgenic animals in which a 58297 gene has been introduced or disrupted. The invention still further provides isolated 58297 proteins, fusion proteins, antigenic peptides and anti-58297 antibodies. Diagnostic and therapeutic methods utilizing compositions of the invention are also provided.

SEQUENCE LISTING

<110> Curtis, Rory A.J.

<120> 58297, AN AMINO ACID TRANSPORTER AND
  USES THEREFOR

<130> P71868EP

<160> 8

<170> FastSEQ for Windows Version 4.0

<210> 1
<211> 2476
<212> DNA
<213> Homo sapiens

<220>
<221> 5'UTR
<222> (1)...(127)

<221> CDS
<222> (128)...(1810)

<221> 3'UTR
<222> (1811)...(2476)

<400> 1
cccacgcgtc cgagttctcc gaggttgaag gctcggcctg ctcagagaag gaaactgagg 60
tccaccgagt tggagaaacc tactcaacac caggactaac ttcttcagtg cttagagtgt 120
gagaaaa atg gca aat atg aat agt gat tct agg cat ctt ggc acc tct 169
        Met Ala Asn Met Asn Ser Asp Ser Arg His Leu Gly Thr Ser
         1               5                  10

gag gta gat cat gaa aga gat cct gga cct atg aat atc cag ttt gag 217
Glu Val Asp His Glu Arg Asp Pro Gly Pro Met Asn Ile Gln Phe Glu
 15                  20                  25                  30

cca tcg gat cta aga tcc aaa agg cct ttc tgt ata gag ccc aca aac 265
Pro Ser Asp Leu Arg Ser Lys Arg Pro Phe Cys Ile Glu Pro Thr Asn
                 35                  40                  45

atc gtg aat gtg aat cat gtc att cag agg gtt agt gac cat gcc tct 313
Ile Val Asn Val Asn His Val Ile Gln Arg Val Ser Asp His Ala Ser
             50                  55                  60

gcc atg aac aag aga att cat tac tac agc cgg ctc acc act cct gca 361
Ala Met Asn Lys Arg Ile His Tyr Tyr Ser Arg Leu Thr Thr Pro Ala
         65                  70                  75

gac aag gca ctg att gcc cca gac cat gta gtt cca gct cca gaa gag 409
Asp Lys Ala Leu Ile Ala Pro Asp His Val Val Pro Ala Pro Glu Glu
     80                  85                  90

tgc tat gtg tat agt cca ttg ggc tct gct tat aaa ctt caa agt tac 457

Cys Tyr Val Tyr Ser Pro Leu Gly Ser Ala Tyr Lys Leu Gln Ser Tyr
95                  100                 105                 110

act gaa gga tac ggt aaa aac acc agt tta gta acc att ttt atg att     505
Thr Glu Gly Tyr Gly Lys Asn Thr Ser Leu Val Thr Ile Phe Met Ile
                115                 120                 125

tgg aat acc atg atg gga aca tct ata cta agc att cct tgg ggc ata     553
Trp Asn Thr Met Met Gly Thr Ser Ile Leu Ser Ile Pro Trp Gly Ile
            130                 135                 140

aaa cag gct gga ttt act act gga atg tgt gtc atc ata ctg atg ggc     601
Lys Gln Ala Gly Phe Thr Thr Gly Met Cys Val Ile Ile Leu Met Gly
        145                 150                 155

ctt tta aca ctt tat tgc tgc tac aga gta gtg aaa tca cgg act atg     649
Leu Leu Thr Leu Tyr Cys Cys Tyr Arg Val Val Lys Ser Arg Thr Met
    160                 165                 170

atg ttt tca ttg gat acc act acc tgg gaa tat cca gat gtc tgc aga     697
Met Phe Ser Leu Asp Thr Thr Thr Trp Glu Tyr Pro Asp Val Cys Arg
175                 180                 185                 190

cat tat ttc ggc tcc ttt ggg cag tgg tcg agt ctc ctc ttc tcc ttg     745
His Tyr Phe Gly Ser Phe Gly Gln Trp Ser Ser Leu Leu Phe Ser Leu
                195                 200                 205

gtg tct ctc att gga gca atg ata gtt tat tgg gtg ctt atg tca aat     793
Val Ser Leu Ile Gly Ala Met Ile Val Tyr Trp Val Leu Met Ser Asn
            210                 215                 220

ttt ctt ttt aat act gga aag ttt att ttt aat ttt att cat cac att     841
Phe Leu Phe Asn Thr Gly Lys Phe Ile Phe Asn Phe Ile His His Ile
        225                 230                 235

aat gac aca gac act ata ctg agt acc aat aat agc aac cct gtg att     889
Asn Asp Thr Asp Thr Ile Leu Ser Thr Asn Asn Ser Asn Pro Val Ile
    240                 245                 250

tgt cca agt gcc ggg agt gga ggc cat cct gac aac agc tct atg att     937
Cys Pro Ser Ala Gly Ser Gly Gly His Pro Asp Asn Ser Ser Met Ile
255                 260                 265                 270

ttc tat gcc aat gac aca gga gcc caa cag ttt gaa aag tgg tgg gat     985
Phe Tyr Ala Asn Asp Thr Gly Ala Gln Gln Phe Glu Lys Trp Trp Asp
                275                 280                 285

aag tcc agg aca gtc ccc ttt tat ctt gta ggg ctc ctc ctc cca ctg     1033
Lys Ser Arg Thr Val Pro Phe Tyr Leu Val Gly Leu Leu Leu Pro Leu
            290                 295                 300

ctc aat ttc aag tct cct tca ttt ttt tca aaa ttt aat atc cta ggc     1081
Leu Asn Phe Lys Ser Pro Ser Phe Phe Ser Lys Phe Asn Ile Leu Gly
        305                 310                 315

aca gtg tct gtc ctt tat ttg att ttc ctt gtc acc ttt aag gct gtt     1129
Thr Val Ser Val Leu Tyr Leu Ile Phe Leu Val Thr Phe Lys Ala Val

320                 325                 330

cgc ttg gga ttt cat ttg gaa ttt cat tgg ttt ata cca aca gaa ttt    1177
Arg Leu Gly Phe His Leu Glu Phe His Trp Phe Ile Pro Thr Glu Phe
335                 340                 345                 350

ttt gta cca gag ata aga ttt cag ttt cca cag ctg act gga gtg ctt    1225
Phe Val Pro Glu Ile Arg Phe Gln Phe Pro Gln Leu Thr Gly Val Leu
                355                 360                 365

acc ctt gct ttt ttt att cat aat tgt atc atc aca ctc ttg aag aac    1273
Thr Leu Ala Phe Phe Ile His Asn Cys Ile Ile Thr Leu Leu Lys Asn
            370                 375                 380

aac aag aaa caa gaa aac aat gtg agg gac ttg tgc att gct tat atg    1321
Asn Lys Lys Gln Glu Asn Asn Val Arg Asp Leu Cys Ile Ala Tyr Met
        385                 390                 395

ctg gtg aca tta act tat ctc tat att gga gtc ctg gtt ttt gct tca    1369
Leu Val Thr Leu Thr Tyr Leu Tyr Ile Gly Val Leu Val Phe Ala Ser
    400                 405                 410

ttt cct tca cca cca tta tcc aaa gat tgt att gag cag aat ttt tta    1417
Phe Pro Ser Pro Pro Leu Ser Lys Asp Cys Ile Glu Gln Asn Phe Leu
415                 420                 425                 430

gac aac ttc cct agc agt gac acc ctg tcc ttc att gca agg ata ttc    1465
Asp Asn Phe Pro Ser Ser Asp Thr Leu Ser Phe Ile Ala Arg Ile Phe
                435                 440                 445

ctg ctg ttc cag atg atg act gta tac cca ctc tta ggc tac ctg gct    1513
Leu Leu Phe Gln Met Met Thr Val Tyr Pro Leu Leu Gly Tyr Leu Ala
            450                 455                 460

cgt gtc cag ctt ttg ggc cat atc ttc ggt gac att tat cct agc att    1561
Arg Val Gln Leu Leu Gly His Ile Phe Gly Asp Ile Tyr Pro Ser Ile
        465                 470                 475

ttc cat gtg ctg gtt ctt aat cta att att gtg gga gct gga gtg atc    1609
Phe His Val Leu Val Leu Asn Leu Ile Ile Val Gly Ala Gly Val Ile
    480                 485                 490

atg gcc tgt ttc tac cca aac ata gga ggg atc ata aga tat tca gga    1657
Met Ala Cys Phe Tyr Pro Asn Ile Gly Gly Ile Ile Arg Tyr Ser Gly
495                 500                 505                 510

gca gca tgt gga ctg gcc ttt gta ttc ata tac cca tct ctc atc tat    1705
Ala Ala Cys Gly Leu Ala Phe Val Phe Ile Tyr Pro Ser Leu Ile Tyr
                515                 520                 525

ata att tcc ctc cac caa gaa gag cgt ctg aca tgg cct aaa tta atc    1753
Ile Ile Ser Leu His Gln Glu Glu Arg Leu Thr Trp Pro Lys Leu Ile
            530                 535                 540

ttc cac gtt ttc atc atc att ttg ggc gtg gct aac ctg att gtt cag    1801
Phe His Val Phe Ile Ile Ile Leu Gly Val Ala Asn Leu Ile Val Gln
        545                 550                 555

ttt ttt atg tgaaatacct caactgtttt tttcaagagc tctcatgata 1850
Phe Phe Met
    560

ttttgagcct tgacaacagt tctatataaa ttcacttgta aatgctgctg ttgtgtaatt 1910
ctaaatattt tctaagataa tttgaaagca agggaaatag tggcccctta atgagtattt 1970
ttttattggg gtggggaaag gggcaaaaag aatgatctta gtgtcttaac ctttctcata 2030
ttaactcacc tctttattct gtggtctttt ctgaatagaa atgtatgccc taggaagaaa 2090
tcatgctggg ttttgctttt agagataaaa ggtggtggat ttattttgcc tgcagtaaag 2150
attctcaggg tgtcagagca gcatattgtc aaatcctgct tctgttttat gtttcagtgt 2210
attcactttc attttcttac ttactagacc atttctgcag tttgcccaaa cctctactgt 2270
ttgggacagt aagccaaata cctcattttt aaaaagaagt tttcatggca tcagtgttaa 2330
taaagtacat ttttaactga gtcttaatct ctatttgaag aaaaagtaga gacaaaagta 2390
atgtcaatgt aatccccagg atcatgaaat gtatacaaaa taaataaagt aggagagttt 2450
gttgctgaaa aaaaaaaaaa aaaaaa 2476

<210> 2
<211> 561
<212> PRT
<213> Homo sapiens

<400> 2
Met Ala Asn Met Asn Ser Asp Ser Arg His Leu Gly Thr Ser Glu Val
1               5                   10                  15
Asp His Glu Arg Asp Pro Gly Pro Met Asn Ile Gln Phe Glu Pro Ser
            20                  25                  30
Asp Leu Arg Ser Lys Arg Pro Phe Cys Ile Glu Pro Thr Asn Ile Val
        35                  40                  45
Asn Val Asn His Val Ile Gln Arg Val Ser Asp His Ala Ser Ala Met
    50                  55                  60
Asn Lys Arg Ile His Tyr Tyr Ser Arg Leu Thr Thr Pro Ala Asp Lys
65                  70                  75                  80
Ala Leu Ile Ala Pro Asp His Val Val Pro Ala Pro Glu Glu Cys Tyr
                85                  90                  95
Val Tyr Ser Pro Leu Gly Ser Ala Tyr Lys Leu Gln Ser Tyr Thr Glu
            100                 105                 110
Gly Tyr Gly Lys Asn Thr Ser Leu Val Thr Ile Phe Met Ile Trp Asn
        115                 120                 125
Thr Met Met Gly Thr Ser Ile Leu Ser Ile Pro Trp Gly Ile Lys Gln
    130                 135                 140
Ala Gly Phe Thr Thr Gly Met Cys Val Ile Ile Leu Met Gly Leu Leu
145                 150                 155                 160
Thr Leu Tyr Cys Cys Tyr Arg Val Val Lys Ser Arg Thr Met Met Phe
                165                 170                 175
Ser Leu Asp Thr Thr Thr Trp Glu Tyr Pro Asp Val Cys Arg His Tyr
            180                 185                 190
Phe Gly Ser Phe Gly Gln Trp Ser Ser Leu Leu Phe Ser Leu Val Ser
        195                 200                 205
Leu Ile Gly Ala Met Ile Val Tyr Trp Val Leu Met Ser Asn Phe Leu
    210                 215                 220
Phe Asn Thr Gly Lys Phe Ile Phe Asn Phe Ile His His Ile Asn Asp
225                 230                 235                 240
Thr Asp Thr Ile Leu Ser Thr Asn Asn Ser Asn Pro Val Ile Cys Pro
                245                 250                 255
Ser Ala Gly Ser Gly Gly His Pro Asp Asn Ser Ser Met Ile Phe Tyr
            260                 265                 270

Ala Asn Asp Thr Gly Ala Gln Gln Phe Glu Lys Trp Trp Asp Lys Ser
        275                 280                 285
Arg Thr Val Pro Phe Tyr Leu Val Gly Leu Leu Leu Pro Leu Leu Asn
    290                 295                 300
Phe Lys Ser Pro Ser Phe Phe Ser Lys Phe Asn Ile Leu Gly Thr Val
305                 310                 315                 320
Ser Val Leu Tyr Leu Ile Phe Leu Val Thr Phe Lys Ala Val Arg Leu
                325                 330                 335
Gly Phe His Leu Glu Phe His Trp Phe Ile Pro Thr Glu Phe Phe Val
            340                 345                 350
Pro Glu Ile Arg Phe Gln Phe Pro Gln Leu Thr Gly Val Leu Thr Leu
        355                 360                 365
Ala Phe Phe Ile His Asn Cys Ile Ile Thr Leu Leu Lys Asn Asn Lys
    370                 375                 380
Lys Gln Glu Asn Asn Val Arg Asp Leu Cys Ile Ala Tyr Met Leu Val
385                 390                 395                 400
Thr Leu Thr Tyr Leu Tyr Ile Gly Val Leu Val Phe Ala Ser Phe Pro
                405                 410                 415
Ser Pro Pro Leu Ser Lys Asp Cys Ile Glu Gln Asn Phe Leu Asp Asn
            420                 425                 430
Phe Pro Ser Ser Asp Thr Leu Ser Phe Ile Ala Arg Ile Phe Leu Leu
        435                 440                 445
Phe Gln Met Met Thr Val Tyr Pro Leu Leu Gly Tyr Leu Ala Arg Val
    450                 455                 460
Gln Leu Leu Gly His Ile Phe Gly Asp Ile Tyr Pro Ser Ile Phe His
465                 470                 475                 480
Val Leu Val Leu Asn Leu Ile Ile Val Gly Ala Gly Val Ile Met Ala
                485                 490                 495
Cys Phe Tyr Pro Asn Ile Gly Gly Ile Ile Arg Tyr Ser Gly Ala Ala
            500                 505                 510
Cys Gly Leu Ala Phe Val Phe Ile Tyr Pro Ser Leu Ile Tyr Ile Ile
        515                 520                 525
Ser Leu His Gln Glu Glu Arg Leu Thr Trp Pro Lys Leu Ile Phe His
    530                 535                 540
Val Phe Ile Ile Ile Leu Gly Val Ala Asn Leu Ile Val Gln Phe Phe
545                 550                 555                 560
Met

<210> 3
<211> 1683
<212> DNA
<213> Homo sapiens

<220>
<221> CDS
<222> (1)...(1683)

<400> 3
atg gca aat atg aat agt gat tct agg cat ctt ggc acc tct gag gta    48
Met Ala Asn Met Asn Ser Asp Ser Arg His Leu Gly Thr Ser Glu Val
 1               5                  10                  15

gat cat gaa aga gat cct gga cct atg aat atc cag ttt gag cca tcg    96
Asp His Glu Arg Asp Pro Gly Pro Met Asn Ile Gln Phe Glu Pro Ser
            20                  25                  30

gat cta aga tcc aaa agg cct ttc tgt ata gag ccc aca aac atc gtg     144
Asp Leu Arg Ser Lys Arg Pro Phe Cys Ile Glu Pro Thr Asn Ile Val
        35                  40                  45

aat gtg aat cat gtc att cag agg gtt agt gac cat gcc tct gcc atg     192
Asn Val Asn His Val Ile Gln Arg Val Ser Asp His Ala Ser Ala Met
    50                  55                  60

aac aag aga att cat tac tac agc cgg ctc acc act cct gca gac aag     240
Asn Lys Arg Ile His Tyr Tyr Ser Arg Leu Thr Thr Pro Ala Asp Lys
65                  70                  75                  80

gca ctg att gcc cca gac cat gta gtt cca gct cca gaa gag tgc tat     288
Ala Leu Ile Ala Pro Asp His Val Val Pro Ala Pro Glu Glu Cys Tyr
                85                  90                  95

gtg tat agt cca ttg ggc tct gct tat aaa ctt caa agt tac act gaa     336
Val Tyr Ser Pro Leu Gly Ser Ala Tyr Lys Leu Gln Ser Tyr Thr Glu
            100                 105                 110

gga tac ggt aaa aac acc agt tta gta acc att ttt atg att tgg aat     384
Gly Tyr Gly Lys Asn Thr Ser Leu Val Thr Ile Phe Met Ile Trp Asn
        115                 120                 125

acc atg atg gga aca tct ata cta agc att cct tgg ggc ata aaa cag     432
Thr Met Met Gly Thr Ser Ile Leu Ser Ile Pro Trp Gly Ile Lys Gln
    130                 135                 140

gct gga ttt act act gga atg tgt gtc atc ata ctg atg ggc ctt tta     480
Ala Gly Phe Thr Thr Gly Met Cys Val Ile Ile Leu Met Gly Leu Leu
145                 150                 155                 160

aca ctt tat tgc tgc tac aga gta gtg aaa tca cgg act atg atg ttt     528
Thr Leu Tyr Cys Cys Tyr Arg Val Val Lys Ser Arg Thr Met Met Phe
                165                 170                 175

tca ttg gat acc act acc tgg gaa tat cca gat gtc tgc aga cat tat     576
Ser Leu Asp Thr Thr Thr Trp Glu Tyr Pro Asp Val Cys Arg His Tyr
            180                 185                 190

ttc ggc tcc ttt ggg cag tgg tcg agt ctc ctc ttc tcc ttg gtg tct     624
Phe Gly Ser Phe Gly Gln Trp Ser Ser Leu Leu Phe Ser Leu Val Ser
        195                 200                 205

ctc att gga gca atg ata gtt tat tgg gtg ctt atg tca aat ttt ctt     672
Leu Ile Gly Ala Met Ile Val Tyr Trp Val Leu Met Ser Asn Phe Leu
    210                 215                 220

ttt aat act gga aag ttt att ttt aat ttt att cat cac att aat gac     720
Phe Asn Thr Gly Lys Phe Ile Phe Asn Phe Ile His His Ile Asn Asp
225                 230                 235                 240

aca gac act ata ctg agt acc aat aat agc aac cct gtg att tgt cca     768
Thr Asp Thr Ile Leu Ser Thr Asn Asn Ser Asn Pro Val Ile Cys Pro
                245                 250                 255

agt gcc ggg agt gga ggc cat cct gac aac agc tct atg att ttc tat 816
Ser Ala Gly Ser Gly Gly His Pro Asp Asn Ser Ser Met Ile Phe Tyr
            260                 265                 270

gcc aat gac aca gga gcc caa cag ttt gaa aag tgg tgg gat aag tcc 864
Ala Asn Asp Thr Gly Ala Gln Gln Phe Glu Lys Trp Trp Asp Lys Ser
        275                 280                 285

agg aca gtc ccc ttt tat ctt gta ggg ctc ctc ctc cca ctg ctc aat 912
Arg Thr Val Pro Phe Tyr Leu Val Gly Leu Leu Leu Pro Leu Leu Asn
    290                 295                 300

ttc aag tct cct tca ttt ttt tca aaa ttt aat atc cta ggc aca gtg 960
Phe Lys Ser Pro Ser Phe Phe Ser Lys Phe Asn Ile Leu Gly Thr Val
305                 310                 315                 320

tct gtc ctt tat ttg att ttc ctt gtc acc ttt aag gct gtt cgc ttg 1008
Ser Val Leu Tyr Leu Ile Phe Leu Val Thr Phe Lys Ala Val Arg Leu
                325                 330                 335

gga ttt cat ttg gaa ttt cat tgg ttt ata cca aca gaa ttt ttt gta 1056
Gly Phe His Leu Glu Phe His Trp Phe Ile Pro Thr Glu Phe Phe Val
            340                 345                 350

cca gag ata aga ttt cag ttt cca cag ctg act gga gtg ctt acc ctt 1104
Pro Glu Ile Arg Phe Gln Phe Pro Gln Leu Thr Gly Val Leu Thr Leu
        355                 360                 365

gct ttt ttt att cat aat tgt atc atc aca ctc ttg aag aac aac aag 1152
Ala Phe Phe Ile His Asn Cys Ile Ile Thr Leu Leu Lys Asn Asn Lys
    370                 375                 380

aaa caa gaa aac aat gtg agg gac ttg tgc att gct tat atg ctg gtg 1200
Lys Gln Glu Asn Asn Val Arg Asp Leu Cys Ile Ala Tyr Met Leu Val
385                 390                 395                 400

aca tta act tat ctc tat att gga gtc ctg gtt ttt gct tca ttt cct 1248
Thr Leu Thr Tyr Leu Tyr Ile Gly Val Leu Val Phe Ala Ser Phe Pro
                405                 410                 415

tca cca cca tta tcc aaa gat tgt att gag cag aat ttt tta gac aac 1296
Ser Pro Pro Leu Ser Lys Asp Cys Ile Glu Gln Asn Phe Leu Asp Asn
            420                 425                 430

ttc cct agc agt gac acc ctg tcc ttc att gca agg ata ttc ctg ctg 1344
Phe Pro Ser Ser Asp Thr Leu Ser Phe Ile Ala Arg Ile Phe Leu Leu
        435                 440                 445

ttc cag atg atg act gta tac cca ctc tta ggc tac ctg gct cgt gtc 1392
Phe Gln Met Met Thr Val Tyr Pro Leu Leu Gly Tyr Leu Ala Arg Val
    450                 455                 460

cag ctt ttg ggc cat atc ttc ggt gac att tat cct agc att ttc cat 1440
Gln Leu Leu Gly His Ile Phe Gly Asp Ile Tyr Pro Ser Ile Phe His
465                 470                 475                 480

gtg ctg gtt ctt aat cta att att gtg gga gct gga gtg atc atg gcc 1488

Val Leu Val Leu Asn Leu Ile Ile Val Gly Ala Gly Val Ile Met Ala
                485                 490                 495

tgt ttc tac cca aac ata gga ggg atc ata aga tat tca gga gca gca     1536
Cys Phe Tyr Pro Asn Ile Gly Gly Ile Ile Arg Tyr Ser Gly Ala Ala
            500                 505                 510

tgt gga ctg gcc ttt gta ttc ata tac cca tct ctc atc tat ata att     1584
Cys Gly Leu Ala Phe Val Phe Ile Tyr Pro Ser Leu Ile Tyr Ile Ile
        515                 520                 525

tcc ctc cac caa gaa gag cgt ctg aca tgg cct aaa tta atc ttc cac     1632
Ser Leu His Gln Glu Glu Arg Leu Thr Trp Pro Lys Leu Ile Phe His
    530                 535                 540

gtt ttc atc atc att ttg ggc gtg gct aac ctg att gtt cag ttt ttt     1680
Val Phe Ile Ile Ile Leu Gly Val Ala Asn Leu Ile Val Gln Phe Phe
545                 550                 555                 560

atg                                                                 1683
Met

<210> 4
<211> 467
<212> PRT
<213> Homo sapiens

<400> 4
Ala His Leu Arg Glu Val Met Gly Gly Asn Asn Ser Ser Val Phe Lys
1               5                   10                  15
Gln Leu Gly Trp Ile Pro Gly Leu Val Leu Leu Leu Leu Val Gly Phe
            20                  25                  30
Ile Thr Leu Tyr Gly His Phe Thr Gly Leu Leu Leu Val Lys Cys Tyr
        35                  40                  45
Glu Glu Glu Gly Glu Tyr Val Pro Gly Lys Arg Glu Gly Ala Lys Ser
    50                  55                  60
Tyr Leu Asp Leu Gly Arg Gln Ser Ala Tyr Gly Gly Lys Gly Leu Leu
65                  70                  75                  80
Leu Thr Ser Phe Val Gly Thr Gly Val Gln Tyr Val Asn Leu Phe Gly
                85                  90                  95
Val Asn Ile Gly Tyr Leu Ile Leu Ala Gly Asp Leu Leu Pro Lys Ile
            100                 105                 110
Ile Ser Ser Phe Leu Cys Phe Leu Leu Ile Gln Val Gly Asp Asn Val
        115                 120                 125
Gly Val Ser Gly Asp Ile Gly Cys Asp Leu Leu Ser Gly Asn Ser Trp
    130                 135                 140
Ile Ile Ile Phe Ala Ala Ile Ile Ile Thr Pro Leu Ser Phe Ile Pro
145                 150                 155                 160
Ala Phe Asn Leu Leu Ser Ala Ser Asn Leu Leu Ser Leu Ser Val Leu
                165                 170                 175
Ile Ile Ser Ser Leu Ser Ala Phe Ser Ser Leu Ala Tyr Ile Ser Ile
            180                 185                 190
Ile Ser Phe Leu Ile Val Val Ala Val Ile Ala Gly Ile Phe Val Leu
        195                 200                 205
Leu Gly Ala Val Tyr Lys Ile Leu Trp Ser Val Glu Thr Leu Ala Val

210                 215                 220
Thr Val Pro Ser Val Thr Lys Leu Thr Gly Leu Phe Leu Ala Ile Gly
225                 230                 235                 240
Ile Ile Val Phe Ala Phe Glu Gly His Ala Val Leu Leu Pro Ile Gln
                245                 250                 255
Asn Thr Met Lys Ser Pro Ser Glu Pro Glu Phe Lys Lys Phe Lys Lys
            260                 265                 270
Val Leu Asn Val Ala Ile Ile Ile Val Thr Val Leu Tyr Ile Leu Val
        275                 280                 285
Gly Phe Val Gly Tyr Leu Thr Phe Gly Asn Ser Val Lys Asp Asn Ile
    290                 295                 300
Leu Leu Asn Phe Lys Leu Leu Pro Asn Asn Pro Phe Trp Leu Ile Val
305                 310                 315                 320
Asn Leu Asn Leu Val Val Ala Ile Leu Leu Thr Phe Pro Leu Gln Cys
                325                 330                 335
His Pro Val Phe Ala Phe Pro Ile Val Arg Ile Leu Glu Asn Lys Leu
            340                 345                 350
Lys Arg Arg Phe Asp Asn Asn Lys Leu Leu Thr Lys Lys Asn Asn Phe
        355                 360                 365
Ala Pro Lys Asn Thr Gly Asp Pro Lys Ser Lys Leu Leu Arg Val Val
    370                 375                 380
Ile Arg Ser Gly Leu Val Val Val Thr Leu Leu Ile Ala Ile Leu Val
385                 390                 395                 400
Pro Phe Leu Gly Asp Leu Leu Ser Leu Val Gly Ala Thr Ser Gly Ala
                405                 410                 415
Pro Leu Thr Phe Ile Leu Pro Pro Leu Phe Glu Met Tyr Ile Leu Leu
            420                 425                 430
Lys Lys Glu Lys Lys Trp Gly Lys Gly Glu Lys Pro Lys Glu Val Leu
        435                 440                 445
Arg Arg Trp Pro Lys Leu Ile Asp Leu Ile Ile Ile Val Val Gly Leu
    450                 455                 460
Leu Leu Ser
465


<210> 5
<211> 505
<212> PRT
<213> Mus musculus

<400> 5
Met Glu Ile Pro Arg Gln Thr Glu Met Val Glu Leu Val Pro Asn Gly
1               5                   10                  15
Lys His Leu Glu Gly Leu Leu Pro Val Gly Val Pro Thr Thr Asp Thr
            20                  25                  30
Gln Arg Thr Glu Asp Thr Gln His Cys Gly Glu Gly Lys Gly Phe Leu
        35                  40                  45
Gln Lys Ser Pro Ser Lys Glu Pro His Phe Thr Asp Phe Glu Gly Lys
    50                  55                  60
Thr Ser Phe Gly Met Ser Val Phe Asn Leu Ser Asn Ala Ile Met Gly
65                  70                  75                  80
Ser Gly Ile Leu Gly Leu Ala Tyr Ala Met Ala Asn Thr Gly Ile Ile
                85                  90                  95
Leu Phe Leu Phe Leu Leu Thr Ala Val Ala Leu Leu Ser Ser Tyr Ser
            100                 105                 110
Ile His Leu Leu Leu Lys Ser Ser Gly Ile Val Gly Ile Arg Ala Tyr
        115                 120                 125

Glu Gln Leu Gly Tyr Arg Ala Phe Gly Thr Pro Gly Lys Leu Ala Ala
    130                 135                 140
Ala Leu Ala Ile Thr Leu Gln Asn Ile Gly Ala Met Ser Ser Tyr Leu
145                 150                 155                 160
Tyr Ile Ile Lys Ser Glu Leu Pro Leu Val Ile Gln Thr Phe Leu Asn
                165                 170                 175
Leu Glu Lys Pro Ala Ser Val Trp Tyr Met Asp Gly Asn Tyr Leu Val
            180                 185                 190
Ile Leu Val Ser Val Thr Ile Ile Leu Pro Leu Ala Leu Met Arg Gln
        195                 200                 205
Leu Gly Tyr Leu Gly Tyr Ser Ser Gly Phe Ser Leu Ser Cys Met Val
    210                 215                 220
Phe Phe Leu Ile Ala Val Ile Tyr Lys Lys Phe Gln Val Pro Cys Pro
225                 230                 235                 240
Leu Ala His Asn Leu Ala Asn Ala Thr Gly Asn Phe Ser His Met Val
                245                 250                 255
Val Ala Glu Glu Lys Ala Gln Leu Gln Gly Glu Pro Asp Ala Ala Ala
            260                 265                 270
Glu Ala Phe Cys Thr Pro Ser Tyr Phe Thr Leu Asn Ser Gln Thr Ala
        275                 280                 285
Tyr Thr Ile Pro Ile Met Ala Phe Ala Phe Val Cys His Pro Glu Val
    290                 295                 300
Leu Pro Ile Tyr Thr Glu Leu Lys Asp Pro Ser Lys Arg Lys Met Gln
305                 310                 315                 320
His Ile Ser Asn Leu Ser Ile Ala Val Met Tyr Val Met Tyr Phe Leu
                325                 330                 335
Ala Ala Leu Phe Gly Tyr Leu Thr Phe Tyr Asp Gly Val Glu Ser Glu
            340                 345                 350
Leu Leu His Thr Tyr Ser Lys Val Asp Pro Phe Asp Val Leu Ile Leu
        355                 360                 365
Cys Val Arg Val Ala Val Leu Ile Ala Val Thr Leu Thr Val Pro Ile
    370                 375                 380
Val Leu Phe Pro Val Arg Arg Ala Ile Gln Gln Met Leu Phe Gln Asn
385                 390                 395                 400
Gln Glu Phe Ser Trp Leu Arg His Val Leu Ile Ala Thr Gly Leu Leu
                405                 410                 415
Thr Cys Ile Asn Leu Leu Val Ile Phe Ala Pro Asn Ile Leu Gly Ile
            420                 425                 430
Phe Gly Ile Ile Gly Ala Thr Ser Ala Pro Cys Leu Ile Phe Ile Phe
        435                 440                 445
Pro Ala Ile Phe Tyr Phe Arg Ile Met Pro Thr Asp Lys Glu Pro Ala
    450                 455                 460
Arg Ser Thr Pro Lys Ile Leu Ala Leu Cys Phe Ala Ala Val Gly Phe
465                 470                 475                 480
Leu Leu Met Thr Met Ser Leu Ser Phe Ile Ile Ile Asp Trp Val Ser
                485                 490                 495
Gly Thr Ser Gln His Gly Gly Asn His
            500                 505

<210> 6
<211> 504
<212> PRT
<213> Rattus norvegicus

<400> 6
   Met Glu Ile Pro Arg Gln Thr Glu Met Val Glu Leu Val Pro Asn Gly
   1               5                   10                  15
   Lys His Leu Glu Gly Leu Leu Pro Val Gly Met Pro Thr Ala Asp Thr
   20                  25                  30
   Gln Arg Ala Glu Asp Ala Gln His Cys Gly Glu Gly Lys Gly Phe Leu
   35                  40                  45
   Gln Gln Ser Ser Ser Lys Glu Pro His Phe Thr Asp Phe Glu Gly Lys
   50                  55                  60
   Thr Ser Phe Gly Met Ser Val Phe Asn Leu Ser Asn Ala Ile Met Gly
   65                  70                  75                  80
   Ser Gly Ile Leu Gly Leu Ala Tyr Ala Met Ala Asn Thr Gly Ile Ile
   85                  90                  95
   Leu Phe Leu Phe Leu Leu Thr Ala Val Ala Leu Leu Ser Ser Tyr Ser
   100                 105                 110
   Ile His Leu Leu Leu Lys Ser Ser Gly Ile Val Gly Ile Arg Ala Tyr
   115                 120                 125
   Glu Gln Leu Gly Tyr Arg Ala Phe Gly Thr Pro Gly Lys Leu Ala Ala
   130                 135                 140
   Ala Leu Ala Ile Thr Leu Gln Asn Ile Gly Ala Met Ser Ser Tyr Leu
   145                 150                 155                 160
   Tyr Ile Ile Lys Ser Glu Leu Pro Leu Val Ile Gln Thr Phe Leu Asn
   165                 170                 175
   Leu Glu Lys Pro Thr Pro Val Trp Tyr Met Asp Gly Asn Tyr Leu Val
   180                 185                 190
   Ile Leu Val Ser Val Ile Ile Ile Leu Pro Leu Ala Leu Met Arg Gln
   195                 200                 205
   Leu Gly Tyr Leu Gly Tyr Ser Ser Gly Phe Ser Leu Ser Cys Met Val
   210                 215                 220
   Phe Phe Leu Ile Ala Val Ile Tyr Lys Lys Phe Gln Val Pro Cys Pro
   225                 230                 235                 240
   Leu Ala His Asn Leu Val Asn Ala Thr Gly Asn Phe Ser His Met Val
   245                 250                 255
   Val Val Glu Glu Lys Ser Gln Leu Gln Ser Glu Pro Asp Thr Ala Glu
   260                 265                 270
   Ala Phe Cys Thr Pro Ser Tyr Phe Thr Leu Asn Ser Gln Thr Ala Tyr
   275                 280                 285
   Thr Ile Pro Ile Met Ala Phe Ala Phe Val Cys His Pro Glu Val Leu
   290                 295                 300
   Pro Ile Tyr Thr Glu Leu Lys Asp Pro Ser Lys Arg Lys Met Gln His
   305                 310                 315                 320
   Ile Ser Asn Leu Ser Ile Ala Val Met Tyr Val Met Tyr Phe Leu Ala
   325                 330                 335
   Ala Leu Phe Gly Tyr Leu Thr Phe Tyr Asp Gly Val Glu Ser Glu Leu
   340                 345                 350
   Leu His Thr Tyr Ser Lys Val Asp Pro Phe Asp Val Leu Ile Leu Cys
   355                 360                 365
   Val Arg Val Ala Val Leu Ile Ala Val Thr Leu Thr Val Pro Ile Val
   370                 375                 380
   Leu Phe Pro Val Arg Arg Ala Ile Gln Gln Met Leu Phe Gln Asn Gln
   385                 390                 395                 400
   Glu Phe Ser Trp Leu Arg His Val Leu Ile Ala Thr Gly Leu Leu Thr
   405                 410                 415
   Cys Ile Asn Leu Leu Val Ile Phe Ala Pro Asn Ile Leu Gly Ile Phe
   420                 425                 430
   Gly Ile Ile Gly Ala Thr Ser Ala Pro Cys Leu Ile Phe Ile Phe Pro
   435                 440                 445
   Ala Ile Phe Tyr Phe Arg Ile Met Pro Thr Glu Lys Glu Pro Val Arg
   450                 455                 460
   Ser Thr Pro Lys Ile Leu Ala Leu Cys Phe Ala Ala Val Gly Phe Leu
   465                 470                 475                 480
   Leu Met Thr Met Ser Leu Ser Phe Ile Ile Thr Asp Trp Val Ser Gly
                   485                 490                 495
   Thr Ser Gln His Gly Gly Asn His
               500

<210> 7
<211> 504
<212> PRT
<213> Homo sapiens

<400> 7
Met Glu Ala Pro Leu Gln Thr Glu Met Val Glu Leu Val Pro Asn Gly
1               5                   10                  15
Lys His Ser Glu Gly Leu Leu Pro Val Ile Thr Pro Met Ala Gly Asn
            20                  25                  30
Gln Arg Val Glu Asp Pro Ala Arg Ser Cys Met Glu Gly Lys Ser Phe
        35                  40                  45
Leu Gln Lys Ser Pro Ser Lys Glu Pro His Phe Thr Asp Phe Glu Gly
    50                  55                  60
Lys Thr Ser Phe Gly Met Ser Val Phe Asn Leu Ser Asn Ala Ile Met
65                  70                  75                  80
Gly Ser Gly Ile Leu Gly Leu Ala Tyr Ala Met Ala Asn Thr Gly Ile
                85                  90                  95
Ile Leu Phe Leu Phe Leu Leu Thr Ala Val Ala Leu Leu Ser Ser Tyr
            100                 105                 110
Ser Ile His Leu Leu Leu Lys Ser Ser Gly Val Val Gly Ile Arg Ala
        115                 120                 125
Tyr Glu Gln Leu Gly Tyr Arg Ala Phe Gly Thr Pro Gly Lys Leu Ala
    130                 135                 140
Ala Ala Leu Ala Ile Thr Leu Gln Asn Ile Gly Ala Met Ser Ser Tyr
145                 150                 155                 160
Leu Tyr Ile Ile Lys Ser Glu Leu Pro Leu Val Ile Gln Thr Phe Leu
                165                 170                 175
Asn Leu Glu Glu Lys Thr Ser Asp Trp Tyr Met Asn Gly Asn Tyr Leu
            180                 185                 190
Val Ile Leu Val Ser Val Thr Ile Ile Leu Pro Leu Ala Leu Met Arg
        195                 200                 205
Gln Leu Gly Tyr Leu Gly Tyr Ser Ser Gly Phe Ser Leu Ser Cys Met
    210                 215                 220
Val Phe Phe Leu Ile Ala Val Ile Tyr Lys Lys Phe His Val Pro Cys
225                 230                 235                 240
Pro Leu Pro Pro Asn Phe Asn Asn Thr Thr Gly Asn Phe Ser His Val
                245                 250                 255
Glu Ile Val Lys Glu Lys Val Gln Leu Gln Val Glu Pro Glu Ala Ser
            260                 265                 270
Ala Phe Cys Thr Pro Ser Tyr Phe Thr Leu Asn Ser Gln Thr Ala Tyr
        275                 280                 285
Thr Ile Pro Ile Met Ala Phe Ala Phe Val Cys His Pro Glu Val Leu
    290                 295                 300
Pro Ile Tyr Thr Glu Leu Lys Asp Pro Ser Lys Lys Lys Met Gln His
305                 310                 315                 320
Ile Ser Asn Leu Ser Ile Ala Val Met Tyr Ile Met Tyr Phe Leu Ala
                325                 330                 335
Ala Leu Phe Gly Tyr Leu Thr Phe Tyr Asn Gly Val Glu Ser Glu Leu
            340                 345                 350
Leu His Thr Tyr Ser Lys Val Asp Pro Phe Asp Val Leu Ile Leu Cys

355                 360                 365
Val Arg Val Ala Val Leu Thr Ala Val Thr Leu Thr Val Pro Ile Val
    370                 375                 380
Leu Phe Pro Val Arg Arg Ala Ile Gln Gln Met Leu Phe Pro Asn Gln
385                 390                 395                 400
Glu Phe Ser Trp Leu Arg His Val Leu Ile Ala Val Gly Leu Leu Thr
                405                 410                 415
Cys Ile Asn Leu Leu Val Ile Phe Ala Pro Asn Ile Leu Gly Ile Phe
            420                 425                 430
Gly Val Ile Gly Ala Thr Ser Ala Pro Phe Leu Ile Phe Ile Phe Pro
        435                 440                 445
Ala Ile Phe Tyr Phe Arg Ile Met Pro Thr Glu Lys Glu Pro Ala Arg
    450                 455                 460
Ser Thr Pro Lys Ile Leu Ala Leu Cys Phe Ala Met Leu Gly Phe Leu
465                 470                 475                 480
Leu Met Thr Met Ser Leu Ser Phe Ile Ile Ile Asp Trp Ala Ser Gly
                485                 490                 495
Thr Ser Arg His Gly Gly Asn His
            500

<210> 8
<211> 476
<212> PRT
<213> Homo sapiens

<400> 8

## Description

### Background of the Invention

Amino acid transport across the plasma membrane introduces essential nutrients into cells, providing critical substrates for such processes as protein biosynthesis, hormone metabolism, regulation of cell growth, and production of metabolic energy. In addition to participating in systemic interorgan amino acid nutrition, amino acid transporters which act as neurotransmitters, neurotransmitter precursors, or synaptic modulators, ensure, among other things, reuptake from the synaptic cleft and a supply of precursors in the central nervous system.

Transfer of amino acids across the hydrophobic domain of the plasma membrane is mediated by proteins that recognize, bind, and transport these amino acids from the extracellular medium into the cell, or vice versa. These transporter proteins are categorized by the type of amino acid they move across the membrane (e.g., acidic, basic, zwitterionic, or other side chain-containing), and by the thermodynamic properties of the transport (i.e., whether the transporter is equilibrative or drives the organic substrate uphill).

Specific transport systems carry different amino acids, and yet some amino acid transport systems show overlapping specificities. Different cells contain a distinct set of transport systems in their plasma membranes as a combination of common (e.g., systems A, ASC, L, and y⁺) and tissue-specific transport systems (e.g., systems B^{0,+}, N^{m}, and b^{0,+}). For instance, zwitterionic amino acid transport systems A, ASC, and L are present in almost all cell types, and account for much of the amino acid uptake in general by mammalian cells. By comparison, the zwitterionic transport N system is far less common, expressed in cells of the brain, liver, and skeletal muscle, and exhibiting a narrow substrate specificity, preferentially transporting histidine, asparagine, and glutamine.

A wide variety of human diseases and disorders are associated with defects in amino acid transporters, including conditions associated with insulin deficiency or resistance, such as diabetes and starvation (Palacin et al. (1998) *Phys. Rev.* 78:969-1054); aminoacidurias (e.g., cystinuria, lysinuria; dicarboxylic amino aciduria (Gu et al. (2000) *PNAS* 97:3230-3235); dibasicaminoaciduria; Hartnup disease; and iminoglycinuria, which are characterized by impaired tubular reabsortion and excessive urinary secretion of amino acids; tryptophan malabsorption; methionine malabsorption; and CNS-disorders (such as amyotrophic lateral sclerosis and Alzheimer's disease), as glutamate excitotoxicity is thought to participate in the selective motor neuron degeneration of the disease (Rothstein (1996) *Clin. Neurosci.* 3:348-359).

In view of the important physiological activities attributable to amino acid transporters, including cellular nutrition, and further in view of the limited extent to which the key molecular mechanisms of amino acid transporters have been identified, a need exists for discovery of further members of this protein family. The present invention satisfies this need by providing a novel human amino acid transporter.

### Summary of the Invention

The present invention is based, in part, on the discovery of a novel gene encoding a amino acid transporter, the gene being referred to herein as "58297". The nucleotide sequence of a cDNA encoding 58297 is shown in SEQ ID NO:1, and the amino acid sequence of a 58297 polypeptide is shown in SEQ ID NO:2. In addition, the nucleotide sequence of the coding region is depicted in SEQ ID NO:3.

Accordingly, in one aspect, the invention features a nucleic acid molecule that encodes a 58297 protein or polypeptide, e.g., a biologically active portion of the 58297 protein. In a preferred embodiment the isolated nucleic acid molecule encodes a polypeptide having the amino acid sequence SEQ ID NO:2. In other embodiments, the invention provides isolated 58297 nucleic acid molecules having the nucleotide sequence of one of SEQ ID NO:1 and SEQ ID NO:3.

In still other embodiments, the invention provides nucleic acid molecules that have sequences that are substantially identical (e.g., naturally occurring allelic variants) to the nucleotide sequence of one of SEQ ID NO:1 and SEQ ID NO:3. In other embodiments, the invention provides a nucleic acid molecule which hybridizes under stringent hybridization conditions with a nucleic acid molecule having a sequence comprising the nucleotide sequence of one of SEQ ID NO:1 and SEQ ID NO:3, wherein the nucleic acid encodes a full length 58297 protein or an active fragment thereof.

In a related aspect, the invention further provides nucleic acid constructs that include a 58297 nucleic acid molecule described herein. In certain embodiments, the nucleic acid molecules of the invention are operatively linked to native or heterologous regulatory sequences. Also included are vectors and host cells containing the 58297 nucleic acid molecules of the invention, e.g., vectors and host cells suitable for producing 58297 nucleic acid molecules and polypeptides.

In another related aspect, the invention provides nucleic acid fragments suitable as primers or hybridization probes for detection of 58297-encoding nucleic acids.

In still another related aspect, isolated nucleic acid molecules that are antisense to a 58297-encoding nucleic acid molecule are provided.

In another aspect, the invention features 58297 polypeptides, and biologically active or antigenic fragments thereof, that are useful, e.g., as reagents or targets in assays applicable to treatment and diagnosis of 58297-mediated or -related disorders. In another embodiment, the invention provides 58297 polypeptides having a 58297 activity.
Preferred polypeptides are 58297 proteins including at least one transmembrane domain (and preferably at least 10 to 12 transmembrane domains) and at least one transmembrane amino acid transporter protein domain.

In other embodiments, the invention provides 58297 polypeptides, e.g., a 58297 polypeptide having the amino acid sequence shown in SEQ ID NO:2; an amino acid sequence that is substantially identical to the amino acid sequence shown in SEQ ID NO:2; or an amino acid sequence encoded by a nucleic acid molecule having a nucleotide sequence which hybridizes under stringent hybridization conditions to a nucleic acid molecule comprising the nucleotide sequence of any of SEQ ID NO:1 and SEQ ID NO:3, wherein the nucleic acid encodes a full length 58297 protein or an active fragment thereof.

In a related aspect, the invention further provides nucleic acid constructs that include a 58297 nucleic acid molecule described herein.

In a related aspect, the invention provides 58297 polypeptides or fragments operatively linked to non-58297 polypeptides to form fusion proteins.

In another aspect, the invention features antibodies and antigen-binding fragments thereof, that react with, or more preferably, specifically or selectively bind, 58297 polypeptides.

In another aspect, the invention provides methods of screening for compounds that modulate the expression or activity of the 58297 polypeptides or nucleic acids.

In still another aspect, the invention provides a process for modulating 58297 polypeptide or nucleic acid expression or activity, e.g., using the compounds identified in the screens described herein. In certain embodiments, the methods involve treatment of conditions related to aberrant activity or expression of the 58297 polypeptides or nucleic acids, such as conditions involving aberrant or deficient amino acid transport across the plasma membrane and/or cellular nutrition, and aminoacidurias, which are characterized by impaired tubular reabsortion and excessive urinary secretion of amino acids.

The invention also provides assays for determining the activity of or the presence or absence of 58297 polypeptides or nucleic acid molecules in a biological sample, including for disease diagnosis.

In further aspect the invention provides assays for determining the presence or absence of a genetic alteration in a 58297 polypeptide or nucleic acid molecule, including for disease diagnosis.

Other features and advantages of the invention will be apparent from the following detailed description, and from the claims.

### Brief Description of the Drawings

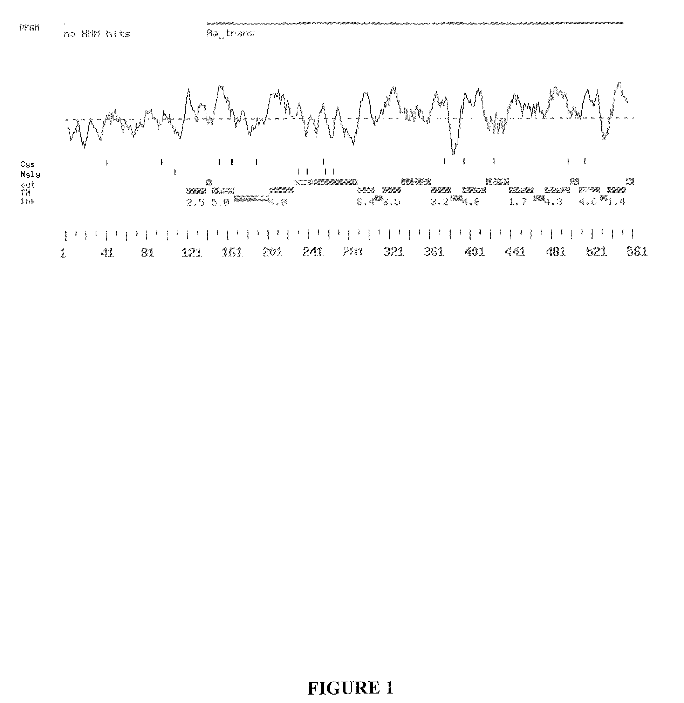

*Figure 1* depicts a hydropathy plot of human 58297. Relatively hydrophobic residues are shown above the dashed horizontal line, and relatively hydrophilic residues are below the dashed horizontal line. The cysteine residues (cys) are indicated by short vertical lines below the hydropathy trace. The numbers corresponding to the amino acid sequence of human 58297 are indicated. Polypeptides of the invention include fragments which include: all or part of a hydrophobic sequence, i.e., a sequence above the dashed line, e.g., the sequence of about residues 202-225 of SEQ ID NO:2; all or part of a hydrophilic sequence, i.e., a sequence below the dashed line, e.g., the sequence of residues 381-392 of SEQ ID NO:2; a sequence which includes a cysteine residue; or a glycosylation site.

### Detailed Description of the Invention

The human 58297 cDNA sequence (SEQ ID NO:1), which is approximately 2476 nucleotide residues long including non-translated regions, contains a methionine-initiated coding sequence of about 1683 nucleotide residues, excluding termination codon (i.e., nucleotide residues 128-1810 of SEQ ID NO:1; also shown in SEQ ID NO:3). The coding sequence encodes a 561 amino acid protein having the amino acid sequence SEQ ID NO:2.

Human 58297 contains the following regions or other structural features (for general information regarding PFAM identifiers, PS prefix and PF prefix domain identification numbers, refer to Sonnhammer et al. (1997, Protein 28:405-420) and http://www.psc.edu/general/
software/packages/pfam/pfam.html) :

a transmembrane amino acid transporter domain (PF01490) at about amino acid residues 141 to 551 of SEQ ID NO:2;

transmembrane domains at about amino acid residues 120-138, 145-166, 202-225, 289-305, 314-331, 362-380, 393-415, 439-462, 474-498, 508-528, and 536-553 of SEQ ID NO:2. 58297 protein is therefore has about 10, 11, or 12 transmembrane domains, as is characteristic of previously characterized amino acid transporters; and

post translational modification sites including: predicted N-glycosylation sites (Pfam accession number PS00001) at about amino acid residues 117-120, 239-242, 248-251, 266-269, and 274-277 of SEQ ID NO:2; predicted protein kinase C phosphorylation sites (Pfam accession number PS00005) at about amino acid residues 36-38, 227-229, and 330-332 of SEQ ID NO:2; predicted casein kinase II phosphorylation sites (Pfam accession number PS00006) located at about amino acid residues 14-17, 76-79, 109-112, and 181-184 of SEQ ID NO:2; and predicted N-myristoylation sites (Pfam accession number PS00008) at about amino acid residues 141-146, 197-202, 492-497, and 510-515 of SEQ ID NO:2.

The 58297 protein contains a significant number of structural characteristics in common with members of the amino acid transporter family. The term "family" when referring to the protein and nucleic acid molecules of the invention means two or more proteins or nucleic acid molecules having a common structural domain (e.g., a transmembrane amino acid transporter protein domain) or motif and having sufficient amino acid or nucleotide sequence homology as defined herein. Such family members can be naturally or non-naturally occurring and can be from either the same or different species. For example, a family can contain a first protein of human origin as well as other distinct proteins of human origin, or alternatively, can contain homologues of non-human origin, e.g., amino acid transporter proteins for any species described in the art (e.g., Steiner et al. (1995) *Mol. Microbiol.* 16:825-834, and references cited therein). Members of a family can also have common functional characteristics.

In one embodiment, the 58297 protein is a member of the N system amino acid transporter family of proteins, due to its sequence homology to identified members of the N system subset of amino acid transporters, including the following: human and rat system N transporter (SN-1)(Chaudrhy et al. (1999) *Cell* 99:769-780) (Fei et al. (2000) *J. Biol. Chem* 275:23707-17); and mouse NAT-1 (Gu et al. (2000) *PNAS* 97:3230-35).

The N transport system exhibits a narrow substrate specificity, preferentially transporting histidine, asparagine, and glutamine, and contains at least the following family members: human and rat system N transporter (SN1)(Chaudrhy et al. (1999) *Cell* 99:769-780) (Fei et al. (2000) *J. Biol. Chem* 275:23707-17); mouse NAT-1 (Gu et al. (2000) *PNAS* 97:3230-35); and human system N1 sodium and hydrogen coupled glutamine transporter protein (direct Genbank submission by NIH, accession number XP_003264). The N transport system family members are expressed in the liver, skeletal muscle, and brain (associated in particular with the blood-brain barrier), varying slightly in each of these tissues in terms of its pH sensitivity and sodium ion tolerance.

For instance, the liver N system can transport amino acids even if lithium ions replace sodium, though it requires a pH of roughly 7 or above. Contrarily, the brain/neuron N transport system (N^{b}) and the muscle N transport system (N^{m}) are comparatively lithium intolerant and pH dependent. System N transporters in general can mediate proton exchange as well as sodium ion cotransport.

The N system has an important role in glutamine uptake for the urea cycle, and exports newly synthesized glutamine for glutamine metabolism in the liver. Glutamine is the most abundant amino acid in the blood, and is involved in such metabolic pathways as ammonia metabolism, the synthesis of purines and pyrimidines, and the glutamine-glutamiate cycle that occurs between neurons and glial cells in the brain and between placenta and liver in the developing fetus. Glutamine also is implicated in the intercellular glutamine cycle that occurs in the liver between periportal hepatocytes and perivenous hepatocytes.

To determine whether a polypeptide or protein of interest has a conserved sequence or domain common to members of a protein family, the amino acid sequence of the protein can be searched against a database of profile hidden Markov models (profile HMMs), which uses statistical descriptions of a sequence family's consensus (e.g., HMMER, version 2.1.1) and PFAM, a collection of multiple sequence alignments and hidden Markov models covering many common protein domains (e.g., PFAM, version 5.5) using the default parameters
(http://www.sanger.ac.uk/Software/Pfam/HMM search). For example, the hmmsf program, which is available as part of the HMMER package of search programs, is a family specific default program for MILPAT0063 and a score of 15 is the default threshold score for determining a hit. Alternatively, the threshold score for determining a hit can be lowered (e.g., to 8 bits). A description of the PFAM database can be found in Sonhammer et al., (1997) *Proteins* 28(3):405-420 and a detailed description of HMMs can be found, for example, in Gribskov et al., (1990) *Meth. Enzymol.* 183:146-159; Gribskov et al., (1987) *Proc. Natl. Acad. Sci. USA* 84:4355-4358; Krogh et al., (1994) *J. Mol. Biol.* 235:1501-1531; and Stultz et al., (1993) *Protein Sci.* 2:305-314, the contents of which are incorporated herein by reference. See also, for example, The HMMER User's Guide at http://hmmer.wustl.edu/hmmer-html. For general information regarding PFAM identifiers, PS prefix and PF prefix domain identification numbers, refer to Sonnhammer et al. (1997) *Protein* 28:405-420 and
http//www.psc.edu/general/software/packages/pfam/pfam.html. See also, for example, http://www.expasy.ch/prosite and http://smart.embl-heidelberg.de/.

Using such search tools, a transmembrane amino acid transporter domain profile was identified in the amino acid sequence of SEQ ID NO:2 (e.g., amino acids 141-551 of SEQ ID NO:2). Accordingly, a 58297 protein having at least about 60-70%, more preferably about 70-80%, or about 80-90% homology with the transmembrane amino acid transporter domain profile of human 58297 are within the scope of the invention.

A 58297 protein can include a transmembrane amino acid transporter domain. As used herein, the term "transmembrane amino acid transporter domain" refers to a protein domain having an amino acid sequence of about 200-500 amino acid residues in length, preferably, at least about 300-450 amino acids, more preferably about 355-425 amino acid residues, even more preferably about 380-382 amino acid residues; which has a bit score for the alignment of the sequence to the transmembrane amino acid transporter domain (HMM) of at least -100 or greater, preferably -50 or greater, and most preferably, -30 or greater; and which has an E-value for the alignment of the sequence to the transmembrane amino acid transporter domain (HMM) of at least .05 or lower, preferably .025 or lower, preferably .01 or lower, and more preferably .005 or lower. The transmembrane amino acid transporter domain has been assigned the PFAM accession PF01490 (http://pfam.wustl.edu/cgi-bin/getdesc?name=Aa_trans).

A 58297 transmembrane amino acid transporter domain is found in at least the following amino acid transport proteins: human and rat system N transporter (SN1)(Chaudrhy et al. (1999) *Cell* 99:769-780) (Fei et al. (2000) *J. Biol. Chem* 275:23707-17); mouse NAT-1 (Gu et al. (2000) *PNAS* 97:3230-35); human system N1 sodium and hydrogen coupled glutamine transporter protein (direct Genbank submission by NIH, accession number XP_003264); and gamma aminobutyric acid (A) receptor (GABA) (Mclntire et al. (1997) *Nature* 389:870-876)).

In one embodiment, the 58297 polypeptide or protein has a transmembrane amino acid transporter domain or region which includes at least about 200-500, more preferably 300-450, 355-425, or 382-411 amino acid residues and has at least about 60%, 70%, 80%, 82%, 84%, 85%, 86%, 87%, 88%, 89%, 90%, 91%, 92%, 93%, 94%, 95%, 96%, 97%, 98%, 99%, or 100% homology with a transmembrane amino acid transporter domain, e.g., the transmembrane amino acid transporter domain of human 58297 (e.g., residues 141 to 551 of SEQ ID NO:2).

In still another embodiment, the 58297 polypeptide or protein has a transmembrane amino acid transporter domain or a region which includes at least about 200-500, more preferably 300-450, 355-425, or 382-411 200-500, more preferably 300-450, 355-425, or 382-411 amino acid residues and has at least about 60%, 70%, 80%, 82%, 84%, 85%, 86%, 87%, 88%, 89%, 90%, 91%, 92%, 93%, 94%, 95%, 96%, 97%, 98%, 99%, or 100% homology with a transmembrane amino acid transporter domain, e.g., the transmembrane amino acid transporter domain of human 58297 (e.g., residues 141 to 551 of SEQ ID NO:2), and has at least one 58297 biological activity as described herein.

In one embodiment, a 58297 protein includes at least eleven transmembrane domains. As used herein, the term "transmembrane domain" includes an amino acid sequence of about 5 amino acid residues in length that spans the plasma membrane. More preferably, a transmembrane domain includes about at least 10, 15, 20 or 22-25 amino acid residues and spans a membrane. Transmembrane domains are rich in hydrophobic residues, and typically have an alpha-helical structure. In a preferred embodiment, at least 50%, 60%, 70%, 80%, 90%, or 95% or more of the amino acids of a transmembrane domain are hydrophobic, e.g., leucines, isoleucines, tyrosines, or tryptophans. Transmembrane domains are described in, for example, htto://pfam.wustl.edu/cgi-bin/getdesc?name=7tm-1, and Zagotta W.N. et al. (1996) *Annu. Rev. Neurosci*. 19:235-263, the contents of which are incorporated herein by reference. Transmembrane domains exist at least from about amino acid residues 120-138, 145-166, 202-225, 289-305, 314-331, 362-380, 393-415, 439-462, 474-498, 508-528, and 536-553 of SEQ ID NO:2.

A 58297 family member can include at least one transmembrane amino acid transporter domain. Furthermore, a 58297 family member can include at least one, preferably at least 5, more preferably at least 9, and still more preferably 10-11 transmembrane domains; at least one, preferably five, N-glycosylation sites; at least one, preferably three protein kinase C phosphorylation sites; at least one, preferably four casein kinase II phosphorylation sites; and at least one, preferably four N-myristoylation sites.

58297 is homologous to murine NAT1 (Genbank accession number AF15856; SEQ ID NO:5) (Cu et al. (2000) *PNAS* 97:3230-35), a murine N-system amino acid transporter. An alignment between murine NAT1 and the amino acid sequence of 58297 (SEQ ID NO:2) reveals 17.5% identity and has a global alignment score of 86. Using the tools described herein, the murine NAT1 protein was searched against a database of profile hidden Markov models (profile HMMs), which uses statistical descriptions of a sequence family's consensus (e.g., HMMER, version 2.1.1) and PFAM, a collection of multiple sequence alignments and hidden Markov models covering many common protein domains (e.g., PFAM, version 5.5). Like the 58297 protein, murine NAT1 contains a transmembrane amino acid transporter domain, as well as 11 transmembrane domains.

58297 is homologous to rat SN1 (Genbank accession number AAF81797; SEQ ID NO:6), a system N protein related to a vesicular neurotransmitter transporter (Chaudrhy et al. (1999) *Cell* 99:769-780). An alignment between rat SN1 and the amino acid sequence of 58297 (SEQ ID NO:2) reveals 17.3% identity and has a global alignment score of 78. Using the tools described herein, the rat SN1 protein was searched against a database of profile hidden Markov models (profile HMMs), which uses statistical descriptions of a sequence family's consensus (e.g., HMMER, version 2.1.1) and PFAM, a collection of multiple sequence alignments and hidden Markov models covering many common protein domains (e.g., PFAM, version 5.5). Like the 58297 protein, rat SN1 contains a transmembrane amino acid transporter domain, as well as 10 transmembrane domains.

58297 is homologous to human SN1 protein, also known as human g17 protein (Genbank accession numbers NP_006832 and XP_003264; SEQ ID NO:7), an sodium and hydrogen coupled amino acid transport system protein (Fei et al. (2000) *J. Biol. Chem* 275:23707-17; and direct Genbank submission by NIH). An alignment between human SN1 and the amino acid sequence of 58297 (SEQ ID NO:2) reveals 18.6% identity and has a global alignment score of 107. Using the tools described herein, the human SN1 protein was searched against a database of profile hidden Markov models (profile HMMs), which uses statistical descriptions of a sequence family's consensus (e.g., HMMER, version 2.1.1) and PFAM, a collection of multiple sequence alignments and hidden Markov models covering many common protein domains (e.g., PFAM, version 5.5). Like the 58297 protein, human SN1 contains a transmembrane amino acid transporter domain, as well as 11 transmembrane domains.

58297 is homologous to human JM24 protein (Genbank accession number AAF06800; SEQ ID NO:8), a predicted transporter protein that was directly submitted to Genbank by The Institute of Molecular Biology, Germany. An alignment of human JM24 and the amino acid sequence of 58297 (SEQ ID NO:2) reveals 18.0% identity and has a global alignment score of - 11. Using the tools described herein, the human JM24 protein was searched against a database of profile hidden Markov models (profile HMMs), which uses statistical descriptions of a sequence family's consensus (e.g., HMMER, version 2.1.1) and PFAM, a collection of multiple sequence alignments and hidden Markov models covering many common protein domains (e.g., PFAM, version 5.5). Like the 58297 protein, human JM24 contains a transmembrane amino acid transporter domain, as well as 10 transmembrane domains.

Based on the above described sequence similarities, the 58297 molecules of the present invention belong to the amino acid transporter family (as described herein). Consequently, the 58297 molecules of the invention have similar biological activities as amino acid transporter family members, and useful in treating the same disorders as amino acid transporter family members.

58297 is also homologous to sequences appearing in the art which are not annotated as amino acid transporters, likely because these sequences appear in patent applications listing hundreds or thousands of genes in a largely unannotated fashion. Nevertheless, based on sequence similarities to these sequences of known expression pattern, 58297 molecules of the invention can exhibit similar expression patterns, and therefore can be useful in treating disorders related to tissues in which they are expressed.

Because the 58297 polypeptides of the invention can modulate 58297-mediated activities, they can be used to develop novel diagnostic and therapeutic agents for 58297-mediated or related disorders (e.g., disorders associated with amino acid transporter family members), as described below.

As used herein, a "58297 activity", "biological activity of 58297", or "functional activity of 58297", refers to an activity of an amino acid transporter family member, and refers to an activity exerted by a 58297 protein, polypeptide or nucleic acid molecule on, for example, a 58297-responsive cell or on a 58297 substrate (e.g., a protein substrate) as determined *in vivo* or *in vitro.* In one embodiment, a 58297 activity is a direct activity, such as association with a 58297 target molecule. A "target molecule" or "binding partner" of a 58297 protein is a molecule with which the 58297 protein binds or interacts in nature. In an exemplary embodiment, such a target molecule includes amino acids, e.g., amino acids which 58297 proteins can transport across the plasma membrane, e.g., amino acids which serve as substrates for N transport system proteins, e.g., histidine, asparagine, and glutamine.

A 58297 activity can also be an indirect activity, such as an activity mediated by interaction of the 58297 protein with a 58297 target molecule such that the target molecule modulates a downstream cellular activity, e.g., a cellular signaling activity modulated indirectly by interaction of the 58297 protein with a 58297 target molecule (e.g., an amino acid).

For example, the 58297 proteins of the present invention can have one or more of the following activities: (1) the ability to modulate (e.g., promote, catalyze, regulate, initiate, facilitate or inhibit) transmembrane transport of amino acids (e.g., amino acids associated with N system transport, e.g., histidine, asparagine, and glutamine) across the plasma membrane, e.g., from an extracellular medium into a cell, or vice versa; (2) the ability to modulate (e.g., promote, regulate, initiate, facilitate or inhibit) cellular nutrition, e.g., by modulating the transport of amino acids, e.g., amino acids associated with N system transport; (3) the ability to modulate (e.g., promote, regulate, initiate, facilitate or inhibit) protein biosynthesis, e.g., by modulating the transport of amino acids, e.g., amino acids associated with N system transport; (4) the ability to modulate (e.g., promote, regulate, initiate, facilitate or inhibit) hormone metabolism, e.g., by modulating the transport of amino acids, e.g., amino acids associated with N system transport; (5) the ability to modulate (e.g., promote, regulate, initiate, facilitate or inhibit) cell growth, e.g., by modulating the transport of amino acids, e.g., amino acids associated with N system transport; (6) the ability to modulate (e.g., promote, regulate, initiate, facilitate or inhibit) metabolic energy production, e.g., by modulating the transport of amino acids, e.g., amino acids associated with N system transport; and (7) the ability to modulate (e.g., promote, regulate, initiate, facilitate or inhibit) CNS signal transduction/transmission, e.g., by modulating reuptake from the synaptic cleft, or by supplying CNS precursors (e.g., by modulating the transport of amino acids, e.g., amino acids associated with N system transport).

Other activities of the 58297 proteins of the present invention include one or more of the following:

(1) the ability to modulate (e.g., promote, catalyze, regulate, initiate, facilitate or inhibit) ammonia metabolism, e.g., via cellular uptake of glutamine; (2) the ability to modulate (e.g., promote, catalyze, regulate, initiate, facilitate or inhibit) synthesis of purines and/or pyrimidines, e.g., via cellular uptake of glutamine; (3) the ability to modulate (e.g., maintain, promote, or arrest) the glutamine-glutamate cycle (e.g., that which occurs between neurons and glial cells in the brain, between placenta and liver in the developing fetus, and in the liver, between periportal hepatocytes and perivenous hepatocytes), e.g., via cellular uptake of glutamine; and (4) the ability to modulate (e.g., promote, catalyze, regulate, initiate, facilitate or inhibit) CNS-related disorders (e.g., amyotrophic later sclerosis, Alzheimer's disease) (e.g., modulate the onset, continuation, or cessation of), e.g., via cellular uptake of glutamine, as glutamate can be a excitotoxin which contributes to the death of nerve cells in a variety of neurodegenerative disorders.

Thus, 58297 molecules described herein can act as novel diagnostic targets and therapeutic agents for prognosticating, diagnosing, preventing, inhibiting, alleviating, or curing amino acid transporter-related disorders.

Other activities, as described below, include the ability to modulate function, survival, morphology, proliferation and/or differentiation of, and oligopeptide uptake by cells of tissues in which 58297 molecules are expressed. Thus, the 58297 molecules can act as novel diagnostic targets and therapeutic agents for controlling disorders involving aberrant activities of these cells.

As used herein, a "amino acid transport disorder" includes a disorder, disease or condition which is caused by, characterized by, or associated with a misregulation (e.g., an aberrant downregulation or upregulation) of an amino acid transport activity or an abnormal amino acid transport activity. Amino acid transport disorders can detrimentally affect cellular functions such as amino acid nutrition, cellular regulation of homeostasis, and inter- or intracellular communication.

Accordingly, the 58297 molecules of the invention, as amino acid transporters, can mediate, and can act as novel diagnostic targets and therapeutic agents for controlling, one or more amino acid transporter-associated disorders, including genetic disorders of membrane transport (e.g., the amino acid transporter subset thereof); CNS-related (e.g., neurological) disorders; liver-related (i.e., hepatic) disorders; skeletal muscle-related disorders; cellular proliferative and/or differentiative disorders; hormonal disorders; immune and inflammatory disorders; cardiovascular disorders; blood vessel disorders; neutrophil disorders; testicular disorders; and platelet disorders. As the 58297 molecules of the invention can modulate amino acid transporter activities, they are useful for developing novel diagnostic and therapeutic agents for 58297-mediated or related disorders, as described herein.

Examples of amino acid transport disorders include genetic diseases of membrane transport (e.g., the amino acid transporter subset thereof). Genetic diseases of membrane transport which can be treated or diagnosed by methods described herein include but are not limited to, classic cystinuria, dibasicaminoaciduria, hypercystinurai, lysinuria, Hartnup disease, tryptophan malabsorption, methionine malabsorption, histidinuria, iminoglycinuria, dicarboxlicaminoaciduria, and cystinosis.

Additional amino acid transport disorders include neurological disorders. Neurological disorders which can be treated or diagnosed by methods described herein include, but are not limited to, disorders involving neurons, and disorders involving glia, such as astrocytes, oligodendrocytes, ependymal cells, and microglia; cerebral edema, raised intracranial pressure and herniation, and hydrocephalus; malformations and developmental diseases, such as neural tube defects, forebrain anomalies, posterior fossa anomalies, and syringomyelia and hydromyelia; perinatal brain injury; cerebrovascular diseases, such as those related to hypoxia, ischemia, and infarction, including hypotension, hypoperfusion, and low-flow states--global cerebral ischemia and focal cerebral ischemia--infarction from obstruction of local blood supply, intracranial hemorrhage, including intracerebral (intraparenchymal) hemorrhage, subarachnoid hemorrhage and ruptured berry aneurysms, and vascular malformations, hypertensive cerebrovascular disease, including lacunar infarcts, slit hemorrhages, and hypertensive encephalopathy; infections, such as acute meningitis, including acute pyogenic (bacterial) meningitis and acute aseptic (viral) meningitis, acute focal suppurative infections, including brain abscess, subdural empyema, and extradural abscess, chronic bacterial meningoencephalitis, including tuberculosis and mycobacterioses, neurosyphilis, and neuroborreliosis (Lyme disease), viral meningoencephalitis, including arthropod-borne (Arbo) viral encephalitis, *Herpes simplex* virus Type 1, *Herpes simplex* virus Type 2, *Varicellazoster* virus (*Herpes zoster*), cytomegalovirus, poliomyelitis, rabies, and human immunodeficiency virus 1, including HIV-1 meningoencephalitis (subacute encephalitis), vacuolar myelopathy, AIDS-associated myopathy, peripheral neuropathy, and AIDS in children, progressive multifocal leukoencephalopathy, subacute sclerosing panencephalitis, fungal meningoencephalitis, other infectious diseases of the nervous system; transmissible spongiform encephalopathies (prion diseases); demyelinating diseases, including multiple sclerosis, multiple sclerosis variants, acute disseminated encephalomyelitis and acute necrotizing hemorrhagic encephalomyelitis, and other diseases with demyelination; degenerative diseases, such as degenerative diseases affecting the cerebral cortex, including Alzheimer's disease and Pick's disease, degenerative diseases of basal ganglia and brain stem, including Parkinsonism, idiopathic Parkinson's disease (paralysis agitans), progressive supranuclear palsy, corticobasal degenration, multiple system atrophy, including striatonigral degenration, Shy-Drager syndrome, and olivopontocerebellar atrophy, and Huntington's disease; spinocerebellar degenerations, including spinocerebellar ataxias, including Friedreich ataxia, and ataxiatelanglectasia, degenerative diseases affecting motor neurons, including amyotrophic lateral sclerosis (motor neuron disease), bulbospinal atrophy (Kennedy syndrome), and spinal muscular atrophy; inborn errors of metabolism, such as leukodystrophies, including Krabbe disease, metachromatic leukodystrophy, adrenoleukodystrophy, Pelizaeus-Merzbacher disease, and Canavan disease, mitochondrial encephalomyopathies, including Leigh disease and other mitochondrial encephalomyopathies; toxic and acquired metabolic diseases, including vitamin deficiencies such as thiamine (vitamin B₁) deficiency and vitamin B₁₂ deficiency, neurologic sequelae of metabolic disturbances, including hypoglycemia, hyperglycemia, and hepatic encephatopathy, toxic disorders, including carbon monoxide, methanol, ethanol, and radiation, including combined methotrexate and radiation-induced injury; tumors, such as gliomas, including astrocytoma, including fibrillary (diffuse) astrocytoma and glioblastoma multiforme, pilocytic astrocytoma, pleomorphic xanthoastrocytoma, and brain stem glioma, oligodendroglioma, and ependymoma and related paraventricular mass lesions, neuronal tumors, poorly differentiated neoplasms, including medulloblastoma, other parenchymal tumors, including primary brain lymphoma, germ cell tumors, and pineal parenchymal tumors, meningiomas, metastatic tumors, paraneoplastic syndromes, peripheral nerve sheath tumors, including schwannoma, neurofibroma, and malignant peripheral nerve sheath tumor (malignant schwannoma), and neurocutaneous syndromes (phakomatoses), including neurofibromotosis, including Type 1 neurofibromatosis (NF1) and TYPE 2 neurofibromatosis (NF2), tuberous sclerosis, and Von Hippel-Lindau disease.

Additional amino acid transport disorders include hepatic disorders. Hepatic disorders which can be treated or diagnosed by methods described herein include, but are not limited to, disorders associated with an accumulation in the liver of fibrous tissue, such as that resulting from an imbalance between production and degradation of the extracellular matrix accompanied by the collapse and condensation of preexisting fibers. The methods described herein can be used to diagnose or treat hepatocellular necrosis or injury induced by a wide variety of agents including processes which disturb homeostasis, such as an inflammatory process, tissue damage resulting from toxic injury or altered hepatic blood flow, and infections (e.g., bacterial, viral and parasitic). For example, the methods can be used for the early detection of hepatic injury, such as portal hypertension or hepatic fibrosis. In addition, the methods can be employed to detect liver fibrosis attributed to inborn errors of metabolsim, for example, fibrosis resulting from a storage disorder such as Gaucher's disease (lipid abnormalities) or a glycogen storage disease, A1-antitrypsin deficiency; a disorder mediating the accumulation (e.g., storage) of an exogenous substance, for example, hemochromatosis (iron-overload syndrome) and copper storage diseases (Wilson's disease), disorders resulting in the accumulation of a toxic metabolite (e.g., tyrosinemia, fructosemia and galactosemia) and peroxisomal disorders (e.g., Zellweger syndrome). Additionally, the methods described herein may be useful for the early detection and treatment of liver injury associated with the administration of various chemicals or drugs, such as for example, methotrexate, isonizaid, oxyphenisatin, methyldopa, chlorpromazine, tolbutamide or alcohol, or which represents a hepatic manifestation of a vascular disorder such as obstruction of either the intrahepatic or extrahepatic bile flow or an alteration in hepatic circulation resulting, for example, from chronic heart failure, veno-occlusive disease, portal vein thrombosis or Budd-Chiari syndrome.

Additional amino acid transport disorders include skeletal muscle-related disorders. Skeletal muscle-related disorders which can be treated or diagnosed by methods described herein include, but are not limited to, muscular dystrophy (e.g., duchenne muscular dystrophy, becker muscular dystrophy, emery-dreifuss muscular dystrophy, limb-girdle muscular dystrophy, facioscapulohumeral muscular dystrophy, myotonic dystrophy, oculopharyngeal muscular dystrophy, distal muscular dystrophy, and congenital muscular dystrophy), motor neuron diseases (e.g., amyotrophic lateral sclerosis, infantile progressive spinal muscular atrophy, intermediate spinal muscular atrophy, spinal bulbar muscular atrophy, and adult spinal muscular atrophy), myopathies (e.g., inflammatory myopathies (e.g., dermatomyositis and polymyositis), myotonia congenita, paramyotonia congenita, central core disease, nemaline myopathy, myotubular myopathy, and periodic paralysis), and metabolic diseases of muscle (e.g., phosphorylase deficiency, acid maltase deficiency, phosphofructokinase deficiency, debrancher enzyme deficiency, mitochondrial myopathy, carnitine deficiency, carnitine palmityl transferase deficiency, phosphoglycerate kinase deficiency, phosphoglycerate mutase deficiency, lactate dehydrogenase deficiency, and myoadenylate deaminase deficiency).

Examples of cellular proliferative and/or differentiative disorders include cancer, e.g., carcinoma, sarcoma, metastatic disorders or hematopoietic neoplastic disorders, e.g., leukemias. A metastatic tumor can arise from a multitude of primary tumor types, including but not limited to those of prostate, colon, lung, breast and liver origin.

As used herein, the term "cancer" (also used interchangeably with the terms, "hyperproliferative" and "neoplastic") refers to cells having the capacity for autonomous growth, i.e., an abnormal state or condition characterized by rapidly proliferating cell growth. Cancerous disease states may be categorized as pathologic, i.e., characterizing or constituting a disease state, e.g., malignant tumor growth, or may be categorized as non-pathologic, i.e., a deviation from normal but not associated with a disease state, e.g., cell proliferation associated with wound repair. The term is meant to include all types of cancerous growths or oncogenic processes, metastatic tissues or malignantly transformed cells, tissues, or organs, irrespective of histopathologic type or stage of invasiveness. The term "cancer" includes malignancies of the various organ systems, such as those affecting lung, breast, thyroid, lymphoid, gastrointestinal, and genito-urinary tract, as well as adenocarcinomas which include malignancies such as most colon cancers, renal-cell carcinoma, prostate cancer and/or testicular tumors, non-small cell carcinoma of the lung, cancer of the small intestine and cancer of the esophagus. The term "carcinoma" is art recognized and refers to malignancies of epithelial or endocrine tissues including respiratory system carcinomas, gastrointestinal system carcinomas, genitourinary system carcinomas, testicular carcinomas, breast carcinomas, prostatic carcinomas, endocrine system carcinomas, and melanomas. Exemplary carcinomas include those forming from tissue of the cervix, lung, prostate, breast, head and neck, colon and ovary. The term "carcinoma" also includes carcinosarcomas, e.g., which include malignant tumors composed of carcinomatous and sarcomatous tissues. An "adenocarcinoma" refers to a carcinoma derived from glandular tissue or in which the tumor cells form recognizable glandular structures. The term "sarcoma" is art recognized and refers to malignant tumors of mesenchymal derivation.

The 58297 molecules of the invention can be used to monitor, treat and/or diagnose a variety of proliferative disorders. Such disorders include hematopoietic neoplastic disorders. As used herein, the term "hematopoietic neoplastic disorders" includes diseases involving hyperplastic/neoplastic cells of hematopoietic origin, *e.g*., arising from myeloid, lymphoid or erythroid lineages, or precursor cells thereof. Typically, the diseases arise from poorly differentiated acute leukemias, *e.g*., erythroblastic leukemia and acute megakaryoblastic leukemia. Additional exemplary myeloid disorders include, but are not limited to, acute promyeloid leukemia (APML), acute myelogenous leukemia (AML) and chronic myelogenous leukemia (CML) (reviewed in Vaickus, L., (1991) *Crit. Rev. in Oncol.*/*Hemotol*. 11:267-97); lymphoid malignancies include, but are not limited to acute lymphoblastic leukemia (ALL) which includes B-lineage ALL and T-lineage ALL, chronic lymphocytic leukemia (CLL), prolymphocytic leukemia (PLL), hairy cell leukemia (HLL) and Waldenstrom's macroglobulinemia (WM). Additional forms of malignant lymphomas include, but are not limited to non-Hodgkin lymphoma and variants thereof, peripheral T cell lymphomas, adult T cell leukemia/lymphoma (ATL), cutaneous T-cell lymphoma (CTCL), large granular lymphocytic leukemia (LGF), Hodgkin's disease and Reed-Sternberg disease.

Amino acid transport disorders can include hormonal disorders, such as conditions or diseases in which the production and/or regulation of hormones in an organism is aberrant. Examples of such disorders and diseases include type I and type II diabetes mellitus, pituitary disorders (*e.g*., growth disorders), thyroid disorders (*e.g*., hypothyroidism or hyperthyroidism), and reproductive or fertility disorders (*e.g*., disorders which affect the organs of the reproductive system, *e.g*., the prostate gland, the uterus, or the vagina; disorders which involve an imbalance in the levels of a reproductive hormone in a subject; disorders affecting the ability of a subject to reproduce; and disorders affecting secondary sex characteristic development, *e.g*., adrenal hyperplasia).

Amino acid transport disorders also include immune disorders, such as autoimmune disorders or immune deficiency disorders, *e.g*., congenital X-linked infantile hypogammaglobulinemia, transient hypogammaglobulinemia, common variable immunodeficiency, selective IgA deficiency, chronic mucocutaneous candidiasis, or severe combined immunodeficiency. Other examples of disorders include autoimmune diseases (including, for example, diabetes mellitus, arthritis (including rheumatoid arthritis, juvenile rheumatoid arthritis, osteoarthritis, psoriatic arthritis), multiple sclerosis, encephalomyelitis, myasthenia gravis, systemic lupus erythematosis, autoimmune thyroiditis, dermatitis (including atopic dermatitis and eczematous dermatitis), psoriasis, Sjögren's Syndrome, sepsis, acne, inflammatory bowel disease (e.g., Crohn's disease and ulcerative colitis), aphthous ulcer, iritis, conjunctivitis, keratoconjunctivitis, respiratory inflammation (e.g., asthma, allergic asthma, and chronic obstructive pulmonary disease), cutaneous lupus erythematosus, scleroderma, vaginitis, proctitis, drug eruptions, leprosy reversal reactions, erythema nodosum leprosum, autoimmune uveitis, allergic encephalomyelitis, acute necrotizing hemorrhagic encephalopathy, idiopathic bilateral progressive sensorineural hearing loss, aplastic anemia, pure red cell anemia, idiopathic thrombocytopenia, polychondritis, Wegener's granulomatosis, chronic active hepatitis, Stevens-Johnson syndrome, idiopathic sprue, lichen planus, Graves' disease, sarcoidosis, primary biliary cirrhosis, uveitis posterior, and interstitial lung fibrosis), graft-versus-host disease, cases of transplantation, and allergy such as, atopic allergy.

Cardiovascular disorders include, but are not limited to, heart failure, including but not limited to, cardiac hypertrophy, left-sided heart failure, and right-sided heart failure; ischemic heart disease, including but not limited to angina pectoris, myocardial infarction, chronic ischemic heart disease, and sudden cardiac death; hypertensive heart disease, including but not limited to, systemic (left-sided) hypertensive heart disease and pulmonary (right-sided) hypertensive heart disease; valvular heart disease, including but not limited to, valvular degeneration caused by calcification, such as calcification of a congenitally bicuspid aortic valve, and mitral annular calcification, and myxomatous degeneration of the mitral valve (mitral valve prolapse), rheumatic fever and rheumatic heart disease, infective endocarditis, and noninfected vegetations, such as nonbacterial thrombotic endocarditis and endocarditis of systemic lupus erythematosus (Libman-Sacks disease), carcinoid heart disease, and complications of artificial valves; myocardial disease, including but not limited to dilated cardiomyopathy, hypertrophic cardiomyopathy, restrictive cardiomyopathy, and myocarditis; pericardial disease, including but not limited to, pericardial effusion and hemopericardium and pericarditis, including acute pericarditis and healed pericarditis, and rheumatoid heart disease; neoplastic heart disease, including but not limited to, primary cardiac tumors, such as myxoma, lipoma, papillary fibroelastoma, rhabdomyoma, and sarcoma, and cardiac effects of noncardiac neoplasms; congenital heart disease, including but not limited to, left-to-right shunts--late cyanosis, such as atrial septal defect, ventricular septal defect, patent ductus arteriosus, and atrioventricular septal defect, right-to-left shunts--early cyanosis, such as tetralogy of fallot, transposition of great arteries, truncus arteriosus, tricuspid atresia, and total anomalous pulmonary venous connection, obstructive congenital anomalies, such as coarctation of aorta, pulmonary stenosis and atresia, and aortic stenosis and atresia, disorders involving cardiac transplantation, and congestive heart failure.

Disorders involving blood vessels include, but are not limited to, responses of vascular cell walls to injury, such as endothelial dysfunction and endothelial activation and intimal thickening; vascular diseases including, but not limited to, congenital anomalies, such as arteriovenous fistula, atherosclerosis, and hypertensive vascular disease, such as hypertension; inflammatory disease--the vasculitides, such as giant cell (temporal) arteritis, Takayasu arteritis, polyarteritis nodosa (classic), Kawasaki syndrome (mucocutaneous lymph node syndrome), microscopic polyanglitis (microscopic polyarteritis, hypersensitivity or leukocytoclastic anglitis), Wegener granulomatosis, thromboanglitis obliterans (Buerger disease), vasculitis associated with other disorders, and infectious arteritis; Raynaud disease; aneurysms and dissection, such as abdominal aortic aneurysms, syphilitic (luetic) aneurysms, and aortic dissection (dissecting hematoma); disorders of veins and lymphatics, such as varicose veins, thrombophlebitis and phlebothrombosis, obstruction of superior vena cava (superior vena cava syndrome), obstruction of inferior vena cava (inferior vena cava syndrome), and lymphangitis and lymphedema; tumors, including benign tumors and tumor-like conditions, such as hemangioma, lymphangioma, glomus tumor (glomangioma), vascular ectasias, and bacillary angiomatosis, and intermediate-grade (borderline low-grade malignant) tumors, such as Kaposi's sarcoma and hemangloendothelioma, and malignant tumors, such as angiosarcoma and hemangiopericytoma; and pathology of therapeutic interventions in vascular disease, such as balloon angioplasty and related techniques and vascular replacement, such as coronary artery bypass graft surgery.

Neutrophil associated disorders include neutropenias that result from or accompany a number of conditions, including, but not limited to, chemotherapy; chronic idopathic neutropenia; Felty's syndrome' acute infectious disease, lymphoma or aleukemic lymphocytic leukemia, myelodysplastic syndrome, and rhematic diseases such as systemic lupus erythematosus, rheumatoid arthritis, and polymyositis. Also included is neutrophilia, for example, accompanying chronic myelogenous leukemia.

Testicular disorders include, but are not limited to, unilateral testicular enlargement (e.g., nontuberculous, granulomatous orchitis); inflammatory diseases resulting in testicular dysfunction (e.g., gonorrhea and mumps); epididymo-orchitis; cryptorchidism; sperm cell disorders (e.g., immotile cilia syndrome and germinal cell aplasia); acquired testicular defects (e.g., viral orchitis and mumps orchitis); genesis and metastasis of testicular cancers; and tumors (e.g., germ cell tumors, interstitial cell tumors, androblastoma, testicular lymphoma and adenomatoid tumors).

Blood platelet disorders include, but are not limited to, thrombocytopenia due to a reduced number of megakaryocytes in the bone marrow, for example, as a result of chemotherapy; invasive disorders, such as leukemia, idiopathic or drug- or toxin-induced aplasia of the marrow, or rare hereditary amegakaryocytic thrombocytopenias; ineffective thrombopoiesis, for example, as a result of megaloblastic anemia, alcohol toxicity, vitamin B12 or folate deficiency, myelodysplastic disorders, or rare hereditary disorders (e.g., Wiskott-Aldrich syndrome and May-hegglin anomaly); a reduction in platelet distribution, for example, as a result of cirrhosis, a splenic invasive disease (e.g., Gaucher's disease), or myelofibrosis with extramedullary myeloid metaplasia; increased platelet destruction, for example, as a result of removal of IgG-coated platelets by the mononuclear phagocytic system (e.g., idiopathic thrombocytopenic purpura (ITP), secondary immune thrombocytopenia (e.g., systemic lupus erythematosus, lymphoma, or chronic lymphocytic leukemia), drug-related immune thrombocytopenias (e.g., as with quinidine, aspirin, and heparin), post-transfusion purpura, and neonatal thrombocytopenia as a result of maternal platelet autoantibodies or maternal platelet alloantibodies). Also included are thrombocytopenia secondary to intravascular clotting and thrombin induced damage to platelets as a result of, for example, obstetric complications, metastatic tumors, severe gram-negative bacteremia, thrombotic thrombocytopenic purpura, or severe illness. Also included is dilutional thrombocytopenia, for example, due to massive hemorrhage. Blood platelet disorders also include, but are not limited to, essential thrombocytosis and thrombocytosis associated with, for example, splenectomy, acute or chronic inflammatory diseases, hemolytic anemia, carcinoma, Hodgkin's disease, lymphoproliferative disorders, and malignant lymphomas.

The 58297 protein, fragments thereof, and derivatives and other variants of the sequence in SEQ ID NO:2 thereof are collectively referred to as "polypeptides or proteins of the invention" or "58297 polypeptides or proteins". Nucleic acid molecules encoding such polypeptides or proteins are collectively referred to as "nucleic acids of the invention" or "58297 nucleic acids." 58297 molecules refer to 58297 nucleic acids, polypeptides, antibodies, as well as modulators and variants thereof.

As used herein, the term "nucleic acid molecule" includes DNA molecules (e.g., a cDNA or genomic DNA) and RNA molecules (e.g., an mRNA) and analogs of the DNA or RNA generated, e.g., by the use of nucleotide analogs. The nucleic acid molecule can be single-stranded or double-stranded, but preferably is double-stranded DNA.

The term "isolated or purified nucleic acid molecule" includes nucleic acid molecules that are separated from other nucleic acid molecules that are present in the natural source of the nucleic acid. For example, with regards to genomic DNA, the term "isolated" includes nucleic acid molecules that are separated from the chromosome with which the genomic DNA is naturally associated. Preferably, an "isolated" nucleic acid is free of sequences that naturally flank the nucleic acid (i.e., sequences located at the 5'-and/or 3'-ends of the nucleic acid) in the genomic DNA of the organism from which the nucleic acid is derived. For example, in various embodiments, the isolated nucleic acid molecule can contain less than about 5 kilobases, 4 kilobases, 3 kilobases, 2 kilobases, 1 kilobase, 0.5 kilobase or 0.1 kilobase of 5'-and/or 3'-nucleotide sequences which naturally flank the nucleic acid molecule in genomic DNA of the cell from which the nucleic acid is derived. Moreover, an "isolated" nucleic acid molecule, such as a cDNA molecule, can be substantially free of other cellular material, or culture medium when produced by recombinant techniques, or substantially free of chemical precursors or other chemicals when chemically synthesized.

As used herein, the term "hybridizes under stringent conditions" describes conditions for hybridization and washing. Stringent conditions are known to those skilled in the art and can be found in available references (e.g., Current Protocols in Molecular Biology, John Wiley & Sons, N.Y., 1989, 6.3.1-6.3.6). Aqueous and non-aqueous methods are described in that reference and either can be used. A preferred example of stringent hybridization conditions are hybridization in 6× sodium chloride/sodium citrate (SSC) at about 45□C, followed by one or more washes in 0.2× SSC, 0.1% (w/v) SDS at 50°C. Another example of stringent hybridization conditions are hybridization in 6× SSC at about 45□C, followed by one or more washes in 0.2× SSC, 0.1% (w/v) SDS at 55°C. A further example of stringent hybridization conditions are hybridization in 6× SSC at about 45□C, followed by one or more washes in 0.2× SSC, 0.1% (w/v) SDS at 60°C. Preferably, stringent hybridization conditions are hybridization in 6× SSC at about 45□C, followed by one or more washes in 0.2× SSC, 0.1% (w/v) SDS at 65°C. Particularly preferred stringency conditions (and the conditions that should be used if the practitioner is uncertain about what conditions should be applied to determine if a molecule is within a hybridization limitation of the invention) are 0.5 molar sodium phosphate, 7% (w/v) SDS at 65°C, followed by one or more washes at 0.2× SSC, 1% (w/v) SDS at 65°C. Preferably, an isolated nucleic acid molecule of the invention that hybridizes under stringent conditions to the sequence of SEQ ID NO:1 or SEQ ID NO:3, corresponds to a naturally-occurring nucleic acid molecule.

As used herein, a "naturally-occurring" nucleic acid molecule refers to an RNA or DNA molecule having a nucleotide sequence that occurs in nature (e.g., encodes a natural protein).

As used herein, the terms "gene" and "recombinant gene" refer to nucleic acid molecules which include an open reading frame encoding a 58297 protein, preferably a mammalian 58297 protein, and can further include non-coding regulatory sequences and introns.

An "isolated" or "purified" polypeptide or protein is substantially free of cellular material or other contaminating proteins from the cell or tissue source from which the protein is derived, or substantially free from chemical precursors or other chemicals when chemically synthesized. In one embodiment, the language "substantially free" means preparation of 58297 protein having less than about 30%, 20%, 10% and more preferably 5% (by dry weight), of non-58297 protein (also referred to herein as a "contaminating protein"), or of chemical precursors or non-58297 chemicals. When the 58297 protein or biologically active portion thereof is recombinantly produced, it is also preferably substantially free of culture medium, i.e., culture medium represents less than about 20%, more preferably less than about 10%, and most preferably less than about 5% of the volume of the protein preparation. The invention includes isolated or purified preparations of at least 0.01, 0.1, 1.0, and 10 milligrams in dry weight.

A "non-essential" amino acid residue is a residue that can be altered from the wild-type sequence of 58297 (e.g., the sequence of SEQ ID NO:1, SEQ ID NO:3 or the deposited nucleotide sequence) without abolishing or, more preferably, without substantially altering a biological activity, whereas an "essential" amino acid residue results in such a change. For example, amino acid residues that are conserved among the polypeptides of the present invention, e.g., those present in the transmembrane amino acid transporter domain are predicted to be particularly non-amenable to alteration, except that amino acid residues in transmembrane domains can generally be replaced by other residues having approximately equivalent hydrophobicity without significantly altering 58297 activity.

A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), non-polar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). Thus, a nonessential amino acid residue in a 58297 protein is preferably replaced with another amino acid residue from the same side chain family. Alternatively, in another embodiment, mutations can be introduced randomly along all or part of a 58297 coding sequence, such as by saturation mutagenesis, and the resultant mutants can be screened for 58297 biological activity to identify mutants that retain activity. Following mutagenesis of SEQ ID NO:1, SEQ ID NO:3, or the deposited nucleotide sequence, the encoded protein can be expressed recombinantly and the activity of the protein can be determined.

As used herein, a "biologically active portion" of a 58297 protein includes a fragment of a 58297 protein that participates in an interaction between a 58297 molecule and a non-58297 molecule. Biologically active portions of a 58297 protein include peptides comprising amino acid sequences sufficiently homologous to or derived from the amino acid sequence of the 58297 protein, e.g., the amino acid sequence shown in SEQ ID NO:2, which include less amino acids than the full length 58297 proteins, and exhibit at least one activity of a 58297 protein. Typically, biologically active portions comprise a domain or motif with at least one activity of the 58297 protein, e.g., the ability to modulate (e.g., promote, catalyze, regulate, initiate, facilitate, or inhibit) transmembrane transport of amino acids (e.g., amino acids associated with N system transport, e.g., histidine, asparagine, and glutamine) across the plasma membrane, e.g., from an extracellular medium into a cell, or vice versa.

A biologically active portion of a 58297 protein can be a polypeptide that is, for example, 100, 200, 300, or 400 or more amino acids in length. Biologically active portions of a 58297 protein can be used as targets for developing agents that modulate a 58297-mediated activity, e.g., a biological activity described herein.

Calculations of sequence homology or identity (the terms are used interchangeably herein) between sequences are performed as follows.

To determine the percent identity of two amino acid sequences, or of two nucleic acid sequences, the sequences are aligned for optimal comparison purposes (e.g., gaps can be introduced in one or both of a first and a second amino acid or nucleic acid sequence for optimal alignment and non-homologous sequences can be disregarded for comparison purposes). In a preferred embodiment, the length of a reference sequence aligned for comparison purposes is at least 30%, preferably at least 40%, more preferably at least 50%, even more preferably at least 60%, and even more preferably at least 70%, 60%, 70%, 80%, 82%, 84%, 85%, 86%, 87%, 88%, 89%, 90%, 91%, 92%, 93%, 94%, 95%, 96%, 97%, 98%, 99%, or 100% of the length of the reference sequence (e.g., when aligning a second sequence to the 58297 amino acid sequence of SEQ ID NO:2 having 561 amino acid residues, at least 140, preferably at least 165, more preferably at least 210, even more preferably at least 281, and even more preferably at least 290, 300, 310, 340, 370, 400, 430, 450, 455, 460, 465, 470, 480, 500, 520, 540, 550, 560, or 561 amino acid residues are aligned). The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid "identity" is equivalent to amino acid or nucleic acid "homology"). The percent identity between the two sequences is a function of the number of identical positions shared by the sequences, taking into account the number of gaps, and the length of each gap, which need to be introduced for optimal alignment of the two sequences.

The comparison of sequences and determination of percent identity between two sequences can be accomplished using a mathematical algorithm. In a preferred embodiment, the percent identity between two amino acid sequences is determined using the Needleman et al. (1970) *J. Mol. Biol.* 48:444-453) algorithm which has been incorporated into the GAP program in the GCG software package (available at http://www.gcg.com), using either a BLOSUM 62 matrix or a PAM250 matrix, and a gap weight of 16, 14, 12, 10, 8, 6, or 4 and a length weight of 1, 2, 3, 4, 5, or 6. In yet another preferred embodiment, the percent identity between two nucleotide sequences is determined using the GAP program in the GCG software package (available at http://www.gcg.com), using a NWSgapdna.CMP matrix and a gap weight of 40, 50, 60, 70, or 80 and a length weight of 1, 2, 3, 4, 5, or 6. A particularly preferred set of parameters (and the one that should be used if the practitioner is uncertain about what parameters should be applied to determine if a molecule is within a sequence identity or homology limitation of the invention) are a BLOSUM 62 scoring matrix with a gap penalty of 12, a gap extend penalty of 4, and a frameshift gap penalty of 5.

The percent identity between two amino acid or nucleotide sequences can be determined using the algorithm of Meyers et al. (1989) *CABIOS* 4:11-17) which has been incorporated into the ALIGN program (version 2.0), using a PAM120 weight residue table, a gap length penalty of 12 and a gap penalty of 4.

The nucleic acid and protein sequences described herein can be used as a "query sequence" to perform a search against public databases to, for example, identify other family members or related sequences. Such searches can be performed using the NBLAST and XBLAST programs (version 2.0) of Altschul, et al. (1990) *J. Mol. Biol.* 215:403-410). BLAST nucleotide searches can be performed with the NBLAST program, score = 100, wordlength = 12 to obtain nucleotide sequences homologous to 58297 nucleic acid molecules of the invention. BLAST protein searches can be performed with the XBLAST program, score = 50, wordlength = 3 to obtain amino acid sequences homologous to 58297 protein molecules of the invention. To obtain gapped alignments for comparison purposes, gapped BLAST can be utilized as described in Altschul et al. (1997, Nucl. Acids Res. 25:3389-3402). When using BLAST and gapped BLAST programs, the default parameters of the respective programs (e.g., XBLAST and NBLAST) can be used. See <http://www.ncbi.nlm.nih.gov>.

58297 polypeptides of the present invention can have amino acid sequences sufficiently identical to the amino acid sequence of SEQ ID NO:2. The term "sufficiently identical" or "substantially identical" is used herein to refer to a first amino acid or nucleotide sequence that contains a sufficient or minimum number of identical or equivalent (e.g., with a similar side chain) amino acid residues or nucleotides to a second amino acid or nucleotide sequence such that the first and second amino acid or nucleotide sequences have a common structural domain or common functional activity. For example, amino acid or nucleotide sequences that contain a common structural domain having at least about 60%, or 65% identity, likely 75% identity, more likely 85%, 90%. 91%, 92%, 93%, 94%, 95%, 96%, 97%, 98% or 99% identity are defined herein as sufficiently or substantially identical.

"Misexpression or aberrant expression", as used herein, refers to a non-wild type pattern of gene expression, at the RNA or protein level. It includes: expression at non-wild type levels, i.e., over- or under-expression; a pattern of expression that differs from wild type in terms of the time or stage at which the gene is expressed, e.g., increased or decreased expression (as compared with wild type) at a predetermined developmental period or stage; a pattern of expression that differs from wild type in terms of decreased expression (as compared with wild type) in a predetermined cell type or tissue type; a pattern of expression that differs from wild type in terms of the splicing size, amino acid sequence, post-transitional modification, or biological activity of the expressed polypeptide; a pattern of expression that differs from wild type in terms of the effect of an environmental stimulus or extracellular stimulus on expression of the gene, e.g., a pattern of increased or decreased expression (as compared with wild type) in the presence of an increase or decrease in the strength of the stimulus.

"Subject," as used herein, can refer to a mammal, e.g., a human, or to an experimental animal or disease model. The subject can also be a non-human animal, e.g., a horse, cow, goat, or other domestic animal.

A "purified preparation of cells," as used herein, refers to, in the case of plant or animal cells, an in vitro preparation of cells and not an entire intact plant or animal. In the case of cultured cells or microbial cells, it consists of a preparation of at least 10%, and more preferably, 50% of the subject cells.

Various aspects of the invention are described in further detail below.

### Isolated Nucleic Acid Molecules

In one aspect, the invention provides, an isolated or purified, nucleic acid molecule that encodes a 58297 polypeptide described herein, e.g., a full length 58297 protein or a fragment thereof, e.g., a biologically active portion of 58297 protein. Also included is a nucleic acid fragment suitable for use as a hybridization probe, which can be used, e.g., to identify a nucleic acid molecule encoding a polypeptide of the invention, 58297 mRNA, and fragments suitable for use as primers, e.g., PCR primers for the amplification or mutation of nucleic acid molecules.

In one embodiment, an isolated nucleic acid molecule of the invention includes the nucleotide sequence shown in SEQ ID NO:1, or a portion or fragment thereof. In one embodiment, the nucleic acid molecule includes sequences encoding the human 58297 protein (i.e., "the coding region", from nucleotides 128-1810 of SEQ ID NO:1, excluding the termination codon, shown as in SEQ ID NO:3), as well as untranslated (e.g., noncoding) sequences, e.g., 5' untranslated sequence (i.e., nucleotides 1-127 of SEQ ID NO:1) and/or 3' untranslated sequence (i.e., nucleotides 1811-2476 of SEQ ID NO:1). Alternatively, the nucleic acid molecule can include only the coding region of SEQ ID NO:1 (e.g., nucleotides 1 to 1683 of SEQ ID NO:3) and, e.g., no flanking sequences which normally accompany the subject sequence. In another embodiment, the nucleic acid molecule encodes a sequence corresponding to the mature protein of SEQ ID NO:2. In yet another embodiment, the nucleic acid molecule encodes a sequence corresponding to a fragment of the protein from about amino acid 141-551.

In another embodiment, an isolated nucleic acid molecule of the invention includes a nucleic acid molecule which is a complement of the nucleotide sequence shown in SEQ ID NO:1 or SEQ ID NO:3, or a portion or fragment thereof. In other embodiments, the nucleic acid molecule of the invention is sufficiently complementary to the nucleotide sequence shown in SEQ ID NO:1 or SEQ ID NO:3 such that it can hybridize to the nucleotide sequence shown in SEQ ID NO:1 or 3, thereby forming a stable duplex.

In one embodiment, an isolated nucleic acid molecule of the present invention includes a nucleotide sequence which is at least about: 60%, 65%, 70%, 75%, 80%, 85%, 90%, 91%, 92%, 93%, 94%, 95%, 96%, 97%, 98%, 99% or more, homologous to the entire length of the nucleotide sequence shown in SEQ ID NO:1 or SEQ ID NO:3, or a portion or fragment thereof, preferably of the same length, of any of these nucleotide sequences.

### 58297 Nucleic Acid Fragments

A nucleic acid molecule of the invention can include only a portion or fragment of the nucleic acid sequence of SEQ ID NO:1 or 3. For example, such a nucleic acid molecule can include a fragment which can be used as a probe or primer or a fragment encoding a portion of a 58297 protein, e.g., an immunogenic or biologically active portion of a 58297 protein. A fragment can comprise those nucleotides of SEQ ID NO:1 which encode a transmembrane amino acid transporter protein domain of human 58297. The nucleotide sequence determined from the cloning of the 58297 gene allows for the generation of probes and primers designed for use in identifying and/or cloning other 58297 family members, or fragments thereof, as well as 58297 homologues, or fragments thereof, from other species.

In another embodiment, a nucleic acid includes a nucleotide sequence that includes part, or all, of the coding region and extends into either (or both) the 5' or 3' noncoding or untranslated region. Other embodiments include a fragment which includes a nucleotide sequence encoding an amino acid fragment described herein. Nucleic acid fragments can encode a specific domain or site described herein or fragments thereof, particularly fragments thereof which are at least 75 amino acids in length. Fragments also include nucleic acid sequences corresponding to specific amino acid sequences described above or fragments thereof. Nucleic acid fragments should not to be construed as encompassing those fragments that may have been disclosed prior to the invention.

A nucleic acid fragment can include a sequence corresponding to a domain, region, or functional site described herein. A nucleic acid fragment can also include one or more domain, region, or functional site described herein. Thus, for example, a 58297 nucleic acid fragment can include a sequence corresponding to a transmembrane amino acid transporter protein domain.

58297 probes and primers are provided. Typically a probe/primer is an isolated or purified oligonucleotide. The oligonucleotide typically includes a region of nucleotide sequence that hybridizes under stringent conditions to at least about 7, 12 or 15, preferably about 20 or 25, more preferably about 30, 35, 40, 45, 50, 55, 60, 65, or 75 consecutive nucleotides of a sense or antisense sequence of SEQ ID NO:1 or SEQ ID NO:3, or of a naturally occurring allelic variant or mutant of SEQ ID NO:1 or SEQ ID NO:3.

In a preferred embodiment the nucleic acid is a probe which is at least 5 or 10, and less than 200, more preferably less than 100, or less than 50, base pairs in length. It should be identical, or differ by 1, or less than in 5 or 10 bases, from a sequence disclosed herein. If alignment is needed for this comparison the sequences should be aligned for maximum homology. "Looped" out sequences from deletions or insertions, or mismatches, are considered differences.

A probe or primer can be derived from the sense or anti-sense strand of a nucleic acid which encodes a transmembrane amino acid transporter domain (e.g., at about nucleotides 548-1780 of SEQ ID NO:1), or a fragment thereof.

In another embodiment a set of primers is provided, e.g., primers suitable for use in a PCR, which can be used to amplify a selected region of a 58297 sequence, e.g., a domain, region, site or other sequence described herein. The primers should be at least 5, 10, or 50 base pairs in length and less than 100, or less than 200, base pairs in length. The primers should be identical, or differs by one base from a sequence disclosed herein or from a naturally occurring variant. For example, primers suitable for amplifying all or a portion of any of the following regions are provided: a transmembrane amino acid transporter domain from about amino acid 141-551 of SEQ ID NO:2; or transmembrane domains at about amino acid residues 120-138, 145-166, 202-225, 289-305, 314-331, 362-380, 393-415, 439-462, 474-498, 508-528, and 536-553 of SEQ ID NO:2.

A nucleic acid fragment can encode an epitope bearing region of a polypeptide described herein.

A nucleic acid fragment encoding a "biologically active portion of a 58297 polypeptide" can be prepared by isolating a portion of the nucleotide sequence of SEQ ID NO:1 or 3, which encodes a polypeptide having a 58297 biological activity (e.g., the biological activities of the 58297 proteins are described herein), expressing the encoded portion of the 58297 protein (e.g., by recombinant expression *in vitro*) and assessing the activity of the encoded portion of the 58297 protein. For example, a nucleic acid fragment encoding a biologically active portion of 58297 includes a transmembrane amino acid transporter domain, e.g., amino acid residues about 141-551 of SEQ ID NO:2. A nucleic acid fragment encoding a biologically active portion of a 58297 polypeptide, may comprise a nucleotide sequence which is greater than 80 or more nucleotides in length.

In preferred embodiments, a nucleic acid includes a nucleotide sequence which is about 440, 480, 500, 520, 530, 560, 600, 640, 680, 700, 720, 740, 760, 780, 800, 820, 840, 860, 880, 900, 920, 940, 960, 980, 1000, 1020, 1040, 1060, 1080, 1100, 1120, 1140, 1160, 1180, 1200, 1220, 1240, 1260, 1280, 1300, 1340, 1360, 1380, 1400, 1420, 1440, 1460, 1480, 1500, 1520, 1540, 1560, 1580, 1600, 1620, 1640, 1660, 1680, or more nucleotides in length and hybridizes under stringent hybridization conditions to a nucleic acid molecule of SEQ ID NO:1, or SEQ ID NO:3, or a complement thereof.

### 58297 Nucleic Acid Variants

The invention further encompasses nucleic acid molecules that differ from the nucleotide sequence shown in SEQ ID NO:1 or SEQ ID NO:3. Such differences can be due to degeneracy of the genetic code and result in a nucleic acid which encodes the same 58297 proteins as those encoded by the nucleotide sequence disclosed herein. In another embodiment, an isolated nucleic acid molecule of the invention has a nucleotide sequence encoding a protein having an amino acid sequence which differs, by at least 1, but less than 5, 10, 20, 50, or 100 amino acid residues that shown in SEQ ID NO:2. If alignment is needed for this comparison, the sequences should be aligned for maximum homology. "Looped" out sequences from deletions or insertions, or mismatches, are considered differences.

Nucleic acids of the invention can be chosen for having codons which are preferred or non-preferred for a particular expression system. For example, the nucleic acid can be one in which at least one codon, preferably at least 10% or 20% of the codons, has been altered such that the sequence is optimized for expression in bacterial (e.g., *E. coli),* yeast, human, insect, or nonhuman mammalian (e.g., CHO) cells.

Nucleic acid variants can be naturally occurring, such as allelic variants (same locus), homologs (different locus), and orthologs (different organism) or can be non-naturally occurring. Non-naturally occurring variants can be made by mutagenesis techniques, including those applied to polynucleotides, cells, or organisms. The variants can contain nucleotide substitutions, deletions, inversions and insertions. Variation can occur in either or both the coding and non-coding regions. The variations can produce both conservative and non-conservative amino acid substitutions (as compared in the encoded product).

In a preferred embodiment, the nucleic acid differs from that of SEQ ID NO:1 or 3, e.g., as follows: by at least one but less than 10, 20, 30, or 40 nucleotides; at least one, but less than 1%, 5%, 10% or 20%, of the nucleotides in the subject nucleic acid. If necessary for this analysis the sequences should be aligned for maximum homology. "Looped" out sequences from deletions or insertions, or mismatches, are considered differences.

Orthologs, homologs, and allelic variants can be identified using methods known in the art. These variants comprise a nucleotide sequence encoding a polypeptide that is 50%, at least about 55%, typically at least about 70-75%, more typically at least about 80-85%, and most typically at least about 90-95% or more, identical to the nucleotide sequence shown in SEQ ID NO:2, or a fragment of this sequence. Such nucleic acid molecules can readily be identified as being able to hybridize under stringent conditions to the nucleotide sequence shown in SEQ ID NO:2, or a fragment of the sequence. Nucleic acid molecules corresponding to orthologs, homologs, and allelic variants of the 58297 cDNAs of the invention can further be isolated by mapping to the same chromosome or locus as the 58297 gene.

Preferred variants include those that are correlated with at least one of the following 58297 biological activities:

(1) the ability to modulate (e.g., promote, catalyze, regulate, initiate, facilitate or inhibit) transmembrane transport of amino acids (e.g., amino acids associated with N system transport, e.g., histidine, asparagine, and glutamine) across the plasma membrane, e.g., from an extracellular medium into a cell, or vice versa; (2) the ability to modulate (e.g., promote, regulate, initiate, facilitate or inhibit) cellular nutrition, e.g., by modulating the transport of amino acids, e.g., amino acids associated with N system transport; (3) the ability to modulate (e.g., promote, regulate, initiate, facilitate or inhibit) protein biosynthesis, e.g., by modulating the transport of amino acids, e.g., amino acids associated with N system transport; (4) the ability to modulate (e.g., promote, regulate, initiate, facilitate or inhibit) hormone metabolism, e.g., by modulating the transport of amino acids, e.g., amino acids associated with N system transport; (5) the ability to modulate (e.g., promote, regulate, initiate, facilitate or inhibit) cell growth, e.g., by modulating the transport of amino acids, e.g., amino acids associated with N system transport; (6) the ability to modulate (e.g., promote, regulate, initiate, facilitate or inhibit) metabolic energy production, e.g., by modulating the transport of amino acids, e.g., amino acids associated with N system transport; and (7) the ability to modulate (e.g., promote, regulate, initiate, facilitate or inhibit) CNS signal transduction/transmission, e.g., by modulating reuptake form the synaptic cleft, or by supplying CNS precursors (e.g., by modulating the transport of amino acids, e.g., amino acids associated with N system transport).

Other activities of the 58297 proteins of the present invention include one or more of the following:

(1) the ability to modulate (e.g., promote, catalyze, regulate, initiate, facilitate or inhibit) ammonia metabolism, e.g., via cellular uptake of glutamine; (2) the ability to modulate (e.g., promote, catalyze, regulate, initiate, facilitate or inhibit) synthesis of purines and/or pyrimidines, e.g., via cellular uptake of glutamine; (3) the ability to modulate (e.g., maintain, promote, or arrest) the glutamine-glutamate cycle (e.g., that which occurs between neurons and glial cells in the brain, between placenta and liver in the developing fetus, and in the liver, between periportal hepatocytes and perivenous hepatocytes), e.g., via cellular uptake of glutamine; and (4) the ability to modulate (e.g., promote, catalyze, regulate, initiate, facilitate or inhibit) CNS-related disorders (e.g., amyotrophic later sclerosis, Alzheimer's disease) (e.g., modulate the onset, continuation, or cessation of), e.g., via cellular uptake of glutamine, as glutamate can be a excitotoxin which contributes to the death of nerve cells in a variety of neurodegenerative disorders.

Allelic variants of 58297, e.g., human 58297, include both functional and non-functional proteins. Functional allelic variants are naturally occurring amino acid sequence variants of the 58297 protein within a population that maintain the 58297 biological activities described herein.

Functional allelic variants will typically contain only conservative substitution of one or more amino acids of SEQ ID NO:2, or substitution, deletion or insertion of non-critical residues in non-critical regions of the protein. Non-functional allelic variants are naturally-occurring amino acid sequence variants of the 58297, e.g., human 58297, protein within a population that do not have the ability to (1) modulate (e.g., promote, catalyze, regulate, initiate, facilitate or inhibit) transmembrane transport of amino acids (e.g., amino acids associated with N system transport, e.g., histidine, asparagine, and glutamine) across the plasma membrane, e.g., from an extracellular medium into a cell, or vice versa; (2) modulate (e.g., promote, regulate, initiate, facilitate or inhibit) cellular nutrition, e.g., by modulating the transport of amino acids, e.g., amino acids associated with N system transport; (3) modulate (e.g., promote, regulate, initiate, facilitate or inhibit) protein biosynthesis, e.g., by modulating the transport of amino acids, e.g., amino acids associated with N system transport; (4) modulate (e.g., promote, regulate, initiate, facilitate or inhibit) hormone metabolism, e.g., by modulating the transport of amino acids, e.g., amino acids associated with N system transport; (5) modulate (e.g., promote, regulate, initiate, facilitate or inhibit) cell growth, e.g., by modulating the transport of amino acids, e.g., amino acids associated with N system transport; (6) modulate (e.g., promote, regulate, initiate, facilitate or inhibit) metabolic energy production, e.g., by modulating the transport of amino acids, e.g., amino acids associated with N system transport; and (7) modulate (e.g., promote, regulate, initiate, facilitate or inhibit) CNS signal transduction/transmission, e.g., by modulating reuptake form the synaptic cleft, or by supplying CNS precursors (e.g., by modulating the transport of amino acids, e.g., amino acids associated with N system transport).

Non-functional allelic variants can typically contain a non-conservative substitution, a deletion, or insertion, or premature truncation of the amino acid sequence of SEQ ID NO:2, or a substitution, insertion, or deletion in critical residues or critical regions of the protein.

Moreover, nucleic acid molecules encoding other 58297 family members and, thus, which have a nucleotide sequence which differs from the 58297 sequences of SEQ ID NO:1 or SEQ ID NO:3 are intended to be within the scope of the invention.

### 1.1 Antisense Nucleic Acid Molecules, Ribozymes and Modified 58297 Nucleic Acid Molecules

In another aspect, the invention features, an isolated nucleic acid molecule which is antisense to 58297. An "antisense" nucleic acid can include a nucleotide sequence which is complementary to a "sense" nucleic acid encoding a protein, e.g., complementary to the coding strand of a double-stranded cDNA molecule or complementary to an mRNA sequence. The antisense nucleic acid can be complementary to an entire 58297 coding strand, or to only a portion thereof (e.g., the coding region of human 58297 corresponding to SEQ ID NO:3). In another embodiment, the antisense nucleic acid molecule is antisense to a "noncoding region" of the coding strand of a nucleotide sequence encoding 58297 (e.g., the 5' and 3' untranslated regions).

An antisense nucleic acid can be designed such that it is complementary to the entire coding region of 58297 mRNA, but more preferably is an oligonucleotide which is antisense to only a portion of the coding or noncoding region of 58297 mRNA. For example, the antisense oligonucleotide can be complementary to the region surrounding the translation start site of 58297 mRNA, e.g., between the -10 and +10 regions of the target gene nucleotide sequence of interest. An antisense oligonucleotide can be, for example, about 7, 10, 15, 20, 25, 30, 35, 40, 45, 50, 55, 60, 65, 70, 75, 80, or more nucleotides in length.

An antisense nucleic acid of the invention can be constructed using chemical synthesis and enzymatic ligation reactions using procedures known in the art. For example, an antisense nucleic acid (e.g., an antisense oligonucleotide) can be chemically synthesized using naturally occurring nucleotides or variously modified nucleotides designed to increase the biological stability of the molecules or to increase the physical stability of the duplex formed between the antisense and sense nucleic acids, e.g., phosphorothioate derivatives and acridine substituted nucleotides can be used. The antisense nucleic acid also can be produced biologically using an expression vector into which a nucleic acid has been subcloned in an antisense orientation (i.e., RNA transcribed from the inserted nucleic acid will be of an antisense orientation to a target nucleic acid of interest, described further in the following subsection).

The antisense nucleic acid molecules of the invention are typically administered to a subject (e.g., by direct injection at a tissue site), or generated *in situ* such that they hybridize with or bind to cellular mRNA and/or genomic DNA encoding a 58297 protein to thereby inhibit expression of the protein, e.g., by inhibiting transcription and/or translation. Alternatively, antisense nucleic acid molecules can be modified to target selected cells and then administered systemically. For systemic administration, antisense molecules can be modified such that they specifically bind to receptors or antigens expressed on a selected cell surface, e.g., by linking the antisense nucleic acid molecules to peptides or antibodies which bind to cell surface receptors or antigens. The antisense nucleic acid molecules can also be delivered to cells using the vectors described herein. To achieve sufficient intracellular concentrations of the antisense molecules, vector constructs in which the antisense nucleic acid molecule is placed under the control of a strong pol II or pol III promoter are preferred.

In yet another embodiment, the antisense nucleic acid molecule of the invention is an α-anomeric nucleic acid molecule. An α-anomeric nucleic acid molecule forms specific double-stranded hybrids with complementary RNA in which, contrary to the usual β-units, the strands run parallel to each other (Gaultier et al. (1987) *Nucleic Acids. Res.* 15:6625-6641). The antisense nucleic acid molecule can also comprise a 2'-o-methylribonucleotide (Inoue et al. (1987) *Nucleic Acids Res.* 15:6131-6148) or a chimeric RNA-DNA analogue (Inoue et al. (1987) *FEBS Lett.* 215:327-330).

In still another embodiment, an antisense nucleic acid of the invention is a ribozyme. A ribozyme having specificity for a 58297-encoding nucleic acid can include one or more sequences complementary to the nucleotide sequence of a 58297 cDNA disclosed herein (i.e., SEQ ID NO:1 or SEQ ID NO:3), and a sequence having known catalytic sequence responsible for mRNA cleavage (see U.S. Pat. No. 5,093,246 or Haselhoff and Gerlach (1988) *Nature* 334:585-591). For example, a derivative of a *Tetrahymena* L-19 IVS RNA can be constructed in which the nucleotide sequence of the active site is complementary to the nucleotide sequence to be cleaved in a 58297-encoding mRNA. See, e.g., Cech et al. U.S. Patent No. 4,987,071; and Cech et al. U.S. Patent No. 5,116,742. Alternatively, 58297 mRNA can be used to select a catalytic RNA having a specific ribonuclease activity from a pool of RNA molecules. See, e.g., Bartel, D. and Szostak, J.W. (1993) *Science* 261:1411-1418.

58297 gene expression can be inhibited by targeting nucleotide sequences complementary to the regulatory region of the 58297 (e.g., the 58297 promoter and/or enhancers) to form triple helical structures that prevent transcription of the 58297 gene in target cells. See generally, Helene, C. (1991) *Anticancer Drug Des.* 6:569-84; Helene, C. i (1992) *Ann. N.Y. Acad. Sci.* 660:27-36; and Maher, L.J. (1992) *Bioassays* 14:807-15. The potential sequences that can be targeted for triple helix formation can be increased by creating a so called "switchback" nucleic acid molecule. Switchback molecules are synthesized in an alternating 5'-3', 3'-5' manner, such that they base pair with first one strand of a duplex and then the other, eliminating the necessity for a sizeable stretch of either purines or pyrimidines to be present on one strand of a duplex.

The invention also provides detectably labeled oligonucleotide primer and probe molecules. Typically, such labels are chemiluminescent, fluorescent, radioactive, or colorimetric.

A 58297 nucleic acid molecule can be modified at the base moiety, sugar moiety or phosphate backbone to improve, e.g., the stability, hybridization, or solubility of the molecule. For example, the deoxyribose phosphate backbone of the nucleic acid molecules can be modified to generate peptide nucleic acids (see Hyrup B. et al. (1996) *Bioorganic & Medicinal Chemistry* 4: 5-23). As used herein, the terms "peptide nucleic acid" or "PNA" refers to a nucleic acid mimic, e.g., a DNA mimic, in which the deoxyribose phosphate backbone is replaced by a pseudopeptide backbone and only the four natural nucleobases are retained. The neutral backbone of a PNA can allow for specific hybridization to DNA and RNA under conditions of low ionic strength. The synthesis of PNA oligomers can be performed using standard solid phase peptide synthesis protocols as described in Hyrup B. et al. (1996) *supra*; Perry-O'Keefe et al. *Proc. Natl. Acad. Sci.* 93: 14670-675.

PNAs of 58297 nucleic acid molecules can be used in therapeutic and diagnostic applications. For example, PNAs can be used as antisense or antigene agents for sequence-specific modulation of gene expression by, for example, inducing transcription or translation arrest or inhibiting replication. PNAs of 58297 nucleic acid molecules can also be used in the analysis of single base pair mutations in a gene, (e.g., by PNA-directed PCR clamping); as 'artificial restriction enzymes' when used in combination with other enzymes, (e.g., S1 nucleases (Hyrup B. et al. (1996) *supra*)); or as probes or primers for DNA sequencing or hybridization (Hyrup B. et al. (1996) *supra;* Perry-O'Keefe *supra*).

In other embodiments, the oligonucleotide may include other appended groups such as peptides (e.g., for targeting host cell receptors *in vivo*), or agents facilitating transport across the cell membrane (see, e.g., Letsinger et al. (1989) *Proc. Natl. Acad. Sci. USA* 86:6553-6556; Lemaitre et al. (1987) *Proc. Natl. Acad. Sci. USA* 84:648-652; PCT Publication No. W088/09810) or the blood-brain barrier (see, e.g., PCT Publication No. W089/10134). In addition, oligonucleotides can be modified with hybridization-triggered cleavage agents (see, e.g., Krol et al. (1988) *Bio-Techniques* 6:958-976) or intercalating agents. (see, e.g., Zon (1988) *Pharm. Res.* 5:539-549). To this end, the oligonucleotide may be conjugated to another molecule, (e.g., a peptide, hybridization triggered cross-linking agent, transport agent, or hybridization-triggered cleavage agent).

The invention also includes molecular beacon oligonucleotide primer and probe molecules having at least one region which is complementary to a 58297 nucleic acid of the invention, two complementary regions one having a fluorophore and one a quencher such that the molecular beacon is useful for quantitating the presence of the 58297 nucleic acid of the invention in a sample. Molecular beacon nucleic acids are described, for example, in Lizardi et al., U.S. Patent No. 5,854,033; Nazarenko et al., U.S. Patent No. 5,866,336, and Livak et al., U.S. Patent 5,876,930.

### Isolated 58297 Polypeptides

In another aspect, the invention features, an isolated 58297 protein, or fragment, e.g., a biologically active portion, for use as immunogens or antigens to raise or test (or more generally to bind) anti-58297 antibodies. 58297 protein can be isolated from cells or tissue sources using standard protein purification techniques. 58297 protein, or fragments thereof, can be produced by recombinant DNA techniques or synthesized chemically.

Polypeptides of the invention include those which arise as a result of the existence of multiple genes, alternative transcription events, alternative RNA splicing events, and alternative translational and post-translational events. The polypeptide can be expressed in systems, e.g., cultured cells, which result in substantially the same post-translational modifications present when expressed the polypeptide is expressed in a native cell, or in systems which result in the alteration or omission of post-translational modifications, e.g., glycosylation or cleavage, present when expressed in a native cell.

In a preferred embodiment, a 58297 polypeptide has one or more of the following characteristics:

the ability to: (1) modulate (e.g., promote, catalyze, regulate, initiate, facilitate or inhibit) transmembrane transport of amino acids (e.g., amino acids associated with N system transport, e.g., histidine, asparagine, and glutamine) across the plasma membrane, e.g., from an extracellular medium into a cell, or vice versa; (2) modulate (e.g., promote, regulate, initiate, facilitate or inhibit) cellular nutrition, e.g., by modulating the transport of amino acids, e.g., amino acids associated with N system transport; (3) modulate (e.g., promote, regulate, initiate, facilitate or inhibit) protein biosynthesis, e.g., by modulating the transport of amino acids, e.g., amino acids associated with N system transport; (4) modulate (e.g., promote, regulate, initiate, facilitate or inhibit) hormone metabolism, e.g., by modulating the transport of amino acids, e.g., amino acids associated with N system transport; (5) modulate (e.g., promote, regulate, initiate, facilitate or inhibit) cell growth, e.g., by modulating the transport of amino acids, e.g., amino acids associated with N system transport; (6) modulate (e.g., promote, regulate, initiate, facilitate or inhibit) metabolic energy production, e.g., by modulating the transport of amino acids, e.g., amino acids associated with N system transport; and (7) modulate (e.g., promote, regulate, initiate, facilitate or inhibit) CNS signal transduction/transmission, e.g., by modulating reuptake form the synaptic cleft, or by supplying CNS precursors (e.g., by modulating the transport of amino acids, e.g., amino acids associated with N system transport).

the ability to (1) modulate (e.g., promote, catalyze, regulate, initiate, facilitate or inhibit) ammonia metabolism, e.g., via cellular uptake of glutamine; (2) modulate (e.g., promote, catalyze, regulate, initiate, facilitate or inhibit) synthesis of purines and/or pyrimidines, e.g., via cellular uptake of glutamine; (3) modulate (e.g., maintain, promote, or arrest) the glutamine-glutamate cycle (e.g., that which occurs between neurons and glial cells in the brain, between placenta and liver in the developing fetus, and in the liver, between periportal hepatocytes and perivenous hepatocytes), e.g., via cellular uptake of glutamine; and (4) modulate (e.g., promote, catalyze, regulate, initiate, facilitate or inhibit) CNS-related disorders (e.g., amyotrophic later sclerosis, Alzheimer's disease) (e.g., modulate the onset, continuation, or cessation of), e.g., via cellular uptake of glutamine, as glutamate can be a excitotoxin which contributes to the death of nerve cells in a variety of neurodegenerative disorders.

a molecular weight, e.g., a deduced molecular weight (e.g., of 63.8 kDa), preferably ignoring any contribution of post translational modifications, amino acid composition or other physical characteristic of SEQ ID NO:2;

a mapping position, e.g., a mapping position deduced by comparing 58297 sequence with sequences of known positions in the genome. 58297 maps to chromosome 5, based on at least several regions of homology to a human chromosome 5 clone in the art (clone CTD-2028D11, Genbank accession number AC008784), including by way of example: 99% homology over 812 base pairs (the reverse complement of base pairs 1646-2457) of SEQ ID NO:1; 99% homology over 201 base pairs (the reverse complement of base pairs 882-1082) of SEQ ID NO:1; and 97% homology over 173 base pairs (the reverse complement of base pairs 826-654) of SEQ ID NO:1.

an overall sequence similarity of at least 50%, preferably at least 60%, more preferably at least 70, 80, 90, or 95%, with a polypeptide of SEQ ID NO:2;

expression in at least liver, CNS-associated tissues (e.g., brain and spinal fluid); skeletal muscle; testes; and neutrophils; and

a transmembrane amino acid transporter domain which is preferably about 70%, 80%, 82%, 84%, 85%, 86%, 87%, 88%, 89%, 90%, 91%, 92%, 93%, 94%, 95%, 96%, 97%, 98%, 99%, or 100% identical with the sequence containing amino acid residues about 141 to 551 of SEQ ID NO:2.

In a preferred embodiment, the 58297 protein, or a fragment thereof, differs from the corresponding sequence in SEQ ID NO:2. In one embodiment it differs by at least one, but by less than 15, 10 or 5, amino acid residues. In another it differs from the corresponding sequence in SEQ ID NO:2 by at least one residue, but less than 20%, 15%, 10% or 5%, of the residues in it differ from the corresponding sequence in SEQ ID NO:2. (If this comparison requires alignment the sequences should be aligned for maximum homology. "Looped" out sequences from deletions or insertions, or mismatches, are considered differences.) The differences are, preferably, differences or changes at a nonessential residue or a conservative substitution. In a preferred embodiment the differences are not in the transmembrane amino acid transporter domain. In another preferred embodiment one or more differences are in the transmembrane amino acid transporter domain.

Other embodiments include a protein that contains one or more changes in amino acid sequence, e.g., a change in an amino acid residue which is not essential for activity. Such 58297 proteins differ in amino acid sequence from SEQ ID NO:2, yet retain biological activity.

In one embodiment, the protein includes an amino acid sequence at least about 50%, 55%, 60%, 65%, 70%, 75%, 60%, 70%, 80%, 82%, 84%, 85%, 86%, 87%, 88%, 89%, 90%, 91%, 92%, 93%, 94%, 95%, 96%, 97%, 98%, or more, homologous to SEQ ID NO:2.

A 58297 protein or fragment is provided which varies from the sequence of SEQ ID NO:2 in regions that do not correspond to a domain specifically defined herein (e.g., from about amino acids 1 to 140 or 551 to 561) by at least one, but by less than 15, 10 or 5, amino acid residues in the protein or fragment, but which does not differ from the sequence of SEQ ID NO:2 in regions that correspond to a domain specifically defined herein (e.g., from about amino acids about 141 to 551). (If this comparison requires alignment the sequences should be aligned for maximum homology. "Looped" out sequences from deletions or insertions, or mismatches, are considered differences.) In some embodiments the difference is at a non-essential residue or is a conservative substitution, while in others the difference is at an essential residue or is a non-conservative substitution.

In one embodiment, a biologically active portion of a 58297 protein includes a transmembrane amino acid transporter domain. Moreover, other biologically active portions, in which other regions of the protein are deleted, can be prepared by recombinant techniques and evaluated for one or more of the functional activities of a native 58297 protein.

In a preferred embodiment, the 58297 protein has an amino acid sequence shown in SEQ ID NO:2. In other embodiments, the 58297 protein is substantially identical to SEQ ID NO:2. In yet another embodiment, the 58297 protein is substantially identical to SEQ ID NO:2 and retains the functional activity of the protein of SEQ ID NO:2, as described herein.

### 1.2 58297 Chimeric or Fusion Proteins

In another aspect, the invention provides 58297 chimeric or fusion proteins. As used herein, a 58297 "chimeric protein" or "fusion protein" includes a 58297 polypeptide linked to a non-58297 polypeptide. A "non-58297 polypeptide" refers to a polypeptide having an amino acid sequence corresponding to a protein which is not substantially homologous to the 58297 protein, e.g., a protein which is different from the 58297 protein and which is derived from the same or a different organism. The 58297 polypeptide of the fusion protein can correspond to all or a portion, e.g., a fragment, described herein of a 58297 amino acid sequence. In a preferred embodiment, a 58297 fusion protein includes at least one (or two) biologically active portion of a 58297 protein. The non-58297 polypeptide can be fused to the N-terminus or C-terminus of the 58297 polypeptide.

One useful fusion protein is a GST fusion protein in which the polypeptide of the invention is fused with the carboxyl terminus of GST sequences. Such fusion proteins can facilitate purification of a recombinant polypeptide of the invention.

In another embodiment, the fusion protein contains a heterologous signal sequence at its amino terminus. For example, the native signal sequence of a polypeptide of the invention can be removed and replaced with a signal sequence from another protein. For example, the gp67 secretory sequence of the baculovirus envelope protein can be used as a heterologous signal sequence (Current Protocols in Molecular Biology, Ausubel et al., eds., John Wiley & Sons, 1992).
Other examples of eukaryotic heterologous signal sequences include the secretory sequences of melittin and human placental alkaline phosphatase (Stratagene; La Jolla, California). In yet another example, useful prokaryotic heterologous signal sequences include the phoA secretory signal (Sambrook et al., supra) and the protein A secretory signal (Pharmacia Biotech; Piscataway, New Jersey).

Fusion proteins can include all or a part of a serum protein, e.g., a portion of an immunoglobulin protein (e.g., IgG, IgA, or IgE); an Fc region; and/or the hinge C1 and C2 sequences of an immunoglobulin or human serum albumin.

Moreover, the immunoglobulin fusion proteins of the invention can be used as immunogens to produce anti-58297 antibodies directed against a polypeptide of the invention in a subject, to purify 58297 ligands and in screening assays to identify molecules which inhibit the interaction of 58297 receptors with 58297 ligands. The immunoglobulin fusion protein can, for example, comprise a portion of a polypeptide of the invention fused with the amino-terminus or the carboxyl-terminus of an immunoglobulin constant region, as disclosed in U.S. Patent No. 5,714,147, U.S. Patent No. 5,116,964, U.S. Patent No. 5,514,582, and U.S. Patent No. 5,455,165.

The immunoglobulin fusion proteins of the invention can be incorporated into pharmaceutical compositions and administered to a subject to inhibit an interaction between a ligand (soluble or membrane-bound) and a protein on the surface of a cell (receptor), to thereby suppress signal transduction in vivo. The immunoglobulin fusion protein can be used to affect the bioavailability of a cognate ligand of a polypeptide of the invention. Inhibition of ligand / receptor interaction can be useful therapeutically, both for treating disorders caused by, for example: (i) aberrant modification or mutation of a gene encoding a 58297 protein; (ii) mis-regulation of the 58297 gene; and (iii) aberrant post-translational modification of a 58297 protein.

Chimeric and fusion proteins of the invention can be produced by standard recombinant DNA techniques. In another embodiment, the fusion gene can be synthesized by conventional techniques including automated DNA synthesizers. Alternatively, PCR amplification of gene fragments can be performed using anchor primers which give rise to complementary overhangs between two consecutive gene fragments and which can subsequently be annealed and re-amplified to generate a chimeric gene sequence (see, e.g., Ausubel et al., supra). Moreover, many expression vectors are commercially available that already encode a fusion moiety (e.g., a GST polypeptide). A nucleic acid encoding a polypeptide of the invention can be cloned into such an expression vector such that the fusion moiety is linked in-frame to the polypeptide of the invention.

### Variants of 58297 Proteins

In another aspect, the invention also features a variant of a 58297 polypeptide, e.g., which functions as an agonist (mimetics) or as an antagonist. Variants of the 58297 proteins can be generated by mutagenesis, e.g., discrete point mutation, the insertion or deletion of sequences or the truncation of a 58297 protein. An agonist of the 58297 proteins can retain substantially the same, or a subset, of the biological activities of the naturally occurring form of a 58297 protein. An antagonist of a 58297 protein can inhibit one or more of the activities of the naturally occurring form of the 58297 protein by, for example, competitively modulating a 58297-mediated activity of a 58297 protein. Thus, specific biological effects can be elicited by treatment with a variant of limited function. Preferably, treatment of a subject with a variant having a subset of the biological activities of the naturally occurring form of the protein has fewer side effects in a subject relative to treatment with the naturally occurring form of the 58297 protein.

Variants of a protein of the invention which function as either agonists (e.g., mimetics) or as antagonists can be identified by screening combinatorial libraries of mutants, e.g., truncation mutants, of the 58297 protein for agonist or antagonist activity. In one embodiment, a variegated library of variants is generated by combinatorial mutagenesis at the nucleic acid level and is encoded by a variegated gene library. A variegated library of variants can be produced by, for example, enzymatically ligating a mixture of synthetic oligonucleotides into gene sequences such that a degenerate set of potential protein sequences can be expressed as individual polypeptides, or alternatively, as a set of larger fusion proteins (e.g., for phage display). There are a variety of methods which can be used to produce libraries of potential variants of the polypeptides of the invention from a degenerate oligonucleotide sequence. Methods for synthesizing degenerate oligonucleotides are known in the art (see, e.g., Narang (1983) Tetrahedron 39:3; Itakura et al. (1984) Annu. Rev. Biochem. 53:323; Itakura et al. (1984) Science 198:1056; Ike et al. (1983) Nucleic Acid Res. 11:477).

In addition, libraries of fragments of the coding sequence of a polypeptide of the invention can be used to generate a variegated population of polypeptides for screening and subsequent selection of variants. For example, a library of coding sequence fragments can be generated by treating a double stranded PCR fragment of the coding sequence of interest with a nuclease under conditions wherein nicking occurs only about once per molecule, denaturing the double stranded DNA, re-naturing the DNA to form double stranded DNA which can include sense / antisense pairs from different nicked products, removing single stranded portions from reformed duplexes by treatment with S1 nuclease, and ligating the resulting fragment library into an expression vector. By this method, an expression library can be derived which encodes amino terminal and internal fragments of various sizes of the protein of interest.

Variants in which a cysteine residues is added or deleted or in which a residue which is glycosylated is added or deleted are particularly preferred.

Methods for screening gene products of combinatorial libraries made by point mutations or truncation, and for screening cDNA libraries for gene products having a selected property. Recursive ensemble mutagenesis (REM), a new technique which enhances the frequency of functional mutants in the libraries, can be used in combination with the screening assays to identify 58297 variants (Arkin and Yourvan (1992) *Proc. Natl. Acad. Sci. USA 8*9:7811-7815; Delgrave et al. (1993) *Protein Engineering* 6:327-331).

Cell based assays can be exploited to analyze a variegated 58297 library. For example, a library of expression vectors can be transfected into a cell line, e.g., a cell line, which ordinarily responds to 58297 in a substrate-dependent manner. The transfected cells are then contacted with 58297 and the effect of the expression of the mutant on signaling by the 58297 substrate can be detected, for example, by assaying (i) the interaction of a 58297 protein with a 58297 target molecule; (ii) the interaction of a 58297 protein with a 58297 target molecule, wherein the 58297 target is a ligand, e.g., phosphorylated amino acid residue of a phosphorylated protein (e.g., a phosphatase, for example, a cell cycle regulatory phosphatase, e.g., Cdc25A phosphatase, a cell cycle phosphatase which regulates the G1/S-phas transition, a 58297ase), a kinase (e.g., MAP kinase); (iii) the interaction of a 58297 protein with a 58297 target molecule, wherein the 58297 target is a receptor, e.g., insulin receptor, insulin receptor substrate 1; or (iv) the interaction of a 58297 protein with a 58297 target molecule, wherein the 58297 target is a viral protein, e.g., *vaccinia* viral transcription-mediating proteins, Myxoma viral proteins, Shope Fibroma viral proteins, *Leishmania donovani, Trypanosoma brucei* and *Trypanosoma cruzi* viral proteins. Plasmid DNA can then be recovered from the cells which score for inhibition, or alternatively, potentiation of signaling by the 58297 substrate, and the individual clones further characterized.

In another aspect, the invention features a method of making a 58297 polypeptide, e.g., a peptide having a non-wild type activity, e.g., an antagonist, agonist, or super agonist of a naturally occurring 58297 polypeptide, e.g., a naturally occurring 58297 polypeptide. The method includes: altering the sequence of a 58297 polypeptide, e.g., altering the sequence, e.g., by substitution or deletion of one or more residues of a non-conserved region, a domain or residue disclosed herein, and testing the altered polypeptide for the desired activity.

In another aspect, the invention features a method of making a fragment or analog of a 58297 polypeptide a biological activity of a naturally occurring 58297 polypeptide. The method includes: altering the sequence, e.g., by substitution or deletion of one or more residues, of a 58297 polypeptide, e.g., altering the sequence of a non-conserved region, or a domain or residue described herein, and testing the altered polypeptide for the desired activity.

### Anti-58297 Antibodies

In another aspect, the invention provides an anti-58297 antibody. The term "antibody" as used herein refers to an immunoglobulin molecule and immunologically active portions of immunoglobulin molecules, i.e., molecules that contain an antigen binding site which specifically binds (immunoreacts with) an antigen, such as a 58297 molecule. Examples of immunologically active portions of immunoglobulin molecules include scFV and dcFV fragments, Fab and F(ab')₂ fragments which can be generated by treating the antibody with an enzyme such as papain or pepsin, respectively.

The antibody can be a polyclonal, monoclonal, recombinant, e.g., a chimeric, humanized, fully human, non-human (*e.g*., murine, rat, rabbit, or goat), or single chain antibody. In a preferred embodiment it has effector function and can fix complement. The antibody can be coupled to a toxin or imaging agent.

The term "monoclonal antibody" or "monoclonal antibody composition", as used herein, refers to a population of antibody molecules that contain only one species of an antigen binding site capable of immunoreacting with a particular epitope of 58297. A monoclonal antibody composition thus typically displays a single binding affinity for a particular 58297 protein with which it immunoreacts.

Polyclonal anti-58297 antibodies can be prepared as described above by immunizing a suitable subject with a 58297 immunogen. The anti-58297 antibody titer in the immunized subject can be monitored over time by standard techniques, such as with an enzyme linked immunosorbent assay (ELISA) using immobilized 58297. If desired, the antibody molecules directed against 58297 can be isolated from the mammal (e.g., from the blood) and further purified by well known techniques, such as protein A chromatography to obtain the IgG fraction. At an appropriate time after immunization, *e.g*., when the anti-58297 antibody titers are highest, antibody-producing cells can be obtained from the subject and used to prepare monoclonal antibodies by standard techniques, such as the hybridoma technique originally described by Kohler and Milstein (1975) *Nature* 256:495-497) (see also, Brown et al. (1981) *J. Immunol*. 127:539-46; Brown et al. (1980) *J. Biol. Chem* .255:4980-83; Yeh et al. (1976) *Proc. Natl. Acad. Sci. USA* 76:2927-31; and Yeh et al. (1982) *Int. J. Cancer* 29:269-75), the more recent human B cell hybridoma technique (Kozbor et al. (1983) *Immunol Today* 4:72), the EBV-hybridoma technique (Cole et al. (1985), *Monoclonal Antibodies and Cancer Therapy,* Alan R. Liss, Inc., pp. 77-96) or trioma techniques. The technology for producing monoclonal antibody hybridomas is well known (see generally R. H. Kenneth, in *Monoclonal Antibodies: A New Dimension In Biological Analyses,* Plenum Publishing Corp., New York, New York (1980); E. A. Lerner (1981) *Yale J. Biol. Med.,* 54:387-402; M. L. Gefter et al. (1977) *Somatic Cell Genet.* 3:231-36). Briefly, an immortal cell line (typically a myeloma) is fused to lymphocytes (typically splenocytes) from a mammal immunized with a 58297 immunogen as described above, and the culture supernatants of the resulting hybridoma cells are screened to identify a hybridoma producing a monoclonal antibody that binds 58297.

Any of the many well known protocols used for fusing lymphocytes and immortalized cell lines can be applied for the purpose of generating an anti-58297 monoclonal antibody (see, *e.g*., G. Galfre et al. (1977) *Nature* 266:55052; Gefter et al. *Somatic Cell Genet.,* cited *supra;* Lerner, *Yale J. Biol. Med.,* cited *supra;* Kenneth, *Monoclonal Antibodies,* cited *supra*). Moreover, the ordinarily skilled worker will appreciate that there are many variations of such methods which also would be useful. Typically, the immortal cell line (*e.g.*, a myeloma cell line) is derived from the same mammalian species as the lymphocytes. For example, murine hybridomas can be made by fusing lymphocytes from a mouse immunized with an immunogenic preparation of the present invention with an immortalized mouse cell line. Preferred immortal cell lines are mouse myeloma cell lines that are sensitive to culture medium containing hypoxanthine, aminopterin and thymidine ("HAT medium"). Any of a number of myeloma cell lines can be used as a fusion partner according to standard techniques, *e.g.*, the P3-NS1/1-Ag4-1, P3-x63-Ag8.653 or Sp2/O-Ag14 myeloma lines. These myeloma lines are available from ATCC. Typically, HAT-sensitive mouse myeloma cells are fused to mouse splenocytes using polyethylene glycol ("PEG"). Hybridoma cells resulting from the fusion are then selected using HAT medium, which kills unfused and unproductively fused myeloma cells (unfused splenocytes die after several days because they are not transformed). Hybridoma cells producing a monoclonal antibody of the invention are detected by screening the hybridoma culture supernatants for antibodies that bind 58297, *e.g.*, using a standard ELISA assay.

Alternative to preparing monoclonal antibody-secreting hybridomas, a monoclonal anti-58297 antibody can be identified and isolated by screening a recombinant combinatorial immunoglobulin library (*e.g.*, an antibody phage display library) with 58297 to thereby isolate immunoglobulin library members that bind 58297. Kits for generating and screening phage display libraries are commercially available (*e.g*., the Pharmacia *Recombinant Phage Antibody System,* Catalog No. 27-9400-01; and the Stratagene *SurfZAP*™ *Phage Display Kit*, Catalog No. 240612). Additionally, examples of methods and reagents particularly amenable for use in generating and screening antibody display library can be found in, for example, Ladner et al. U.S. Patent No. 5,223,409; Kang et al. PCT International Publication No. WO 92/18619; Dower et al. PCT International Publication No. WO 91/17271; Winter et al. PCT International Publication WO 92/20791; Markland et al. PCT International Publication No. WO 92/15679; Breitling et al. PCT International Publication WO 93/01288; McCafferty et al. PCT International Publication No. WO 92/01047; Garrard et al. PCT International Publication No. WO 92/09690; Ladner et al. PCT International Publication No. WO 90/02809; Fuchs et al. (1991) *Bio*/*Technology* 9:1370-1372; Hay et al. (1992) *Hum. Antibod. Hybridomas* 3:81-85; Huse et al. (1989) *Science* 246:1275-1281; Griffiths et al. (1993) *EMBO J* 12:725-734; Hawkins et al. (1992) *J. Mol. Biol.* 226:889-896; Clarkson et al. (1991) *Nature* 352:624-628; Gram et al. (1992) *Proc. Natl. Acad. Sci. USA* 89:3576-3580; Garrad et al. (1991) *Bio*/*Technology* 9:1373-1377; Hoogenboom et al. (1991) *Nuc. Acid Res.* 19:4133-4137; Barbas et al. (1991) *Proc. Natl. Acad. Sci. USA* 88:7978-7982; and McCafferty et al. *Nature* (1990) 348:552-554.

Additionally, chimeric, humanized, and completely human antibodies are also within the scope of the invention. Chimeric, humanized, but most preferably, completely human antibodies are desirable for applications which include repeated administration, e.g., therapeutic treatment of human patients, and some diagnostic applications.

Chimeric and humanized monoclonal antibodies, comprising both human and non-human portions, can be made using standard recombinant DNA techniques. Such chimeric and humanized monoclonal antibodies can be produced by recombinant DNA techniques known in the art, for example using methods described in Robinson et al. International Application No. PCT/US86/02269; Akira, et al. European Patent Application 184,187; Taniguchi, M., European Patent Application 171,496; Morrison et al. European Patent Application 173,494; Neuberger et al. PCT International Publication No. WO 86/01533; Cabilly et al. U.S. Patent No. 4,816,567; Cabilly et al. European Patent Application 125,023; Better et al. (1988) *Science* 240:1041-1043; Liu et al. (1987) *Proc. Natl. Acad. Sci. USA* 84:3439-3443; Liu et al. (1987) *J. Immunol*. 139:3521-3526; Sun et al. (1987) *Proc. Natl. Acad. Sci. USA* 84:214-218; Nishimura et al. (1987) *Canc. Res.* 47:999-1005; Wood et al. (1985) *Nature* 314:446-449; and Shaw et al. (1988) *J. Natl. Cancer Inst.* 80:1553-1559); Morrison, S. L. (1985) *Science* 229:1202-1207; Oi et al. (1986) *BioTechniques* 4:214; Winter U.S. Patent 5,225,539; Jones et al. (1986) *Nature* 321:552-525; Verhoeyan et al. (1988) *Science* 239:1534; and Beidler et al. (1988) *J. Immunol.* 141:4053-4060.

Completely human antibodies are particularly desirable for therapeutic treatment of human patients. Such antibodies can be produced using transgenic mice that are incapable of expressing endogenous immunoglobulin heavy and light chains genes, but which can express human heavy and light chain genes. *See*, for example, Lonberg and Huszar (1995) *Int. Rev. Immunol. 13*:65-93); and U.S. Patent Nos. 5,625,126; 5,633,425; 5,569,825; 5,661,016; and 5,545,806. In addition, companies such as Abgenix, Inc. (Fremont, CA) and Medarex, Inc. (Princeton, NJ), can be engaged to provide human antibodies directed against a selected antigen using technology similar to that described above.

Completely human antibodies that recognize a selected epitope can be generated using a technique referred to as "guided selection." In this approach a selected non-human monoclonal antibody, e.g., a murine antibody, is used to guide the selection of a completely human antibody recognizing the same epitope. This technology is described by Jespers et al. (1994) *Bio*/*Technology 12*:899-903).

A full-length 58297 protein, or an antigenic peptide fragment of 58297, can be used as an immunogen or can be used to identify anti-58297 antibodies made with other immunogens, e.g., cells, membrane preparations, and the like. The antigenic peptides of 58297 should include at least 8 amino acid residues of the amino acid sequence shown in SEQ ID NO:2 and encompass an epitope of 58297, respectively. Preferably, the antigenic peptide includes at least 10 amino acid residues, more preferably at least 15 amino acid residues, even more preferably at least 20 amino acid residues, and most preferably at least 30 amino acid residues.

Fragments of 58297 which include, e.g., residues 141 to 551 of SEQ ID NO:2, can be used as immunogens to make an antibody against the transmembrane amino acid transporter domain of the 58297 protein.

Antibodies reactive with, or specific for, any of these regions, or other regions or domains described herein are provided.

In an alternative embodiment, the antibody fails to bind to an Fc receptor, e.g., it is a type which does not support Fc receptor binding or has been modified, e.g., by deletion or other mutation, such that is does not have a functional Fc receptor binding region.

Preferred epitopes encompassed by the antigenic peptide are regions of 58297 which are located on the surface of the protein, *e.g*., hydrophilic regions (depicted, e.g., in the hydropathy plot in *Figure 1,* as residues below the dashed horizontal line), as well as regions with high antigenicity. For example, an Emini surface probability analysis of the human 58297 protein sequence can be used to identify the regions that have a particularly high probability of being localized to the surface of the 58297 protein, and are thus likely to constitute surface residues useful for targeting antibody production.

In a preferred embodiment the antibody binds an epitope on any domain or region on 58297 proteins described herein.

The anti-58297 antibody can be a single chain antibody. A single-chain antibody (scFV) may be engineered as described, for example, in Colcher, D. et al., (1999 ) *Ann. NY Acad. Sci.* 880: 263-80; and Reiter, Y., *Clin. Cancer Res.* 1996 Feb;2(2):245-52. The single chain antibody can be dimerized or multimerized to generate multivalent antibodies having specificities for different epitopes of the same target 58297 protein.

Anti-58297 antibodies (e.g., monoclonal antibodies) can be used to isolate 58297, respectively, by standard techniques, such as affinity chromatography or immunoprecipitation. Moreover, an anti-58297 antibody can be used to detect 58297 protein, respectively, (e.g., in a cellular lysate or cell supernatant) in order to evaluate the abundance and pattern of expression of the protein. Anti-58297 antibodies can be used diagnostically to monitor protein levels in tissue as part of a clinical testing procedure, e.g., to, for example, determine the efficacy of a given treatment regimen. Detection can be facilitated by coupling (i.e., physically linking) the antibody to a detectable substance (i.e., antibody labeling). Examples of detectable substances include various enzymes, prosthetic groups, fluorescent materials, luminescent materials, bioluminescent materials, and radioactive materials. Examples of suitable enzymes include horseradish peroxidase, alkaline phosphatase, □-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include ¹²⁵I, ¹³¹I, ³⁵S or ³H.

### 1.3 Recombinant Expression Vectors, Host Cells and Genetically Engineered Cells

In another aspect, the invention includes, vectors, preferably expression vectors, containing a nucleic acid encoding a polypeptide described herein. As used herein, the term "vector" refers to a nucleic acid molecule capable of transporting another nucleic acid to which it has been linked and can include a plasmid, cosmid or viral vector. The vector can be capable of autonomous replication or it can integrate into a host DNA. Viral vectors include, e.g., replication defective retroviruses, adenoviruses and adeno-associated viruses.

A vector can include a 58297 nucleic acid in a form suitable for expression of the nucleic acid in a host cell. Preferably the recombinant expression vector includes one or more regulatory sequences operatively linked to the nucleic acid sequence to be expressed. The term "regulatory sequence" includes promoters, enhancers and other expression control elements (e.g., polyadenylation signals). Regulatory sequences include those which direct constitutive expression of a nucleotide sequence, as well as tissue-specific regulatory and/or inducible sequences. The design of the expression vector can depend on such factors as the choice of the host cell to be transformed, the level of expression of protein desired, and the like. The expression vectors of the invention can be introduced into host cells to thereby produce proteins or polypeptides, including fusion proteins or polypeptides, encoded by nucleic acids as described herein (e.g., 58297 proteins, mutant forms of 58297 proteins, fusion proteins, and the like).

The recombinant expression vectors of the invention can be designed for expression of 58297 proteins in prokaryotic or eukaryotic cells. For example, polypeptides of the invention can be expressed in *E. coli,* insect cells (e.g., using baculovirus expression vectors), yeast cells or mammalian cells. Suitable host cells are discussed further in Goeddel, (1990) *Gene Expression Technology: Methods in Enzymology* 185, Academic Press, San Diego, CA . Alternatively, the recombinant expression vector can be transcribed and translated *in vitro,* for example using T7 promoter regulatory sequences and T7 polymerase.

Expression of proteins in prokaryotes is most often carried out in *E. coli* with vectors containing constitutive or inducible promoters directing the expression of either fusion or non-fusion proteins. Fusion vectors add a number of amino acids to a protein encoded therein, usually to the amino terminus of the recombinant protein. Such fusion vectors typically serve three purposes: 1) to increase expression of recombinant protein; 2) to increase the solubility of the recombinant protein; and 3) to aid in the purification of the recombinant protein by acting as a ligand in affinity purification. Often, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin, and enterokinase. Typical fusion expression vectors include pGEX (Pharmacia Biotech, Inc; Smith, D.B. and Johnson, K.S. (1988) Gene 67:31-40), pMAL (New England Biolabs, Beverly MA) and pRIT5 (Pharmacia, Piscataway NJ) which fuse glutathione S-transferase (GST), maltose E binding protein, or protein A, respectively, to the target recombinant protein.

Purified fusion proteins can be used in 58297 activity assays, (e.g., direct assays or competitive assays described in detail below), or to generate antibodies specific for 58297 proteins. In a preferred embodiment, a fusion protein expressed in a retroviral expression vector of the present invention can be used to infect bone marrow cells which are subsequently transplanted into irradiated recipients. The pathology of the subject recipient is then examined after sufficient time has passed (e.g., six weeks).

To maximize recombinant protein expression in *E. coli* is to express the protein in a host bacteria with an impaired capacity to proteolytically cleave the recombinant protein (Gottesman, S., (1990) *Gene Expression Technology: Methods in Enzymology* 185, Academic Press, San Diego, California 119-128). Another strategy is to alter the nucleic acid sequence of the nucleic acid to be inserted into an expression vector so that the individual codons for each amino acid are those preferentially utilized in *E. coli* (Wada et al., (1992) *Nucleic Acids Res.* 20:2111-2118). Such alteration of nucleic acid sequences of the invention can be carried out by standard DNA synthesis techniques.

The 58297 expression vector can be a yeast expression vector, a vector for expression in insect cells, e.g., a baculovirus expression vector or a vector suitable for expression in mammalian cells.

When used in mammalian cells, the expression vector's control functions are often provided by viral regulatory elements. For example, commonly used promoters are derived from polyoma, Adenovirus 2, cytomegalovirus and Simian Virus 40.

In another embodiment, the recombinant mammalian expression vector is capable of directing expression of the nucleic acid preferentially in a particular cell type (e.g., tissue-specific regulatory elements are used to express the nucleic acid). Non-limiting examples of suitable tissue-specific promoters include the albumin promoter (liver-specific; Pinkert et al. (1987) *Genes Dev.* 1:268-277), lymphoid-specific promoters (Calame and Eaton (1988) *Adv. Immunol.* 43:235-275), in particular promoters of T cell receptors (Winoto and Baltimore (1989) *EMBO J.* 8:729-733) and immunoglobulins (Banerji et al. (1983) *Cell* 33:729-740; Queen and Baltimore (1983) *Cell* 33:741-748), neuron-specific promoters (e.g., the neurofilament promoter; Byrne and Ruddle (1989) *Proc. Natl. Acad. Sci. USA* 86:5473-5477), pancreas-specific promoters (Edlund et al. (1985) *Science* 230:912-916), and mammary gland-specific promoters (e.g., milk whey promoter; U.S. Patent No. 4,873,316 and European Application Publication No. 264,166). Developmentally-regulated promoters are also encompassed, for example, the murine hox promoters (Kessel and Gruss (1990) *Science* 249:374-379) and the □-fetoprotein promoter (Campes and Tilghman (1989) *Genes Dev.* 3:537-546).

The invention further provides a recombinant expression vector comprising a DNA molecule of the invention cloned into the expression vector in an antisense orientation. Regulatory sequences (e.g., viral promoters and/or enhancers) operatively linked to a nucleic acid cloned in the antisense orientation can be chosen which direct the constitutive, tissue specific or cell type specific expression of antisense RNA in a variety of cell types. The antisense expression vector can be in the form of a recombinant plasmid, phagemid or attenuated virus. For a discussion of the regulation of gene expression using antisense genes see Weintraub, H. et al., (1986) Antisense RNA as a molecular tool for genetic analysis, *Reviews - Trends in Genetics* 1:1.

Another aspect the invention provides a host cell which includes a nucleic acid molecule described herein, e.g., a 58297 nucleic acid molecule within a recombinant expression vector or a 58297 nucleic acid molecule containing sequences which allow it to homologously recombine into a specific site of the host cell's genome. The terms "host cell" and "recombinant host cell" are used interchangeably herein. Such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein.

A host cell can be any prokaryotic or eukaryotic cell. For example, a 58297 protein can be expressed in bacterial cells such as *E. coli*, insect cells, yeast or mammalian cells (such as Chinese hamster ovary cells (CHO) or COS cells). Other suitable host cells are known to those skilled in the art.

Vector DNA can be introduced into host cells via conventional transformation or transfection techniques. As used herein, the terms "transformation" and "transfection" are intended to refer to a variety of art-recognized techniques for introducing foreign nucleic acid (e.g., DNA) into a host cell, including calcium phosphate or calcium chloride coprecipitation, DEAE-dextran-mediated transfection, lipofection, or electroporation.

A host cell of the invention can be used to produce (i.e., express) a 58297 protein. Accordingly, the invention further provides methods for producing a 58297 protein using the host cells of the invention. In one embodiment, the method includes culturing the host cell of the invention (into which a recombinant expression vector encoding a 58297 protein has been introduced) in a suitable medium such that a 58297 protein is produced. In another embodiment, the method further includes isolating a 58297 protein from the medium or the host cell.

In another aspect, the invention features a cell or a purified preparation of cells which includes a 58297 transgene, or which otherwise misexpresses 58297. The cell preparation can consist of human or nonhuman cells, e.g., rodent cells, e.g., mouse or rat cells, rabbit cells, or pig cells. In preferred embodiments, the cell, or cells, include a 58297 transgene, e.g., a heterologous form of 58297, e.g., a gene derived from humans (in the case of a non-human cell). The 58297 transgene can be misexpressed, e.g., overexpressed or underexpressed. In other preferred embodiments, the cell, or cells, includes a gene which misexpresses an endogenous 58297, e.g., a gene, the expression of which is disrupted, e.g., a knockout. Such cells can serve as a model for studying disorders which are related to mutated or mis-expressed 58297 alleles or for use in drug screening.

In another aspect, the invention features, a human cell, e.g., a hematopoietic stem cell, transformed with nucleic acid which encodes a subject 58297 polypeptide.

Also provided are cells, preferably human cells, e.g., human hematopoietic or fibroblast cells, in which an endogenous 58297 gene is under the control of a regulatory sequence that does not normally control the expression of the endogenous 58297 gene. The expression characteristics of an endogenous gene within a cell, e.g., a cell line or microorganism, can be modified by inserting a heterologous DNA regulatory element into the genome of the cell such that the inserted regulatory element is operably linked to the endogenous 58297 gene. For example, an endogenous 58297 gene which is "transcriptionally silent," e.g., not normally expressed, or expressed only at very low levels, may be activated by inserting a regulatory element which is capable of promoting the expression of a normally expressed gene product in that cell. Techniques such as targeted homologous recombinations, can be used to insert the heterologous DNA as described in, e.g., Chappel, US 5,272,071; WO 91/06667, published in May 16, 1991.

### 1.4 Transgenic Animals

The invention provides non-human transgenic animals. Such animals are useful for studying the function and/or activity of a 58297 protein and for identifying and/or evaluating modulators of 58297 activity. As used herein, a "transgenic animal" is a non-human animal, preferably a mammal, more preferably a rodent such as a rat or mouse, in which one or more of the cells of the animal includes a transgene. Other examples of transgenic animals include non-human primates, sheep, dogs, cows, goats, chickens, amphibians, and the like. A transgene is exogenous DNA or a rearrangement, e.g., a deletion of endogenous chromosomal DNA, which preferably is integrated into or occurs in the genome of the cells of a transgenic animal. A transgene can direct the expression of an encoded gene product in one or more cell types or tissues of the transgenic animal, other transgenes, e.g., a knockout, reduce expression. Thus, a transgenic animal can be one in which an endogenous 58297 gene has been altered by, e.g., by homologous recombination between the endogenous gene and an exogenous DNA molecule introduced into a cell of the animal, e.g., an embryonic cell of the animal, prior to development of the animal.

Intronic sequences and polyadenylation signals can also be included in the transgene to increase the efficiency of expression of the transgene. A tissue-specific regulatory sequence(s) can be operably linked to a transgene of the invention to direct expression of a 58297 protein to particular cells. A transgenic founder animal can be identified based upon the presence of a 58297 transgene in its genome and/or expression of 58297 mRNA in tissues or cells of the animals. A transgenic founder animal can then be used to breed additional animals carrying the transgene. Moreover, transgenic animals carrying a transgene encoding a 58297 protein can further be bred to other transgenic animals carrying other transgenes.

58297 proteins or polypeptides can be expressed in transgenic animals or plants, e.g., a nucleic acid encoding the protein or polypeptide can be introduced into the genome of an animal. In preferred embodiments the nucleic acid is placed under the control of a tissue specific promoter, e.g., a milk or egg specific promoter, and recovered from the milk or eggs produced by the animal. Suitable animals are mice, pigs, cows, goats, and sheep.

The invention also includes a population of cells from a transgenic animal, as discussed, e.g., herein.

### 1.5 Uses

The nucleic acid molecules, proteins, protein homologues, and antibodies described herein can be used in one or more of the following methods: a) screening assays; b) predictive medicine (e.g., diagnostic assays, prognostic assays, monitoring clinical trials, and pharmacogenetics); and c) methods of treatment (e.g., therapeutic and prophylactic).

The isolated nucleic acid molecules of the invention can be used, for example, to express a 58297 protein (e.g., via a recombinant expression vector in a host cell in gene therapy applications), to detect a 58297 mRNA (e.g., in a biological sample) or a genetic alteration in a 58297 gene, and to modulate 58297 activity, as described further below. The 58297 proteins can be used to treat disorders characterized by insufficient or excessive production of a 58297 substrate or production of 58297 inhibitors. In addition, the 58297 proteins can be used to screen for naturally occurring 58297 substrates, to screen for drugs or compounds which modulate 58297 activity, as well as to treat disorders characterized by insufficient or excessive production of 58297 protein or production of 58297 protein forms which have decreased, aberrant or unwanted activity compared to 58297 wild type protein. Moreover, the anti-58297 antibodies of the invention can be used to detect and isolate 58297 proteins, regulate the bioavailability of 58297 proteins, and modulate 58297 activity.

A method of evaluating a compound for the ability to interact with, e.g., bind, a subject 58297 polypeptide is provided. The method includes: contacting the compound with the subject 58297 polypeptide; and evaluating ability of the compound to interact with, e.g., to bind or form a complex with the subject 58297 polypeptide. This method can be performed *in vitro,* e.g., in a cell free system, or *in vivo,* e.g., in a two-hybrid interaction trap assay. This method can be used to identify naturally occurring molecules which interact with subject 58297 polypeptide. It can also be used to find natural or synthetic inhibitors of subject 58297 polypeptide. Screening methods are discussed in more detail herein.

### 1.6 Screening Assays:

The invention provides methods (also referred to herein as "screening assays") for identifying modulators, i.e., candidate or test compounds or agents (e.g., proteins, peptides, peptidomimetics, peptoids, small molecules or other drugs) which bind to 58297 proteins, have a stimulatory or inhibitory effect on, for example, 58297 expression or 58297 activity, or have a stimulatory or inhibitory effect on, for example, the expression or activity of a 58297 substrate. Compounds thus identified can be used to modulate the activity of target gene products (e.g., 58297 genes) in a therapeutic protocol, to elaborate the biological function of the target gene product, or to identify compounds that disrupt normal target gene interactions.

In one embodiment, the invention provides assays for screening candidate or test compounds which are substrates of a 58297 protein or polypeptide or a biologically active portion thereof. In another embodiment, the invention provides assays for screening candidate or test compounds which bind to or modulate the activity of a 58297 protein or polypeptide or a biologically active portion thereof.

The test compounds of the present invention can be obtained using any of the numerous approaches in combinatorial library methods known in the art, including: biological libraries; peptoid libraries (libraries of molecules having the functionalities of peptides, but with a novel, non-peptide backbone which are resistant to enzymatic degradation but which nevertheless remain bioactive; see, e.g., Zuckermann, R.N. et al. (1994) *J. Med. Chem.* 37:2678-85); spatially addressable parallel solid phase or solution phase libraries; synthetic library methods requiring deconvolution; the 'one-bead one-compound' library method; and synthetic library methods using affinity chromatography selection. The biological library and peptoid library approaches are limited to peptide libraries, while the other four approaches are applicable to peptide, non-peptide oligomer or small molecule libraries of compounds (Lam, K.S. (1997) *Anticancer Drug Des.* 12:145).

Examples of methods for the synthesis of molecular libraries can be found in the art, for example in: DeWitt et al. (1993) *Proc. Natl. Acad. Sci. U.S.A.* 90:6909; Erb et al. (1994) *Proc. Natl. Acad. Sci. USA* 91:11422; Zuckermann et al. (1994). *J. Med. Chem.* 37:2678; Cho et al. (1993) *Science* 261:1303; Carrell et al. (1994) *Angew. Chem. Int. Ed. Engl.* 33:2059; Carell et al. (1994) *Angew. Chem. Int. Ed. Engl.* 33:2061; and in Gallop et al. (1994) *J. Med. Chem.* 37:1233.Libraries of compounds may be presented in solution (e.g., Houghten (1992) *Biotechniques* 13:412-421), or on beads (Lam (1991) *Nature* 354:82-84), chips (Fodor (1993) *Nature* 364:555-556), bacteria (Ladner, USP 5,223,409), spores (Ladner USP '409), plasmids (Cull et al. (1992) *Proc Natl Acad Sci USA* 89:1865-1869) or on phage (Scott and Smith (1990) *Science* 249:386-390; Devlin (1990) *Science* 249:404-406; Cwirla et al. (1990) *Proc. Natl. Acad. Sci.* 87:6378-6382; Felici (1991) *J. Mol. Biol.* 222:301-310; Ladner *supra*.).

In one embodiment, an assay is a cell-based assay in which a cell which expresses a 58297 protein or biologically active portion thereof is contacted with a test compound, and the ability of the test compound to modulate 58297 activity is determined. Determining the ability of the test compound to modulate 58297 activity can be accomplished by monitoring, for example, (i) the interaction of a 58297 protein with a 58297 target molecule; (ii) the interaction of a 58297 protein with a 58297 target molecule, wherein the 58297 target is an amino acid substrate, e.g., an amino acid which 58297 proteins can transport across the plasma membrane, e.g., an amino acids which can be transported across the plasma membrane by the N transport system proteins, e.g., histidine, asparagine, and glutamine. The cell, for example, can be of mammalian origin, e.g., human.

The ability of the test compound to modulate 58297 binding to a compound, e.g., a 58297 substrate, or to bind to 58297 can also be evaluated. This can be accomplished, for example, by coupling the compound, e.g., the substrate, with a radioisotope or enzymatic label such that binding of the compound, e.g., the substrate, to 58297 can be determined by detecting the labeled compound, e.g., substrate, in a complex. Alternatively, 58297 could be coupled with a radioisotope or enzymatic label to monitor the ability of a test compound to modulate 58297 binding to a 58297 substrate in a complex. For example, compounds (e.g., 58297 substrates) can be labeled with ¹²⁵I, ³⁵S, ¹⁴C, or ³H, either directly or indirectly, and the radioisotope detected by direct counting of radioemmission or by scintillation counting. Alternatively, compounds can be enzymatically labeled with, for example, horseradish peroxidase, alkaline phosphatase, or luciferase, and the enzymatic label detected by determination of conversion of an appropriate substrate to product.

The ability of a compound (e.g., a 58297 substrate) to interact with 58297, with or without the labeling of any of the interactants, can be evaluated. For example, a microphysiometer can be used to detect the interaction of a compound with 58297 without the labeling of either the compound or the 58297. McConnell, H. M. et al. (1992) *Science* 257:1906-1912. As used herein, a "microphysiometer" (e.g., Cytosensor) is an analytical instrument that measures the rate at which a cell acidifies its environment using a light-addressable potentiometric sensor (LAPS). Changes in this acidification rate can be used as an indicator of the interaction between a compound and 58297.

In yet another embodiment, a cell-free assay is provided in which a 58297 protein, or biologically active portion thereof, is contacted with a test compound and the ability of the test compound to bind to the 58297 protein, or biologically active portion thereof, is evaluated. Preferred biologically active portions of the 58297 proteins to be used in assays of the present invention include fragments which participate in interactions with non-58297 molecules, e.g., fragments with high surface probability scores.

Soluble and/or membrane-bound forms of isolated proteins (e.g., 58297 proteins, or biologically active portions thereof) can be used in the cell-free assays of the invention. When membrane-bound forms of the protein are used, it may be desirable to utilize a solubilizing agent. Examples of such solubilizing agents include non-ionic detergents such as n-octylglucoside, n-dodecylglucoside, n-dodecylmaltoside, octanoyl-N-methylglucamide, decanoyl-N-methylglucamide, Triton® X-100, Triton® X-114, Thesit®, Isotridecypoly(ethylene glycol ether)ₙ, 3-[(3-cholamidopropyl)dimethylamminio]-1-propane sulfonate (CHAPS), 3-[(3-cholamidopropyl)dimethylamminio]-2-hydroxy-1-propane sulfonate (CHAPSO), or N-dodecyl=N,N-dimethyl-3-ammonio-1-propane sulfonate.

Cell-free assays involve preparing a reaction mixture of the target gene protein and the test compound under conditions and for a time sufficient to allow the two components to interact and bind, thus forming a complex that can be removed and/or detected.

The interaction between two molecules can also be detected, e.g., using fluorescence energy transfer (FET) (see, for example, Lakowicz et al., U.S. Patent No. 5,631,169; Stavrianopoulos, et al., U.S. Patent No. 4,868,103). A fluorophore label on the first, 'donor' molecule is selected such that its emitted fluorescent energy will be absorbed by a fluorescent label on a second, 'acceptor' molecule, which in turn is able to fluoresce due to the absorbed energy. Alternately, the 'donor' protein molecule may simply utilize the natural fluorescent energy of tryptophan residues. Labels are chosen that emit different wavelengths of light, such that the 'acceptor' molecule label may be differentiated from that of the 'donor'. Since the efficiency of energy transfer between the labels is related to the distance separating the molecules, the spatial relationship between the molecules can be assessed. In a situation in which binding occurs between the molecules, the fluorescent emission of the 'acceptor' molecule label in the assay should be maximal. An FET binding event can be conveniently measured through standard fluorometric detection means well known in the art (e.g., using a fluorimeter).

In another embodiment, determining the ability of the 58297 protein to bind to a target molecule can be accomplished using real-time Biomolecular Interaction Analysis (BIA) (see, e.g., Sjolander, S. and Urbaniczky, C. (1991) *Anal. Chem.* 63:2338-2345 and Szabo et al. (1995) *Curr. Opin. Struct. Biol.* 5:699-705). "Surface plasmon resonance" or "BIA" detects biospecific interactions in real time, without labeling any of the interactants (e.g., BIAcore). Changes in the mass at the binding surface (indicative of a binding event) result in alterations of the refractive index of light near the surface (the optical phenomenon of surface plasmon resonance (SPR)), resulting in a detectable signal which can be used as an indication of real-time reactions between biological molecules.

In one embodiment, the target gene product or the test substance is anchored onto a solid phase. The target gene product/test compound complexes anchored on the solid phase can be detected at the end of the reaction. Preferably, the target gene product can be anchored onto a solid surface, and the test compound, (which is not anchored), can be labeled, either directly or indirectly, with detectable labels discussed herein.

It may be desirable to immobilize 58297, an anti-58297 antibody, or a 58297 target molecule to facilitate separation of complexed from uncomplexed forms of one or both of the proteins, as well as to accommodate automation of the assay. Binding of a test compound to a 58297 protein, or interaction of a 58297 protein with a target molecule in the presence and absence of a candidate compound, can be accomplished in any vessel suitable for containing the reactants. Examples of such vessels include microtiter plates, test tubes, and micro-centrifuge tubes. In one embodiment, a fusion protein can be provided which adds a domain that allows one or both of the proteins to be bound to a matrix. For example, glutathione-S-transferase/58297 fusion proteins or glutathione-S-transferase/target fusion proteins can be adsorbed onto glutathione sepharose beads (Sigma Chemical, St. Louis, MO) or glutathione derivatized microtiter plates, which are then combined with the test compound or the test compound and either the non-adsorbed target protein or 58297 protein, and the mixture incubated under conditions conducive to complex formation (e.g., at physiological conditions for salt and pH). Following incubation, the beads or microtiter plate wells are washed to remove any unbound components, the matrix immobilized in the case of beads, complex determined either directly or indirectly, for example, as described above. Alternatively, the complexes can be dissociated from the matrix, and the level of 58297 binding or activity determined using standard techniques.

Other techniques for immobilizing either a 58297 protein or a target molecule on matrices include using conjugation of biotin and streptavidin. Biotinylated 58297 protein or target molecules can be prepared from biotin-NHS (N-hydroxy-succinimide) using techniques known in the art (e.g., biotinylation kit, Pierce Chemicals, Rockford, IL), and immobilized in the wells of streptavidin-coated 96 well plates (Pierce Chemical).

In order to conduct the assay, the non-immobilized component is added to the coated surface containing the anchored component. After the reaction is complete, unreacted components are removed (e.g., by washing) under conditions such that any complexes formed will remain immobilized on the solid surface. The detection of complexes anchored on the solid surface can be accomplished in a number of ways. Where the previously non-immobilized component is pre-labeled, the detection of label immobilized on the surface indicates that complexes were formed. Where the previously non-immobilized component is not pre-labeled, an indirect label can be used to detect complexes anchored on the surface; e.g., using a labeled antibody specific for the immobilized component (the antibody, in turn, can be directly labeled or indirectly labeled with, e.g., a labeled anti-Ig antibody).

In one embodiment, this assay is performed utilizing antibodies reactive with 58297 protein or target molecules but which do not interfere with binding of the 58297 protein to its target molecule. Such antibodies can be derivatized to the wells of the plate, and unbound target or 58297 protein trapped in the wells by antibody conjugation. Methods for detecting such complexes, in addition to those described above for the GST-immobilized complexes, include immunodetection of complexes using antibodies reactive with the 58297 protein or target molecule, as well as enzyme-linked assays which rely on detecting an enzymatic activity associated with the 58297 protein or target molecule.

Alternatively, cell-free assays can be conducted in a liquid phase. In such an assay, the reaction products are separated from unreacted components, by any of a number of standard techniques, including but not limited to: differential centrifugation (see, for example, Rivas, G., and Minton, A.P., (1993) *Trends Biochem Sci* 18:284-7); chromatography (gel filtration chromatography, ion-exchange chromatography); electrophoresis (see, e.g., Ausubel, F. et al., eds. Current Protocols in Molecular Biology 1999, J. Wiley: New York.); and immunoprecipitation (see, for example, Ausubel, F. et al., eds. (1999) *Current Protocols in Molecular Biology,* J. Wiley: New York). Such resins and chromatographic techniques are known to one skilled in the art (see, e.g., Heegaard, N.H., (1998) *J Mol Recognit* 11:141-8; Hage, D.S., and Tweed, S.A. (1997) *J Chromatogr B Biomed Sci Appl.* 699:499-525). Further, fluorescence energy transfer may also be conveniently utilized, as described herein, to detect binding without further purification of the complex from solution.

In a preferred embodiment, the assay includes contacting the 58297 protein, or biologically active portion thereof, with a known compound which binds 58297 to form an assay mixture, contacting the assay mixture with a test compound, and determining the ability of the test compound to interact with a 58297 protein, wherein determining the ability of the test compound to interact with a 58297 protein includes determining the ability of the test compound to preferentially bind to 58297, or biologically active portion thereof, or to modulate the activity of a target molecule, as compared to the known compound.

The target gene products of the invention can, *in vivo,* interact with one or more cellular or extracellular macromolecules, such as proteins. For the purposes of this discussion, such cellular and extracellular macromolecules are referred to herein as "binding partners." Compounds that disrupt such interactions can be useful in regulating the activity of the target gene product. Such compounds can include, but are not limited to, molecules such as antibodies, peptides, and small molecules. The preferred target genes/products for use in this embodiment are the 58297 genes herein identified. In an alternative embodiment, the invention provides methods for determining the ability of the test compound to modulate the activity of a 58297 protein through modulation of the activity of a downstream effector of a 58297 target molecule. For example, the activity of the effector molecule on an appropriate target can be determined, or the binding of the effector to an appropriate target can be determined, as previously described.

To identify compounds that interfere with the interaction between the target gene product and its cellular or extracellular binding partner(s), a reaction mixture containing the target gene product and the binding partner is prepared, under conditions and for a time sufficient, to allow the two products to form complex. In order to test an inhibitory agent, the reaction mixture is provided in the presence and absence of the test compound. The test compound can be initially included in the reaction mixture, or can be added at a time subsequent to the addition of the target gene and its cellular or extracellular binding partner. Control reaction mixtures are incubated without the test compound or with a placebo. The formation of any complexes between the target gene product and the cellular or extracellular binding partner is then detected. The formation of a complex in the control reaction, but not in the reaction mixture containing the test compound, indicates that the compound interferes with the interaction of the target gene product and the interactive binding partner. Additionally, complex formation within reaction mixtures containing the test compound and normal target gene product can also be compared to complex formation within reaction mixtures containing the test compound and mutant target gene product. This comparison can be important in those cases wherein it is desirable to identify compounds that disrupt interactions of mutant but not normal target gene products.

These assays can be conducted in a heterogeneous or homogeneous format. Heterogeneous assays involve anchoring either the target gene product or the binding partner onto a solid phase, and detecting complexes anchored on the solid phase at the end of the reaction. In homogeneous assays, the entire reaction is carried out in a liquid phase. In either approach, the order of addition of reactants can be varied to obtain different information about the compounds being tested. For example, test compounds that interfere with the interaction between the target gene products and the binding partners, e.g., by competition, can be identified by conducting the reaction in the presence of the test substance. Alternatively, test compounds that disrupt preformed complexes, e.g., compounds with higher binding constants that displace one of the components from the complex, can be tested by adding the test compound to the reaction mixture after complexes have been formed. The various formats are briefly described herein.

In a heterogeneous assay system, either the target gene product or the interactive cellular or extracellular binding partner, is anchored onto a solid surface (e.g., a microtiter plate), while the non-anchored species is labeled, either directly or indirectly. The anchored species can be immobilized by non-covalent or covalent attachments. Alternatively, an immobilized antibody specific for the species to be anchored can be used to anchor the species to the solid surface.

In order to conduct the assay, the partner of the immobilized species is exposed to the coated surface with or without the test compound. After the reaction is complete, unreacted components are removed (e.g., by washing) and any complexes formed will remain immobilized on the solid surface. Where the non-immobilized species is pre-labeled, the detection of label immobilized on the surface indicates that complexes were formed. Where the non-immobilized species is not pre-labeled, an indirect label can be used to detect complexes anchored on the surface; e.g., using a labeled antibody specific for the initially non-immobilized species (the antibody, in turn, can be directly labeled or indirectly labeled with, e.g., a labeled anti-Ig antibody). Depending upon the order of addition of reaction components, test compounds that inhibit complex formation or that disrupt preformed complexes can be detected.

Alternatively, the reaction can be conducted in a liquid phase in the presence or absence of the test compound, the reaction products separated from unreacted components, and complexes detected; e.g., using an immobilized antibody specific for one of the binding components to anchor any complexes formed in solution, and a labeled antibody specific for the other partner to detect anchored complexes. Again, depending upon the order of addition of reactants to the liquid phase, test compounds that inhibit complex or that disrupt preformed complexes can be identified.

In an alternate embodiment of the invention, a homogeneous assay can be used. For example, a preformed complex of the target gene product and the interactive cellular or extracellular binding partner product is prepared in that either the target gene products or their binding partners are labeled, but the signal generated by the label is quenched due to complex formation (see, e.g., U.S. Patent No. 4,109,496 that utilizes this approach for immunoassays). The addition of a test substance that competes with and displaces one of the species from the preformed complex will result in the generation of a signal above background. In this way, test substances that disrupt target gene product-binding partner interaction can be identified.

In yet another aspect, the 58297 proteins can be used as "bait proteins" in a two-hybrid assay or three-hybrid assay (see, e.g., U.S. Patent No. 5,283,317; Zervos et al. (1993) *Cell* 72:223-232; Madura et al. (1993) *J. Biol. Chem.* 268:12046-12054; Bartel et al. (1993) *Biotechniques* 14:920-924; Iwabuchi et al. (1993) *Oncogene* 8:1693-1696; and Brent WO94/10300), to identify other proteins, which bind to or interact with 58297 ("58297-binding proteins" or "58297-bp") and are involved in 58297 activity. Such 58297-bps can be activators or inhibitors of signals by the 58297 proteins or 58297 targets as, for example, downstream elements of a 58297-mediated signaling pathway.

The two-hybrid system is based on the modular nature of most transcription factors, which consist of separable DNA-binding and activation domains. Briefly, the assay utilizes two different DNA constructs. In one construct, the gene that codes for a 58297 protein is fused to a gene encoding the DNA binding domain of a known transcription factor (e.g., GAL-4). In the other construct, a DNA sequence, from a library of DNA sequences, that encodes an unidentified protein ("prey" or "sample") is fused to a gene that codes for the activation domain of the known transcription factor. (Alternatively the: 58297 protein can be the fused to the activator domain.) If the "bait" and the "prey" proteins are able to interact, *in vivo,* forming a 58297-dependent complex, the DNA-binding and activation domains of the transcription factor are brought into close proximity. This proximity allows transcription of a reporter gene (e.g., lacZ) which is operably linked to a transcriptional regulatory site responsive to the transcription factor. Expression of the reporter gene can be detected and cell colonies containing the functional transcription factor can be isolated and used to obtain the cloned gene which encodes the protein which interacts with the 58297 protein.

In another embodiment, modulators of 58297 expression are identified. For example, a cell or cell free mixture is contacted with a candidate compound and the expression of 58297 mRNA or protein evaluated relative to the level of expression of 58297 mRNA or protein in the absence of the candidate compound. When expression of 58297 mRNA or protein is greater in the presence of the candidate compound than in its absence, the candidate compound is identified as a stimulator of 58297 mRNA or protein expression.
Alternatively, when expression of 58297 mRNA or protein is less (statistically significantly less) in the presence of the candidate compound than in its absence, the candidate compound is identified as an inhibitor of 58297 mRNA or protein expression. The level of 58297 mRNA or protein expression can be determined by methods described herein for detecting 58297 mRNA or protein.

In another aspect, the invention pertains to a combination of two or more of the assays described herein. For example, a modulating agent can be identified using a cell-based or a cell free assay, and the ability of the agent to modulate the activity of a 58297 protein can be confirmed *in vivo* in an animal model.

This invention further pertains to novel agents identified by the above-described screening assays. Accordingly, it is within the scope of this invention to further use an agent identified as described herein (e.g., a 58297 modulating agent, an anti-sense 58297 nucleic acid molecule, a 58297-specific antibody, or a 58297-binding partner) in an appropriate animal model to determine the efficacy, toxicity, side effects, or mechanism of action, of treatment with such an agent. Furthermore, novel agents identified by the above-described screening assays can be used for treatments as described herein.

### 1.7 Detection Assays

Portions or fragments of the nucleic acid sequences identified herein can be used as polynucleotide reagents. For example, these sequences can be used to: (i) map their respective genes on a chromosome e.g., to locate gene regions associated with genetic disease or to associate 58297 with a disease; (ii) identify an individual from a minute biological sample (tissue typing); and (iii) aid in forensic identification of a biological sample. These applications are described in the subsections below.

### 1.8 Chromosome Mapping

The 58297 nucleotide sequences or portions thereof can be used to map the location of the 58297 genes on a chromosome. This process is called chromosome mapping. Chromosome mapping is useful in correlating the 58297 sequences with genes associated with disease. For example, 58297 maps to chromosome 5, based on at least several regions of homology to a human chromosome 5 clone in the art (clone CTD-2028D11, Genbank accession number AC008784), as described herein.

Briefly, 58297 genes can be mapped to chromosomes by preparing PCR primers (preferably 15-25 bp in length) from the 58297 nucleotide sequences. These primers can then be used for PCR screening of somatic cell hybrids containing individual human chromosomes. Only those hybrids containing the human gene corresponding to the 58297 sequences will yield an amplified fragment.

A panel of somatic cell hybrids in which each cell line contains either a single human chromosome or a small number of human chromosomes, and a full set of mouse chromosomes, can allow easy mapping of individual genes to specific human chromosomes. (D'Eustachio P. et al. (1983) *Science* 220:919-924).

Other mapping strategies e.g., *in situ* hybridization (described in Fan, Y. et al. (1990) *Proc. Natl. Acad. Sci. USA,* 87:6223-27), pre-screening with labeled flow-sorted chromosomes, and pre-selection by hybridization to chromosome specific cDNA libraries can be used to map 58297 to a chromosomal location.

Fluorescence *in situ* hybridization (FISH) of a DNA sequence to a metaphase chromosomal spread can further be used to provide a precise chromosomal location in one step. The FISH technique can be used with a DNA sequence as short as 500 or 600 bases. However, clones larger than 1,000 bases have a higher likelihood of binding to a unique chromosomal location with sufficient signal intensity for simple detection. Preferably 1,000 bases, and more preferably 2,000 bases will suffice to get good results at a reasonable amount of time. For a review of this technique, see Verma et al., Human Chromosomes: A Manual of Basic Techniques ((1988) Pergamon Press, New York).

Reagents for chromosome mapping can be used individually to mark a single chromosome or a single site on that chromosome, or panels of reagents can be used for marking multiple sites and/or multiple chromosomes. Reagents corresponding to noncoding regions of the genes actually are preferred for mapping purposes. Coding sequences are more likely to be conserved within gene families, thus increasing the chance of cross hybridizations during chromosomal mapping.

Once a sequence has been mapped to a precise chromosomal location, the physical position of the sequence on the chromosome can be correlated with genetic map data. (Such data are found, for example, in V. McKusick, Mendelian Inheritance in Man, available on-line through Johns Hopkins University Welch Medical Library). The relationship between a gene and a disease, mapped to the same chromosomal region, can then be identified through linkage analysis (co-inheritance of physically adjacent genes), described in, for example, Egeland, J. et al. (1987) *Nature,* 325:783-787.

Moreover, differences in the DNA sequences between individuals affected and unaffected with a disease associated with the 58297 gene, can be determined. If a mutation is observed in some or all of the affected individuals but not in any unaffected individuals, then the mutation is likely to be the causative agent of the particular disease. Comparison of affected and unaffected individuals generally involves first looking for structural alterations in the chromosomes, such as deletions or translocations that are visible from chromosome spreads or detectable using PCR based on that DNA sequence. Ultimately, complete sequencing of genes from several individuals can be performed to confirm the presence of a mutation and to distinguish mutations from polymorphisms.

### 1.9 Tissue Typing

58297 sequences can be used to identify individuals from biological samples using, e.g., restriction fragment length polymorphism (RFLP). In this technique, an individual's genomic DNA is digested with one or more restriction enzymes, the fragments separated, e.g., in a Southern blot, and probed to yield bands for identification. The sequences of the present invention are useful as additional DNA markers for RFLP (described in U.S. Patent 5,272,057).

Furthermore, the sequences of the present invention can also be used to determine the actual base-by-base DNA sequence of selected portions of an individual's genome. Thus, the 58297 nucleotide sequences described herein can be used to prepare two PCR primers from the 5' and 3' ends of the sequences. These primers can then be used to amplify an individual's DNA and subsequently sequence it. Panels of corresponding DNA sequences from individuals, prepared in this manner, can provide unique individual identifications, as each individual will have a unique set of such DNA sequences due to allelic differences.

Allelic variation occurs to some degree in the coding regions of these sequences, and to a greater degree in the noncoding regions. Each of the sequences described herein can, to some degree, be used as a standard against which DNA from an individual can be compared for identification purposes. Because greater numbers of polymorphisms occur in the noncoding regions, fewer sequences are necessary to differentiate individuals. The noncoding sequences of SEQ ID NO:1 can provide positive individual identification with a panel of perhaps 10 to 1,000 primers which each yield a noncoding amplified sequence of 100 bases. If coding sequences, such as those in SEQ ID NO:3 are used, a more appropriate number of primers for positive individual identification would be 500-2,000.

If a panel of reagents from 58297 nucleotide sequences described herein is used to generate a unique identification database for an individual, those same reagents can later be used to identify tissue from that individual. Using the unique identification database, positive identification of the individual, living or dead, can be made from extremely small tissue samples.

### 1.10 Use of Partial 58297 Sequences in Forensic Biology

DNA-based identification techniques can also be used in forensic biology. To make such an identification, PCR technology can be used to amplify DNA sequences taken from very small biological samples such as tissues, e.g., hair or skin, or body fluids, e.g., blood, saliva, or semen found at a crime scene. The amplified sequence can then be compared to a standard, thereby allowing identification of the origin of the biological sample.

The sequences of the present invention can be used to provide polynucleotide reagents, e.g., PCR primers, targeted to specific loci in the human genome, which can enhance the reliability of DNA-based forensic identifications by, for example, providing another "identification marker" (i.e. another DNA sequence that is unique to a particular individual). As mentioned above, actual base sequence information can be used for identification as an accurate alternative to patterns formed by restriction enzyme generated fragments. Sequences targeted to noncoding regions of SEQ ID NO:1 (e.g., fragments derived from the noncoding regions of SEQ ID NO:1 having a length of at least 20 bases, preferably at least 30 bases) are particularly appropriate for this use.

The 58297 nucleotide sequences described herein can further be used to provide polynucleotide reagents, e.g., labeled or labelable probes which can be used in, for example, an *in situ* hybridization technique, to identify a specific tissue. This can be very useful in cases where a forensic pathologist is presented with a tissue of unknown origin. Panels of such 58297 probes can be used to identify tissue by species and/or by organ type.

In a similar fashion, these reagents, e.g., 58297 primers or probes can be used to screen tissue culture for contamination (i.e. screen for the presence of a mixture of different types of cells in a culture).

### 1.11 Predictive Medicine

The present invention also pertains to the field of predictive medicine in which diagnostic assays, prognostic assays, and monitoring clinical trials are used for prognostic (predictive) purposes to thereby treat an individual.

Generally, the invention provides, a method of determining if a subject is at risk for a disorder related to a lesion in or the misexpression of a gene which encodes 58297.

Such disorders include, e.g., a disorder associated with the misexpression of 58297 gene.

The method includes one or more of the following:

detecting, in a tissue of the subject, the presence or absence of a mutation which affects the expression of the 58297 gene, or detecting the presence or absence of a mutation in a region which controls the expression of the gene, e.g., a mutation in the 5' control region;

detecting, in a tissue of the subject, the presence or absence of a mutation which alters the structure of the 58297 gene;

detecting, in a tissue of the subject, the misexpression of the 58297 gene, at the mRNA level, e.g., detecting a non-wild type level of a mRNA; or

detecting, in a tissue of the subject, the misexpression of the gene, at the protein level, e.g., detecting a non-wild type level of a 58297 polypeptide.

In preferred embodiments the method includes: ascertaining the existence of at least one of: a deletion of one or more nucleotides from the 58297 gene; an insertion of one or more nucleotides into the gene, a point mutation, e.g., a substitution of one or more nucleotides of the gene, a gross chromosomal rearrangement of the gene, e.g., a translocation, inversion, or deletion.

For example, detecting the genetic lesion can include: (i) providing a probe/primer including an oligonucleotide containing a region of nucleotide sequence which hybridizes to a sense or antisense sequence from SEQ ID NO:1, or naturally occurring mutants thereof or 5' or 3' flanking sequences naturally associated with the 58297 gene; (ii) exposing the probe/primer to nucleic acid of the tissue; and detecting, by hybridization, e.g., *in situ* hybridization, of the probe/primer to the nucleic acid, the presence or absence of the genetic lesion.

In preferred embodiments detecting the misexpression includes ascertaining the existence of at least one of: an alteration in the level of a messenger RNA transcript of the 58297 gene; the presence of a non-wild type splicing pattern of a messenger RNA transcript of the gene; or a non-wild type level of 58297.

Methods of the invention can be used prenatally or to determine if a subject's offspring will be at risk for a disorder.

In preferred embodiments the method includes determining the structure of a 58297 gene, an abnormal structure being indicative of risk for the disorder.

In preferred embodiments the method includes contacting a sample from the subject with an antibody to the 58297 protein or a nucleic acid, which hybridizes specifically with the gene. These and other embodiments are discussed below.

### 1.12 Diagnostic and Prognostic Assays

The presence, level, or absence of 58297 protein or nucleic acid in a biological sample can be evaluated by obtaining a biological sample from a test subject and contacting the biological sample with a compound or an agent capable of detecting 58297 protein or nucleic acid (e.g., mRNA, genomic DNA) that encodes 58297 protein such that the presence of 58297 protein or nucleic acid is detected in the biological sample. The term "biological sample" includes tissues, cells and biological fluids isolated from a subject, as well as tissues, cells and fluids present within a subject. A preferred biological sample is serum. The level of expression of the 58297 gene can be measured in a number of ways, including, but not limited to: measuring the mRNA encoded by the 58297 genes; measuring the amount of protein encoded by the 58297 genes; or measuring the activity of the protein encoded by the 58297 genes.

The level of mRNA corresponding to the 58297 gene in a cell can be determined both by *in situ* and by *in vitro* formats.

The isolated mRNA can be used in hybridization or amplification assays that include, but are not limited to, Southern or Northern analyses, polymerase chain reaction analyses and probe arrays. One preferred diagnostic method for the detection of mRNA levels involves contacting the isolated mRNA with a nucleic acid molecule (probe) that can hybridize to the mRNA encoded by the gene being detected. The nucleic acid probe can be, for example, a full-length 58297 nucleic acid, such as the nucleic acid of SEQ ID NO:1, or a portion thereof, such as an oligonucleotide of at least 7, 15, 30, 50, 100, 250 or 500 nucleotides in length and sufficient to specifically hybridize under stringent conditions to 58297 mRNA or genomic DNA. Other suitable probes for use in the diagnostic assays are described herein.

In one format, mRNA (or cDNA) is immobilized on a surface and contacted with the probes, for example by running the isolated mRNA on an agarose gel and transferring the mRNA from the gel to a membrane, such as nitrocellulose. In an alternative format, the probes are immobilized on a surface and the mRNA (or cDNA) is contacted with the probes, for example, in a two-dimensional gene chip array. A skilled artisan can adapt known mRNA detection methods for use in detecting the level of mRNA encoded by the 58297 genes.

The level of mRNA in a sample that is encoded by one of 58297 can be evaluated with nucleic acid amplification, e.g., by rtPCR (Mullis (1987) U.S. Patent No. 4,683,202), ligase chain reaction (Barany (1991) *Proc. Natl. Acad. Sci. USA* 88:189-193), self sustained sequence replication (Guatelli et al., (1990) *Proc. Natl. Acad. Sci. USA* 87:1874-1878), transcriptional amplification system (Kwoh et al., (1989), *Proc. Natl. Acad. Sci. USA* 86:1173-1177), Q-Beta Replicase (Lizardi et al., (1988) *Bio*/*Technology* 6:1197), rolling circle replication (Lizardi et al., U.S. Patent No. 5,854,033) or any other nucleic acid amplification method, followed by the detection of the amplified molecules using techniques known in the art. As used herein, amplification primers are defined as being a pair of nucleic acid molecules that can anneal to 5' or 3' regions of a gene (plus and minus strands, respectively, or vice-versa) and contain a short region in between. In general, amplification primers are from about 10 to 30 nucleotides in length and flank a region from about 50 to 200 nucleotides in length. Under appropriate conditions and with appropriate reagents, such primers permit the amplification of a nucleic acid molecule comprising the nucleotide sequence flanked by the primers.

For *in situ* methods, a cell or tissue sample can be prepared/processed and immobilized on a support, typically a glass slide, and then contacted with a probe that can hybridize to mRNA that encodes the 58297 gene being analyzed.

In another embodiment, the methods further contacting a control sample with a compound or agent capable of detecting 58297 mRNA, or genomic DNA, and comparing the presence of 58297 mRNA or genomic DNA in the control sample with the presence of 58297 mRNA or genomic DNA in the test sample.

A variety of methods can be used to determine the level of protein encoded by 58297. In general, these methods include contacting an agent that selectively binds to the protein, such as an antibody with a sample, to evaluate the level of protein in the sample. In a preferred embodiment, the antibody bears a detectable label. Antibodies can be polyclonal, or more preferably, monoclonal. An intact antibody, or a fragment thereof (e.g., Fab or F(ab')₂) can be used. The term "labeled", with regard to the probe or antibody, is intended to encompass direct labeling of the probe or antibody by coupling (i.e., physically linking) a detectable substance to the probe or antibody, as well as indirect labeling of the probe or antibody by reactivity with a detectable substance. Examples of detectable substances are provided herein.

The detection methods can be used to detect 58297 protein in a biological sample *in vitro,* as well as *in vivo. In vitro* techniques for detection of 58297 protein include enzyme linked immunosorbent assays (ELISAs), immunoprecipitations, immunofluorescence, enzyme immunoassay (EIA), radioimmunoassay (RIA), and Western blot analysis. *In vivo* techniques for detection of 58297 protein include introducing into a subject a labeled anti-58297 antibody. For example, the antibody can be labeled with a radioactive marker whose presence and location in a subject can be detected by standard imaging techniques.

In another embodiment, the methods further include contacting the control sample with a compound or agent capable of detecting 58297 protein, and comparing the presence of 58297 protein in the control sample with the presence of 58297 protein in the test sample.

The invention also includes kits for detecting the presence of 58297 in a biological sample. For example, the kit can include a compound or agent capable of detecting 58297 protein or mRNA in a biological sample; and a standard. The compound or agent can be packaged in a suitable container. The kit can further comprise instructions for using the kit to detect 58297 protein or nucleic acid.

For antibody-based kits, the kit can include: (1) a first antibody (e.g., attached to a solid support) which binds to a polypeptide corresponding to a marker of the invention; and, optionally, (2) a second, different antibody which binds to either the polypeptide or the first antibody and is conjugated to a detectable agent.

For oligonucleotide-based kits, the kit can include: (1) an oligonucleotide, e.g., a detectably labeled oligonucleotide, which hybridizes to a nucleic acid sequence encoding a polypeptide corresponding to a marker of the invention, or (2) a pair of primers useful for amplifying a nucleic acid molecule corresponding to a marker of the invention. The kit can also includes a buffering agent, a preservative, or a protein stabilizing agent. The kit can also includes components necessary for detecting the detectable agent (e.g., an enzyme or a substrate). The kit can also contain a control sample or a series of control samples which can be assayed and compared to the test sample contained. Each component of the kit can be enclosed within an individual container and all of the various containers can be within a single package, along with instructions for interpreting the results of the assays performed using the kit.

The diagnostic methods described herein can identify subjects having, or at risk of developing, a disease or disorder associated with misexpressed or aberrant or unwanted 58297 expression or activity. As used interchangeably herein, the terms "unwanted" and "undesirable" include an unwanted phenomenon involved in a biological response such as pain or deregulated cell proliferation.

In one embodiment, a disease or disorder associated with aberrant or unwanted 58297 expression or activity is identified. A test sample is obtained from a subject and 58297 protein or nucleic acid (e.g., mRNA or genomic DNA) is evaluated, wherein the level, e.g., the presence or absence, of 58297 protein or nucleic acid is diagnostic for a subject having or at risk of developing a disease or disorder associated with aberrant or unwanted 58297 expression or activity. As used herein, a "test sample" refers to a biological sample obtained from a subject of interest, including a biological fluid (e.g., serum), cell sample, or tissue.

The prognostic assays described herein can be used to determine whether a subject can be administered an agent (e.g., an agonist, antagonist, peptidomimetic, protein, peptide, nucleic acid, small molecule, or other drug candidate) to treat a disease or disorder associated with aberrant or unwanted 58297 expression or activity. For example, such methods can be used to determine whether a subject can be effectively treated with an agent for a cellular proliferative and/or differentiative disorder, a hormonal disorder, an immune or inflammatory disorder, a neurological disorder, a cardiovascular disorder, a blood vessel disorder, or a platelet disorder.

The methods of the invention can also be used to detect genetic alterations in a 58297 gene, thereby determining if a subject with the altered gene is at risk for a disorder characterized by misregulation in 58297 protein activity or nucleic acid expression, such as a cellular proliferative and/or differentiative disorder, a hormonal disorder, an immune or inflammatory disorder, a neurological disorder, a cardiovascular disorder, a blood vessel disorder, or a platelet disorder. In preferred embodiments, the methods include detecting, in a sample from the subject, the presence or absence of a genetic alteration characterized by at least one of an alteration affecting the integrity of a gene encoding a 58297 protein, or the mis-expression of the 58297 gene. For example, such genetic alterations can be detected by ascertaining the existence of at least one of 1) a deletion of one or more nucleotides from a 58297 gene; 2) an addition of one or more nucleotides to a 58297 gene; 3) a substitution of one or more nucleotides of a 58297 gene, 4) a chromosomal rearrangement of a 58297 gene; 5) an alteration in the level of a messenger RNA transcript of a 58297 gene, 6) aberrant modification of a 58297 gene, such as of the methylation pattern of the genomic DNA, 7) the presence of a non-wild type splicing pattern of a messenger RNA transcript of a 58297 gene, 8) a non-wild type level of a 58297 protein, 9) allelic loss of a 58297 gene, and 10) inappropriate post-translational modification of a 58297 protein.

An alteration can be detected without a probe/primer in a polymerase chain reaction, such as anchor PCR or RACE PCR, or, alternatively, in a ligation chain reaction (LCR), the latter of which can be particularly useful for detecting point mutations in the 58297 gene. This method can include the steps of collecting a sample of cells from a subject, isolating nucleic acid (e.g., genomic, mRNA or both) from the sample, contacting the nucleic acid sample with one or more primers which specifically hybridize to a 58297 gene under conditions such that hybridization and amplification of the 58297 gene (if present) occurs, and detecting the presence or absence of an amplification product, or detecting the size of the amplification product and comparing the length to a control sample. It is anticipated that PCR and/or LCR may be desirable to use as a preliminary amplification step in conjunction with any of the techniques used for detecting mutations described herein. Alternatively, other amplification methods described herein or known in the art can be used.

In another embodiment, mutations in a 58297 gene from a sample cell can be identified by detecting alterations in restriction enzyme cleavage patterns. For example, sample and control DNA is isolated, amplified (optionally), digested with one or more restriction endonucleases, and fragment length sizes are determined, e.g., by gel electrophoresis and compared. Differences in fragment length sizes between sample and control DNA indicates mutations in the sample DNA. Moreover, the use of sequence specific ribozymes (see, for example, U.S. Patent No. 5,498,531) can be used to score for the presence of specific mutations by development or loss of a ribozyme cleavage site.

In other embodiments, genetic mutations in 58297 can be identified by hybridizing a sample and control nucleic acids, e.g., DNA or RNA, two dimensional arrays, e.g., chip based arrays. Such arrays include a plurality of addresses, each of which is positionally distinguishable from the other. A different probe is located at each address of the plurality. The arrays can have a high density of addresses, e.g., can contain hundreds or thousands of oligonucleotides probes (Cronin, M.T. et al. (1996) *Human Mutation* 7: 244-255; Kozal, M.J. et al. (1996) *Nature Medicine* 2: 753-759). For example, genetic mutations in 58297 can be identified in two dimensional arrays containing light-generated DNA probes as described in Cronin, M.T. et al. *supra.* Briefly, a first hybridization array of probes can be used to scan through long stretches of DNA in a sample and control to identify base changes between the sequences by making linear arrays of sequential overlapping probes. This step allows the identification of point mutations. This step is followed by a second hybridization array that allows the characterization of specific mutations by using smaller, specialized probe arrays complementary to all variants or mutations detected. Each mutation array is composed of parallel probe sets, one complementary to the wild-type gene and the other complementary to the mutant gene.

In yet another embodiment, any of a variety of sequencing reactions known in the art can be used to directly sequence the 58297 gene and detect mutations by comparing the sequence of the sample 58297 with the corresponding wild-type (control) sequence. Automated sequencing procedures can be utilized when performing the diagnostic assays ((1995) *Biotechniques* 19:448), including sequencing by mass spectrometry.

Other methods for detecting mutations in the 58297 gene include methods in which protection from cleavage agents is used to detect mismatched bases in RNA/RNA or RNA/DNA heteroduplexes (Myers et al. (1985) *Science* 230:1242; Cotton et al. (1988) *Proc. Natl Acad Sci USA* 85:4397; Saleeba et al. (1992) *Methods Enzymol.* 217:286-295).

In still another embodiment, the mismatch cleavage reaction employs one or more proteins that recognize mismatched base pairs in double-stranded DNA (so called "DNA mismatch repair" enzymes) in defined systems for detecting and mapping point mutations in 58297 cDNAs obtained from samples of cells. For example, the mutY enzyme of *E. coli* cleaves A at G/A mismatches and the thymidine DNA glycosylase from HeLa cells cleaves T at G/T mismatches (Hsu et al. (1994) *Carcinogenesis* 15:1657-1662; U.S. Patent No. 5,459,039).

In other embodiments, alterations in electrophoretic mobility will be used to identify mutations in 58297 genes. For example, single strand conformation polymorphism (SSCP) may be used to detect differences in electrophoretic mobility between mutant and wild type nucleic acids (Orita et al. (1989) *Proc Natl. Acad. Sci USA:* 86:2766, see also Cotton (1993) *Mutat. Res.* 285:125-144; and Hayashi (1992) *Genet. Anal. Tech. Appl.* 9:73-79). Single-stranded DNA fragments of sample and control 58297 nucleic acids will be denatured and allowed to renature. The secondary structure of single-stranded nucleic acids varies according to sequence, the resulting alteration in electrophoretic mobility enables the detection of even a single base change. The DNA fragments may be labeled or detected with labeled probes. The sensitivity of the assay may be enhanced by using RNA (rather than DNA), in which the secondary structure is more sensitive to a change in sequence. In a preferred embodiment, the subject method utilizes heteroduplex analysis to separate double stranded heteroduplex molecules on the basis of changes in electrophoretic mobility (Keen et al. (1991) *Trends Genet* 7:5).

In yet another embodiment, the movement of mutant or wild-type fragments in polyacrylamide gels containing a gradient of denaturant is assayed using denaturing gradient gel electrophoresis (DGGE) (Myers et al. (1985) *Nature* 313:495). When DGGE is used as the method of analysis, DNA will be modified to insure that it does not completely denature, for example by adding a GC clamp of approximately 40 bp of high-melting GC-rich DNA by PCR. In a further embodiment, a temperature gradient is used in place of a denaturing gradient to identify differences in the mobility of control and sample DNA (Rosenbaum and Reissner (1987) *Biophys Chem* 265:12753).

Examples of other techniques for detecting point mutations include, but are not limited to, selective oligonucleotide hybridization, selective amplification, or selective primer extension (Saiki et al. (1986) *Nature* 324:163); Saiki et al. (1989) *Proc. Natl Acad. Sci USA* 86:6230).

Alternatively, allele specific amplification technology which depends on selective PCR amplification may be used in conjunction with the instant invention. Oligonucleotides used as primers for specific amplification may carry the mutation of interest in the center of the molecule (so that amplification depends on differential hybridization) (Gibbs et al. (1989) *Nucleic Acids Res.* 17:2437-2448) or at the extreme 3' end of one primer where, under appropriate conditions, mismatch can prevent, or reduce polymerase extension (Prossner (1993) *Tibtech* 11:238). In addition it may be desirable to introduce a novel restriction site in the region of the mutation to create cleavage-based detection (Gasparini et al. (1992) *Mol. Cell Probes* 6:1). It is anticipated that in certain embodiments amplification may also be performed using Taq ligase for amplification (Barany (1991) *Proc. Natl. Acad. Sci USA* 88:189). In such cases, ligation will occur only if there is a perfect match at the 3' end of the 5' sequence making it possible to detect the presence of a known mutation at a specific site by looking for the presence or absence of amplification.

The methods described herein may be performed, for example, by utilizing pre-packaged diagnostic kits comprising at least one probe nucleic acid or antibody reagent described herein, which may be conveniently used, *e.g*., in clinical settings to diagnose patients exhibiting symptoms or family history of a disease or illness involving a 58297 gene.

### Use of 58297 Molecules as Surrogate Markers

The 58297 molecules of the invention are also useful as markers of disorders or disease states, as markers for precursors of disease states, as markers for predisposition of disease states, as markers of drug activity, or as markers of the pharmacogenomic profile of a subject. Using the methods described herein, the presence, absence and/or quantity of the 58297 molecules of the invention may be detected, and may be correlated with one or more biological states *in vivo.* For example, the 58297 molecules of the invention may serve as surrogate markers for one or more disorders or disease states or for conditions leading up to disease states. As used herein, a "surrogate marker" is an objective biochemical marker which correlates with the absence or presence of a disease or disorder, or with the progression of a disease or disorder (e.g., with the presence or absence of a tumor). The presence or quantity of such markers is independent of the disease. Therefore, these markers may serve to indicate whether a particular course of treatment is effective in lessening a disease state or disorder. Surrogate markers are of particular use when the presence or extent of a disease state or disorder is difficult to assess through standard methodologies (e.g., early stage tumors), or when an assessment of disease progression is desired before a potentially dangerous clinical endpoint is reached (e.g., an assessment of cardiovascular disease may be made using cholesterol levels as a surrogate marker, and an analysis of HIV infection may be made using HIV RNA levels as a surrogate marker, well in advance of the undesirable clinical outcomes of myocardial infarction or fully-developed AIDS). Examples of the use of surrogate markers in the art include: Koomen et al. (2000) J. Mass. Spectrom. 35: 258-264; and James (1994) *AIDS Treatment News Archive* 209.

The 58297 molecules of the invention are also useful as pharmacodynamic markers. As used herein, a "pharmacodynamic marker" is an objective biochemical marker which correlates specifically with drug effects. The presence or quantity of a pharmacodynamic marker is not related to the disease state or disorder for which the drug is being administered; therefore, the presence or quantity of the marker is indicative of the presence or activity of the drug in a subject. For example, a pharmacodynamic marker may be indicative of the concentration of the drug in a biological tissue, in that the marker is either expressed or transcribed or not expressed or transcribed in that tissue in relationship to the level of the drug. In this fashion, the distribution or uptake of the drug may be monitored by the pharmacodynamic marker. Similarly, the presence or quantity of the pharmacodynamic marker may be related to the presence or quantity of the metabolic product of a drug, such that the presence or quantity of the marker is indicative of the relative breakdown rate of the drug in vivo. Pharmacodynamic markers are of particular use in increasing the sensitivity of detection of drug effects, particularly when the drug is administered in low doses. Since even a small amount of a drug may be sufficient to activate multiple rounds of marker (e.g., a 58297 marker) transcription or expression, the amplified marker may be in a quantity which is more readily detectable than the drug itself. Also, the marker may be more easily detected due to the nature of the marker itself; for example, using the methods described herein, anti-58297 antibodies may be employed in an immune-based detection system for a 58297 protein marker, or 58297-specific radiolabeled probes may be used to detect a 58297 mRNA marker. Furthermore, the use of a pharmacodynamic marker may offer mechanism-based prediction of risk due to drug treatment beyond the range of possible direct observations. Examples of the use of pharmacodynamic markers in the art include: Matsuda et al. US 6,033,862; Hattis et al. (1991) Env. Health Perspect. 90: 229-238; Schentag (1999) *Am. J. Health-Syst. Pharm.* 56 Suppl. 3: S21-S24; and Nicolau (1999) *Am, J. Health-Syst. Pharm.* 56 Suppl. 3: S16-S20.

The 58297 molecules of the invention are also useful as pharmacogenomic markers. As used herein, a "pharmacogenomic marker" is an objective biochemical marker which correlates with a specific clinical drug response or susceptibility in a subject (see, e.g., McLeod et al. (1999) *Eur. J. Cancer* 35:1650-1652). The presence or quantity of the pharmacogenomic marker is related to the predicted response of the subject to a specific drug or class of drugs prior to administration of the drug. By assessing the presence or quantity of one or more pharmacogenomic markers in a subject, a drug therapy which is most appropriate for the subject, or which is predicted to have a greater degree of success, may be selected. For example, based on the presence or quantity of RNA, or protein (e.g., 58297 protein or RNA) for specific tumor markers in a subject, a drug or course of treatment may be selected that is optimized for the treatment of the specific tumor likely to be present in the subject.
Similarly, the presence or absence of a specific sequence mutation in 58297 DNA may correlate 58297 drug response. The use of pharmacogenomic markers therefore permits the application of the most appropriate treatment for each subject without having to administer the therapy.

### 1.13 Pharmaceutical Compositions

The nucleic acid and polypeptides, fragments thereof, as well as anti-58297 antibodies and small molecule modulators of 58297 molecules (also referred to herein as "active compounds") of the invention can be incorporated into pharmaceutical compositions. Such compositions typically include the nucleic acid molecule, protein, or antibody and a pharmaceutically acceptable carrier. As used herein, a "pharmaceutically acceptable carrier" includes solvents, dispersion media, coatings, antibacterial and antifungal agents, isotonic and absorption delaying agents, and the like, compatible with pharmaceutical administration. Supplementary active compounds can also be incorporated into the compositions.

A pharmaceutical composition is formulated to be compatible with its intended route of administration. Examples of routes of administration include parenteral, e.g., intravenous, intradermal, subcutaneous, oral (e.g., inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany NJ) or phosphate buffered saline (PBS). In all cases, the composition must be sterile and should be fluid to the extent that easy syringability exists. It should be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, sodium chloride in the composition. Prolonged absorption of the injectable compositions can be brought about by including in the composition an agent which delays absorption, for example, aluminum monostearate and gelatin.Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions generally include an inert diluent or an edible carrier. For the purpose of oral therapeutic administration, the active compound can be incorporated with excipients and used in the form of tablets, troches, or capsules, e.g., gelatin capsules. Oral compositions can also be prepared using a fluid carrier for use as a mouthwash. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser which contains a suitable propellant, e.g., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (e.g., with conventional suppository bases such as cocoa butter and other glycerides) or retention enemas for rectal delivery.

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation (Palo Alto CA) and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Patent No. 4,522,811.

It is advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier.

Toxicity and therapeutic efficacy of such compounds can be determined by standard pharmaceutical procedures in cell cultures or experimental animals, *e.g*., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Compounds which exhibit high therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED₅₀ with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range that includes the IC₅₀ (i.e., the concentration of the test compound which achieves a half-maximal inhibition of symptoms) as determined in cell culture. Such information can be used to more accurately determine useful doses in humans. Levels in plasma may be measured, for example, by high performance liquid chromatography.

As defined herein, a therapeutically effective amount of protein or polypeptide (i.e., an effective dosage) ranges from about 0.001 to 30 mg/kg body weight, preferably about 0.01 to 25 mg/kg body weight, more preferably about 0.1 to 20 mg/kg body weight, and even more preferably about 1 to 10 mg/kg, 2 to 9 mg/kg, 3 to 8 mg/kg, 4 to 7 mg/kg, or 5 to 6 mg/kg body weight. The protein or polypeptide can be administered one time per week for between about 1 to 10 weeks, preferably between 2 to 8 weeks, more preferably between about 3 to 7 weeks, and even more preferably for about 4, 5, or 6 weeks. The skilled artisan will appreciate that certain factors may influence the dosage and timing required to effectively treat a subject, including but not limited to the severity of the disease or disorder, previous treatments, the general health and/or age of the subject, and other diseases present. Moreover, treatment of a subject with a therapeutically effective amount of a protein, polypeptide, or antibody can include a single treatment or, preferably, can include a series of treatments.

For antibodies, the preferred dosage is 0.1 mg/kg of body weight (generally 10 mg/kg to 20 mg/kg). If the antibody is to act in the brain, a dosage of 50 mg/kg to 100 mg/kg is usually appropriate. Generally, partially human antibodies and fully human antibodies have a longer half-life within the human body than other antibodies. Accordingly, lower dosages and less frequent administration is often possible. Modifications such as lipidation can be used to stabilize antibodies and to enhance uptake and tissue penetration (e.g., into the brain). A method for lipidation of antibodies is described by Cruikshank et al. ((1997) *J. Acquired Immune Deficiency Syndromes and Human Retrovirology* 14:193).

The present invention encompasses agents which modulate expression or activity. An agent may, for example, be a small molecule. For example, such small molecules include, but are not limited to, peptides, peptidomimetics (e.g., peptoids), amino acids, amino acid analogs, polynucleotides, polynucleotide analogs, nucleotides, nucleotide analogs, organic or inorganic compounds (i.e.,. including heteroorganic and organometallic compounds) having a molecular weight less than about 10,000 grams per mole, organic or inorganic compounds having a molecular weight less than about 5,000 grams per mole, organic or inorganic compounds having a molecular weight less than about 1,000 grams per mole, organic or inorganic compounds having a molecular weight less than about 500 grams per mole, and salts, esters, and other pharmaceutically acceptable forms of such compounds.

Exemplary doses include milligram or microgram amounts of the small molecule per kilogram of subject or sample weight (e.g., about 1 microgram per kilogram to about 500 milligrams per kilogram, about 100 micrograms per kilogram to about 5 milligrams per kilogram, or about 1 microgram per kilogram to about 50 micrograms per kilogram. It is furthermore understood that appropriate doses of a small molecule depend upon the potency of the small molecule with respect to the expression or activity to be modulated. When one or more of these small molecules is to be administered to an animal (e.g., a human) in order to modulate expression or activity of a polypeptide or nucleic acid of the invention, a physician, veterinarian, or researcher may, for example, prescribe a relatively low dose at first, subsequently increasing the dose until an appropriate response is obtained. In addition, it is understood that the specific dose level for any particular animal subject will depend upon a variety of factors including the activity of the specific compound employed, the age, body weight, general health, gender, and diet of the subject, the time of administration, the route of administration, the rate of excretion, any drug combination, and the degree of expression or activity to be modulated.

An antibody (or fragment thereof) may be conjugated to a therapeutic moiety such as a cytotoxin, a therapeutic agent or a radioactive agent (e.g., a radioactive metal ion). A cytotoxin or cytotoxic agent includes any agent that is detrimental to cells. Examples include taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof. Therapeutic agents include, but are not limited to, antimetabolites (e.g., methotrexate, 6-mercaptopurine, 6-thioguanine, cytarabine, 5-fluorouracil decarbazine), alkylating agents (e.g., mechlorethamine, thioepa chlorambucil, melphalan, carmustine (BSNU) and lomustine (CCNU), cyclothosphamide, busulfan, dibromomannitol, streptozotocin, mitomycin C, and cis-dichlorodiamine platinum (II) (DDP) cisplatin), anthracyclines (e.g., daunorubicin (formerly daunomycin) and doxorubicin), antibiotics (e.g., dactinomycin (formerly actinomycin), bleomycin, mithramycin, and anthramycin (AMC)), and anti-mitotic agents (e.g., vincristine and vinblastine).

The conjugates of the invention can be used for modifying a given biological response, the drug moiety is not to be construed as limited to classical chemical therapeutic agents. For example, the drug moiety may be a protein or polypeptide possessing a desired biological activity. Such proteins may include, for example, a toxin such as abrin, ricin A, pseudomonas exotoxin, or diphtheria toxin; a protein such as tumor necrosis factor, □-interferon, □-interferon, nerve growth factor, platelet derived growth factor, tissue plasminogen activator; or, biological response modifiers such as, for example, lymphokines, interleukin-1 ("IL-1"), interleukin-2 ("IL-2"), interleukin-6 ("IL-6"), granulocyte macrophase colony stimulating factor ("GM-CSF"), granulocyte colony stimulating factor ("G-CSF"), or other growth factors.

Techniques for conjugating a therapeutic moiety to an antibody are well known (see, e.g., Arnon et al., 1985, "Monoclonal Antibodies For Immunotargeting Of Drugs In Cancer Therapy", in Monoclonal Antibodies And Cancer Therapy, Reisfeld et al., Eds., Alan R. Liss, Inc. pp. 243-256; Hellstrom et al., 1987, "Antibodies For Drug Delivery", in Controlled Drug Delivery, 2nd ed., Robinson et al., Eds., Marcel Dekker, Inc., pp. 623-653; Thorpe, 1985, "Antibody Carriers Of Cytotoxic Agents In Cancer Therapy: A Review", in Monoclonal Antibodies '84: Biological And Clinical Applications, Pinchera et al., Eds., pp. 475-506; "Analysis, Results, And Future Prospective Of The Therapeutic Use Of Radiolabeled Antibody In Cancer Therapy", in Monoclonal Antibodies For Cancer Detection And Therapy, Baldwin et al., Eds., Academic Press, pp. 303-316, 1985; and Thorpe et al., 1982, Immunol. Rev., 62:119-158). Alternatively, an antibody can be conjugated to a second antibody to form an antibody heteroconjugate as described by Segal in U.S. Patent No. 4,676,980.

The nucleic acid molecules of the invention can be inserted into vectors and used as gene therapy vectors. Gene therapy vectors can be delivered to a subject by, for example, intravenous injection, local administration (see U.S. Patent 5,328,470) or by stereotactic injection (see e.g., Chen et al. (1994) *Proc. Natl. Acad. Sci. USA* 91:3054-3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, e.g., retroviral vectors, the pharmaceutical preparation can include one or more cells which produce the gene delivery system.

The pharmaceutical compositions can be included in a container, pack, or dispenser together with instructions for administration.

### 1.14 Methods of Treatment

The present invention provides for both prophylactic and therapeutic methods of treating a subject at risk of (or susceptible to) a disorder or having a disorder associated with aberrant or undesirable 58297 expression or activity. With regard to both prophylactic and therapeutic methods of treatment, such treatments may be specifically tailored or modified, based on knowledge obtained from the field of pharmacogenomics. "Pharmacogenomics", as used herein, refers to the application of genomics technologies such as gene sequencing, statistical genetics, and gene expression analysis to drugs in clinical development and commercially available. More specifically, the term refers the study of how a patient's genes determine his or her response to a drug (e.g., a patient's "drug response phenotype", or "drug response genotype".) Thus, another aspect of the invention provides methods for tailoring an individual's prophylactic or therapeutic treatment with either the 58297 molecules of the present invention or 58297 modulators according to that individual's drug response genotype. Pharmacogenomics allows a clinician or physician to target prophylactic or therapeutic treatments to patients who will most benefit from the treatment and to identify patients who will experience toxic drug-related side effects.

"Treatment", as used herein, is defined as the application or administration of a therapeutic agent to a patient, or application or administration of a therapeutic agent to an isolated tissue or cell line from a patient, who has a disease, a symptom of disease or a predisposition toward a disease, with the purpose to cure, heal, alleviate, relieve, alter, remedy, ameliorate, palliate, improve or affect the disease, the symptoms of disease or the predisposition toward disease. A therapeutic agent includes, but is not limited to, small molecules, peptides, antibodies, ribozymes and antisense oligonucleotides.

In one aspect, the invention provides a method for preventing in a subject, a disease or condition associated with an aberrant or undesirable 58297 expression or activity, by administering to the subject a 58297 molecule or an agent which modulates 58297 expression or at least one 58297 activity. Subjects at risk for a disease which is caused or contributed to by aberrant or undesirable 58297 expression or activity can be identified by, for example, any or a combination of diagnostic or prognostic assays as described herein. Administration of a prophylactic agent can occur prior to the manifestation of symptoms characteristic of the 58297 aberrance, such that a disease or disorder is prevented or, alternatively, delayed in its progression. Depending on the type of 58297 aberrance, for example, a 58297 molecule (e.g., a 58297 nucleic acid molecule or a 58297 protein or polypeptide, or a fragment thereof, as described herein), or 58297 agonist or 58297 antagonist agent can be used for treating the subject. The appropriate agent can be determined based on screening assays described herein.

It is possible that some 58297 disorders can be caused, at least in part, by an abnormal level of gene product, or by the presence of a gene product exhibiting abnormal activity. As such, the reduction in the level and/or activity of such gene products would bring about the amelioration of disorder symptoms.

As discussed, successful treatment of 58297 disorders can be brought about by techniques that serve to inhibit the expression or activity of 58297 target gene products. For example, compounds, e.g., an agent identified using an assays described above, that proves to exhibit negative modulatory activity, can be used in accordance with the invention to prevent and/or ameliorate symptoms of 58297 disorders. Such molecules can include, but are not limited to peptides, phosphopeptides, small organic or inorganic molecules, or antibodies (including, for example, polyclonal, monoclonal, humanized, human, anti-idiotypic, chimeric or single chain antibodies, and Fab, F(ab')₂ and Fab expression library fragments, scFV molecules, and epitope-binding fragments thereof).

Further, antisense and ribozyme molecules that inhibit expression of the target gene can also be used in accordance with the invention to reduce the level of target gene expression, thus effectively reducing the level of target gene activity. Still further, triple helix molecules can be utilized in reducing the level of target gene activity. Antisense, ribozyme and triple helix molecules are discussed above.

It is possible that the use of antisense, ribozyme, and/or triple helix molecules to reduce or inhibit mutant gene expression can also reduce or inhibit the transcription (triple helix) and/or translation (antisense, ribozyme) of mRNA produced by normal target gene alleles, such that the concentration of normal target gene product present can be lower than is necessary for a normal phenotype. In such cases, nucleic acid molecules that encode and express target gene polypeptides exhibiting normal target gene activity can be introduced into cells via gene therapy method. Alternatively, in instances in that the target gene encodes an extracellular protein, it can be preferable to co-administer normal target gene protein into the cell or tissue in order to maintain the requisite level of cellular or tissue target gene activity.

Another method by which nucleic acid molecules may be utilized in treating or preventing a disease characterized by 58297 expression is through the use of aptamer molecules specific for 58297 protein. Aptamers are nucleic acid molecules having a tertiary structure which permits them to specifically bind to protein ligands (see, e.g., Osborne, et al. (1997) *Curr. Opin. Chem. Biol.* 1(1):5-9; and Patel, D.J. (1997) *Curr. Opin. Chem. Biol.* 1(1):32-46). Since nucleic acid molecules may in many cases be more conveniently introduced into target cells than therapeutic protein molecules may be, aptamers offer a method by which 58297 protein activity may be specifically decreased without the introduction of drugs or other molecules which may have pluripotent effects.

Antibodies can be generated that are both specific for target gene product and that reduce target gene product activity. Such antibodies may, therefore, by administered in instances whereby negative modulatory techniques are appropriate for the treatment of 58297 disorders. For a description of antibodies, see the Antibody section above.

In circumstances wherein injection of an animal or a human subject with a 58297 protein or epitope for stimulating antibody production is harmful to the subject, it is possible to generate an immune response against 58297 through the use of anti-idiotypic antibodies (see, for example, Herlyn, D. (1999) *Ann. Med.* 31(1):66-78; and Bhattacharya-Chatterjee, M., and Foon, K.A. (1998) *Cancer Treat. Res.* 94:51-68). If an anti-idiotypic antibody is introduced into a mammal or human subject, it should stimulate the production of anti-anti-idiotypic antibodies, which should be specific to the 58297 protein. Vaccines directed to a disease characterized by 58297 expression may also be generated in this fashion.

In instances where the target antigen is intracellular and whole antibodies are used, internalizing antibodies may be preferred. Lipofectin or liposomes can be used to deliver the antibody or a fragment of the Fab region that binds to the target antigen into cells. Where fragments of the antibody are used, the smallest inhibitory fragment that binds to the target antigen is preferred. For example, peptides having an amino acid sequence corresponding to the Fv region of the antibody can be used. Alternatively, single chain neutralizing antibodies that bind to intracellular target antigens can also be administered. Such single chain antibodies can be administered, for example, by expressing nucleotide sequences encoding single-chain antibodies within the target cell population (see e.g., Marasco et al. (1993) *Proc. Natl. Acad. Sci. USA* 90:7889-7893).

The identified compounds that inhibit target gene expression, synthesis and/or activity can be administered to a patient at therapeutically effective doses to prevent, treat or ameliorate 58297 disorders. A therapeutically effective dose refers to that amount of the compound sufficient to result in amelioration of symptoms of the disorders.

Toxicity and therapeutic efficacy of such compounds can be determined by standard pharmaceutical procedures in cell cultures or experimental animals, e.g., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Compounds that exhibit large therapeutic indices are preferred. While compounds that exhibit toxic side effects can be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED₅₀ with little or no toxicity. The dosage can vary within this range depending upon the dosage form employed and the route of administration utilized. For any compound used in the method of the invention, the therapeutically effective dose can be estimated initially from cell culture assays. A dose can be formulated in animal models to achieve a circulating plasma concentration range that includes the IC₅₀ (i.e., the concentration of the test compound that achieves a half-maximal inhibition of symptoms) as determined in cell culture. Such information can be used to more accurately determine useful doses in humans. Levels in plasma can be measured, for example, by high performance liquid chromatography.

Another measurement which can be used to determine the effective dose for an individual is to directly assay levels of "free" and "bound" compound in the serum of the test subject. Such assays may utilize antibody mimics and/or "biosensors" that have been created through molecular imprinting techniques. The compound which is able to modulate 58297 activity is used as a template, or "imprinting molecule", to spatially organize polymerizable monomers prior to their polymerization with catalytic reagents. The subsequent removal of the imprinted molecule leaves a polymer matrix which contains a repeated "negative image" of the compound and is able to selectively rebind the molecule under biological assay conditions. A detailed review of this technique is found in Ansell, R. J. et al. (1996) *Current Opinion in Biotechnology* 7:89-94 and in Shea, K.J. (1994) *Trends in Polymer Science* 2:166-173. Such "imprinted" affinity matrixes are amenable to ligand-binding assays, whereby the immobilized monoclonal antibody component is replaced by an appropriately imprinted matrix. An example of the use of such matrices in this way can be seen in Vlatakis, G. et al., (1993) *Nature* 361:645-647. Through the use of isotope-labeling, the "free" concentration of compound which modulates the expression or activity of 58297 can be readily monitored and used in calculations of IC₅₀.

Such "imprinted" affinity matrices can also be designed to include fluorescent groups whose photon-emitting properties measurably change upon local and selective binding of target compound. These changes can be readily assayed in real time using appropriate fiberoptic devices, in turn allowing the dose in a test subject to be quickly optimized based on its individual IC₅₀. A rudimentary example of such a "biosensor" is discussed in Kriz, D. et al. (1995) *Analytical Chemistry* 67:2142-2144.

Another aspect of the invention pertains to methods of modulating 58297 expression or activity for therapeutic purposes. Accordingly, in an exemplary embodiment, the modulatory method of the invention involves contacting a cell with a 58297 molecule (e.g., a 58297 nucleic acid molecule or 58297 protein or polypeptide, or a fragment thereof, as described herein) or an agent that modulates one or more of the activities of the 58297 protein activity associated with the cell. An agent that modulates 58297 protein activity can be an agent as described herein, such as a nucleic acid or a protein, a naturally-occurring target molecule of a 58297 protein (*e.g*., a 58297 substrate, ligand, or receptor), an anti-58297 antibody, a 58297 agonist or antagonist, a peptidomimetic of a 58297 agonist or antagonist, or other small molecule.

In one embodiment, the agent stimulates one or more 58297 activities. Examples of such stimulatory agents include active 58297 proteins and nucleic acid molecules encoding a 58297 protein or polypeptide, or a fragment thereof. In another embodiment, the agent inhibits one or more 58297 activities. Examples of such inhibitory agents include antisense 58297 nucleic acid molecules, anti-58297 antibodies, and 58297 inhibitors. These modulatory methods can be performed *in vitro* (e.g., by culturing the cell with the agent) or, alternatively, *in vivo* (e.g., by administering the agent to a subject), or *in situ.* As such, the present invention provides methods of treating an individual afflicted with a disease or disorder characterized by aberrant or unwanted expression or activity of a 58297 protein or nucleic acid molecule. In one embodiment, the method involves administering an agent (e.g., an agent identified by a screening assay described herein), or combination of agents that modulates (e.g., upregulates or downregulates) 58297 expression or activity. In another embodiment, the method involves administering a 58297 protein or nucleic acid molecule as therapy to compensate for reduced, aberrant, or undesirable 58297 expression or activity.

Stimulation of 58297 expression or activity is desirable in situations in which 58297 expression or activity is abnormally downregulated and/or in which increased 58297 expression or activity is likely to have a beneficial effect. Likewise, inhibition of 58297 expression or activity is desirable in situations in which 58297 expression or activity is abnormally upregulated and/or in which decreased 58297 expression or activity is likely to have a beneficial effect.

The 58297 molecules can act as novel diagnostic targets and therapeutic agents for controlling one or more of cellular proliferative and/or differentiative disorders, hormonal disorders, immune and inflammatory disorders, neurological disorders, cardiovascular disorders, blood vessel disorders, and platelet disorders, as described above, as well as disorders associated with bone metabolism, viral diseases, and pain and metabolic disorders.

Aberrant expression and/or activity of 58297 molecules may mediate disorders associated with bone metabolism. "Bone metabolism" refers to direct or indirect effects in the formation or degeneration of bone structures, e.g., bone formation, bone resorption, etc., which may ultimately affect the concentrations in serum of calcium and phosphate. This term also includes activities mediated by 58297 molecules effects in bone cells, e.g. osteoclasts and osteoblasts, that may in turn result in bone formation and degeneration. For example, 58297 molecules may support different activities of bone resorbing osteoclasts such as the stimulation of differentiation of monocytes and mononuclear phagocytes into osteoclasts. Accordingly, 58297 molecules that modulate the production of bone cells can influence bone formation and degeneration, and thus may be used to treat bone disorders. Examples of such disorders include, but are not limited to, osteoporosis, osteodystrophy, osteomalacia, rickets, osteitis fibrosa cystica, renal osteodystrophy, osteosclerosis, anti-convulsant treatment, osteopenia, fibrogenesis-imperfecta ossium, secondary hyperparathyrodism, hypoparathyroidism, hyperparathyroidism, cirrhosis, obstructive jaundice, drug induced metabolism, medullary carcinoma, chronic renal disease, rickets, sarcoidosis, glucocorticoid antagonism, malabsorption syndrome, steatorrhea, tropical sprue, idiopathic hypercalcemia and milk fever.

Additionally, 58297 molecules may play an important role in the etiology of certain viral diseases, including but not limited to, Hepatitis B, Hepatitis C and Herpes Simplex Virus (HSV). Modulators of 58297activity can be used to control viral diseases. The modulators can be used in the treatment and/or diagnosis of viral infected tissue or virus-associated tissue fibrosis, especially liver and liver fibrosis. Also, 58297 modulators can be used in the treatment and/or diagnosis of virus-associated carcinomas, especially hepatocellular cancers.

Additionally, 58297 may play an important role in the regulation of metabolism or pain disorders. Diseases of metabolic imbalance include, but are not limited to, obesity, anorexia nervosa, bullemia, cachexia, lipid disorders, and diabetes. Examples of pain disorders include, but are not limited to, pain response elicited during various forms of tissue injury, e.g., inflammation, infection, and ischemia, usually referred to as hyperalgesia (described in, for example, Fields, H.L., (1987) *Pain,* New York:McGraw-Hill); pain associated with muscoloskeletal disorders, e.g., joint pain; tooth pain; headaches; pain associated with surgery; http://164.195.100.11/netacgi/nph- Parser?Sect1=PTO2&Sect2=HITOFF&p=1&u=/netahtml/search-bool.html&r=3&f=G&1=50&co1=AND&d=curr&s1=millennium.ASNM.&s2=pain&OS=A N/millennium+AND+pain&RS=AN/ - h3http://164.195.100.11/netacgi/nph-Parser?Sect1=PTO2&Sect2=HITOFF&p=1&u=/netahtml/search-bool.html&r=3&f=G&1=50&co1=AND&d=curr&s1=millennium.ASNM.&s2=pain&OS=A N/millennium+AND+pain&RS=AN/ - h5pain related to irritable bowel syndrome; and chest http://164.195.100.11/netacgi/nph- Parser?Sect1=PTO2&Sect2=HITOFF&p=1&u=/netahtml/search-bool.html&r=3&f=G&l=50&co1=AND&d=curr&s1=millennium.ASNM.&s2=pain&OS=A N/millennium+AND+pain&RS=AN/ - h4http://164.195.100.11/netacgi/nph- Parser?Sect1=PTO2&Sect2=HITOFF&p=1&u=/netahtml/search-bool.html&r=3&f=G&l=50&co1=AND&d=curr&s1=millennium.ASNM.&s2=pain&OS=A N/millennium+AND+pain&RS=AN/ - h6pain.

### Pharmacogenomics

The 58297 molecules of the present invention, as well as agents, and modulators which have a stimulatory or inhibitory effect on a 58297 activity (e.g., 58297 gene expression) as identified by a screening assay described herein can be administered to individuals to treat (prophylactically or therapeutically) 58297 associated disorders (e.g., cellular proliferative and/or differentiative disorders, hormonal disorders, immune and inflammatory disorders, neurological disorders, cardiovascular disorders, blood vessel disorders, and platelet disorders) associated with aberrant or undesirable 58297 activity. In conjunction with such treatment, pharmacogenomics (i.e., the study of the relationship between an individual's genotype and that individual's response to a foreign compound or drug) may be considered. Differences in metabolism of therapeutics can lead to severe toxicity or therapeutic failure by altering the relation between dose and blood concentration of the pharmacologically active drug. Thus, a physician or clinician may consider applying knowledge obtained in relevant pharmacogenomics studies in determining whether to administer a 58297 molecule or 58297 modulator as well as tailoring the dosage and/or therapeutic regimen of treatment with a 58297 molecule or 58297 modulator.

Pharmacogenomics deals with clinically significant hereditary variations in the response to drugs due to altered drug disposition and abnormal action in affected persons. See, for example, Eichelbaum, M. et al. (1996) *Clin. Exp. Pharmacol. Physiol.* 23:983-985 and Linder, M.W. et al. (1997) *Clin. Chem.* 43:254-266. In general, two types of pharmacogenetic conditions can be differentiated. Genetic conditions transmitted as a single factor altering the way drugs act on the body (altered drug action) or genetic conditions transmitted as single factors altering the way the body acts on drugs (altered drug metabolism) . These pharmacogenetic conditions can occur either as rare genetic defects or as naturally-occurring polymorphisms. For example, glucose-6-phosphate dehydrogenase deficiency (G6PD) is a common inherited enzymopathy in which the main clinical complication is haemolysis after ingestion of oxidant drugs (anti-malarials, sulfonamides, analgesics, nitrofurans) and consumption of fava beans.

One pharmacogenomics approach to identifying genes that predict drug response, known as "a genome-wide association", relies primarily on a high-resolution map of the human genome consisting of already known gene-related markers (e.g., a "bi-allelic" gene marker map which consists of 60,000-100,000 polymorphic or variable sites on the human genome, each of which has two variants.) Such a high-resolution genetic map can be compared to a map of the genome of each of a statistically significant number of patients taking part in a Phase II/III drug trial to identify markers associated with a particular observed drug response or side effect. Alternatively, such a high resolution map can be generated from a combination of some ten-million known single nucleotide polymorphisms (SNPs) in the human genome. As used herein, a "SNP" is a common alteration that occurs in a single nucleotide base in a stretch of DNA. For example, a SNP may occur once per every 1000 bases of DNA. A SNP may be involved in a disease process, however, the vast majority may not be disease-associated. Given a genetic map based on the occurrence of such SNPs, individuals can be grouped into genetic categories depending on a particular pattern of SNPs in their individual genome. In such a manner, treatment regimens can be tailored to groups of genetically similar individuals, taking into account traits that may be common among such genetically similar individuals.

Alternatively, a method termed the "candidate gene approach", can be utilized to identify genes that predict drug response. According to this method, if a gene that encodes a drug's target is known (e.g., a 58297 protein of the present invention), all common variants of that gene can be fairly easily identified in the population and it can be determined if having one version of the gene versus another is associated with a particular drug response.

Alternatively, a method termed the "gene expression profiling", can be utilized to identify genes that predict drug response. For example, the gene expression of an animal dosed with a drug (e.g., a 58297 molecule or 58297 modulator of the present invention) can give an indication whether gene pathways related to toxicity have been turned on.

Information generated from more than one of the above pharmacogenomics approaches can be used to determine appropriate dosage and treatment regimens for prophylactic or therapeutic treatment of an individual. This knowledge, when applied to dosing or drug selection, can avoid adverse reactions or therapeutic failure and thus enhance therapeutic or prophylactic efficiency when treating a subject with a 58297 molecule or 58297 modulator, such as a modulator identified by one of the exemplary screening assays described herein.

The present invention further provides methods for identifying new agents, or combinations, that are based on identifying agents that modulate the activity of one or more of the gene products encoded by one or more of the 58297 genes of the present invention, wherein these products may be associated with resistance of the cells to a therapeutic agent. Specifically, the activity of the proteins encoded by the 58297 genes of the present invention can be used as a basis for identifying agents for overcoming agent resistance. By blocking the activity of one or more of the resistance proteins, target cells, e.g., human cells, will become sensitive to treatment with an agent that the unmodified target cells were resistant to.

Monitoring the influence of agents (e.g., drugs) on the expression or activity of a 58297 protein can be applied in clinical trials. For example, the effectiveness of an agent determined by a screening assay as described herein to increase 58297 gene expression or protein levels, or upregulate 58297 activity, can be monitored in clinical trials of subjects exhibiting decreased 58297 gene expression or protein levels, or downregulated 58297 activity. Alternatively, the effectiveness of an agent determined by a screening assay to decrease 58297 gene expression or protein levels, or downregulate 58297 activity, can be monitored in clinical trials of subjects exhibiting increased 58297 gene expression or protein levels, or upregulated 58297 activity. In such clinical trials, the expression or activity of a 58297 gene, and preferably, other genes that have been implicated in, for example, a 58297-associated disorder can be used as a "read out" or markers of the phenotype of a particular cell.

### Other Embodiments

In another aspect, the invention features a method of analyzing a plurality of capture probes. The method is useful, e.g., to analyze gene expression. The method includes: providing a two dimensional array having a plurality of addresses, each address of the plurality being positionally distinguishable from each other address of the plurality, and each address of the plurality having a unique capture probe, e.g., a nucleic acid or peptide sequence, wherein the capture probes are from a cell or subject which expresses 58297 or from a cell or subject in which a 58297 mediated response has been elicited; contacting the array with a 58297 nucleic acid (preferably purified), a 58297 polypeptide (preferably purified), or an anti-58297 antibody, and thereby evaluating the plurality of capture probes. Binding, e.g., in the case of a nucleic acid, hybridization with a capture probe at an address of the plurality, is detected, e.g., by a signal generated from a label attached to the 58297 nucleic acid, polypeptide, or antibody.

The capture probes can be a set of nucleic acids from a selected sample, e.g., a sample of nucleic acids derived from a control or non-stimulated tissue or cell.

The method can include contacting the 58297 nucleic acid, polypeptide, or antibody with a first array having a plurality of capture probes and a second array having a different plurality of capture probes. The results of each hybridization can be compared, e.g., to analyze differences in expression between a first and second sample. The first plurality of capture probes can be from a control sample, e.g., a wild type, normal, or non-diseased, non-stimulated, sample, e.g., a biological fluid, tissue, or cell sample. The second plurality of capture probes can be from an experimental sample, e.g., a mutant type, at risk, disease-state or disorder-state, or stimulated, sample, e.g., a biological fluid, tissue, or cell sample.

The plurality of capture probes can be a plurality of nucleic acid probes each of which specifically hybridizes, with an allele of 58297. Such methods can be used to diagnose a subject, e.g., to evaluate risk for a disease or disorder, to evaluate suitability of a selected treatment for a subject, to evaluate whether a subject has a disease or disorder.

The method can be used to detect SNPs, as described above.

In another aspect, the invention features, a method of analyzing 58297, e.g., analyzing structure, function, or relatedness to other nucleic acid or amino acid sequences. The method includes: providing a 58297 nucleic acid or amino acid sequence; comparing the 58297 sequence with one or more preferably a plurality of sequences from a collection of sequences, e.g., a nucleic acid or protein sequence database; to thereby analyze 58297.

The method can include evaluating the sequence identity between a 58297 sequence and a database sequence. The method can be performed by accessing the database at a second site, e.g., over the internet. Preferred databases include GenBank™ and SwissProt.

In another aspect, the invention features, a set of oligonucleotides, useful, e.g., for identifying SNP's, or identifying specific alleles of 58297. The set includes a plurality of oligonucleotides, each of which has a different nucleotide at an interrogation position, e.g., an SNP or the site of a mutation. In a preferred embodiment, the oligonucleotides of the plurality identical in sequence with one another (except for differences in length). The oligonucleotides can be provided with differential labels, such that an oligonucleotides which hybridizes to one allele provides a signal that is distinguishable from an oligonucleotides which hybridizes to a second allele.

The sequence of a 58297 molecules is provided in a variety of mediums to facilitate use thereof. A sequence can be provided as a manufacture, other than an isolated nucleic acid or amino acid molecule, which contains a 58297 molecule. Such a manufacture can provide a nucleotide or amino acid sequence, e.g., an open reading frame, in a form which allows examination of the manufacture using means not directly applicable to examining the nucleotide or amino acid sequences, or a subset thereof, as they exists in nature or in purified form.

A 58297 nucleotide or amino acid sequence can be recorded on computer readable media. As used herein, "computer readable media" refers to any medium that can be read and accessed directly by a computer. Such media include, but are not limited to: magnetic storage media, such as floppy discs, hard disc storage medium, and magnetic tape; optical storage media such as compact disc and CD-ROM; electrical storage media such as RAM, ROM, EPROM, EEPROM, and the like; and general hard disks and hybrids of these categories such as magnetic/optical storage media. The medium is adapted or configured for having thereon 58297 sequence information of the present invention.

As used herein, the term "electronic apparatus" is intended to include any suitable computing or processing apparatus of other device configured or adapted for storing data or information. Examples of electronic apparatus suitable for use with the present invention include stand-alone computing apparatus; networks, including a local area network (LAN), a wide area network (WAN) Internet, Intranet, and Extranet; electronic appliances such as personal digital assistants (PDAs), cellular phones, pagers, and the like; and local and distributed processing systems.

As used herein, "recorded" refers to a process for storing or encoding information on the electronic apparatus readable medium. Those skilled in the art can readily adopt any of the presently known methods for recording information on known media to generate manufactures comprising the 58297 sequence information.

A variety of data storage structures are available to a skilled artisan for creating a computer readable medium having recorded thereon a 58297 nucleotide or amino acid sequence of the present invention. The choice of the data storage structure will generally be based on the means chosen to access the stored information. In addition, a variety of data processor programs and formats can be used to store the nucleotide sequence information of the present invention on computer readable medium. The sequence information can be represented in a word processing text file, formatted in commercially-available software such as WordPerfect and Microsoft Word, or represented in the form of an ASCII file, stored in a database application, such as DB2, Sybase, Oracle, or the like. The skilled artisan can readily adapt any number of data processor structuring formats (*e.g*., text file or database) in order to obtain computer readable medium having recorded thereon the nucleotide sequence information of the present invention.

By providing the 58297 nucleotide or amino acid sequences of the invention in computer readable form, the skilled artisan can routinely access the sequence information for a variety of purposes. For example, one skilled in the art can use the nucleotide or amino acid sequences of the invention in computer readable form to compare a target sequence or target structural motif with the sequence information stored within the data storage means. A search is used to identify fragments or regions of the sequences of the invention which match a particular target sequence or target motif.

The present invention therefore provides a medium for holding instructions for performing a method for determining whether a subject has a 58297-associated disease or disorder or a pre-disposition to a 58297-associated disease or disorder, wherein the method comprises the steps of determining 58297 sequence information associated with the subject and based on the 58297 sequence information, determining whether the subject has a 58297-associated disease or disorder and/or recommending a particular treatment for the disease, disorder, or pre-disease condition.

The present invention further provides in an electronic system and/or in a network, a method for determining whether a subject has a 58297-associated disease or disorder or a pre-disposition to a disease associated with 58297, wherein the method comprises the steps of determining 58297 sequence information associated with the subject, and based on the 58297 sequence information, determining whether the subject has a 58297-associated disease or disorder or a pre-disposition to a 58297-associated disease or disorder, and/or recommending a particular treatment for the disease, disorder, or pre-disease condition. The method may further comprise the step of receiving phenotypic information associated with the subject and/or acquiring from a network phenotypic information associated with the subject.

The present invention also provides in a network, a method for determining whether a subject has a 58297-associated disease or disorder or a pre-disposition to a 58297-associated disease or disorder, said method comprising the steps of receiving 58297 sequence information from the subject and/or information related thereto, receiving phenotypic information associated with the subject, acquiring information from the network corresponding to 58297 and/or corresponding to a 58297-associated disease or disorder, and based on one or more of the phenotypic information, the 58297 information (e.g., sequence information and/or information related thereto), and the acquired information, determining whether the subject has a 58297-associated disease or disorder or a pre-disposition to a 58297-associated disease or disorder. The method may further comprise the step of recommending a particular treatment for the disease, disorder, or pre-disease condition.

The present invention also provides a business method for determining whether a subject has a 58297-associated disease or disorder or a pre-disposition to a 58297-associated disease or disorder, said method comprising the steps of receiving information related to 58297 (e.g., sequence information and/or information related thereto), receiving phenotypic information associated with the subject, acquiring information from the network related to 58297 and/or related to a 58297-associated disease or disorder, and based on one or more of the phenotypic information, the 58297 information, and the acquired information, determining whether the subject has a 58297-associated disease or disorder or a pre-disposition to a 58297-associated disease or disorder. The method may further comprise the step of recommending a particular treatment for the disease, disorder, or pre-disease condition.

The invention also includes an array comprising a 58297 sequence of the present invention. The array can be used to assay expression of one or more genes in the array. In one embodiment, the array can be used to assay gene expression in a tissue to ascertain tissue specificity of genes in the array. In this manner, up to about 7600 genes can be simultaneously assayed for expression, one of which can be 58297. This allows a profile to be developed showing a battery of genes specifically expressed in one or more tissues.

In addition to such qualitative information, the invention allows the quantitation of gene expression. Thus, not only tissue specificity, but also the level of expression of a battery of genes in the tissue if ascertainable. Thus, genes can be grouped on the basis of their tissue expression *per se* and level of expression in that tissue. This is useful, for example, in ascertaining the relationship of gene expression in that tissue. Thus, one tissue can be perturbed and the effect on gene expression in a second tissue can be determined. In this context, the effect of one cell type on another cell type in response to a biological stimulus can be determined. In this context, the effect of one cell type on another cell type in response to a biological stimulus can be determined. Such a determination is useful, for example, to know the effect of cell-cell interaction at the level of gene expression. If an agent is administered therapeutically to treat one cell type but has an undesirable effect on another cell type, the invention provides an assay to determine the molecular basis of the undesirable effect and thus provides the opportunity to co-administer a counteracting agent or otherwise treat the undesired effect. Similarly, even within a single cell type, undesirable biological effects can be determined at the molecular level. Thus, the effects of an agent on expression of other than the target gene can be ascertained and counteracted.

In another embodiment, the array can be used to monitor the time course of expression of one or more genes in the array. This can occur in various biological contexts, as disclosed herein, for example development of a 58297-associated disease or disorder, progression of 58297-associated disease or disorder, and processes, such a cellular transformation associated with the 58297-associated disease or disorder.

The array is also useful for ascertaining the effect of the expression of a gene on the expression of other genes in the same cell or in different cells (*e.g*., ascertaining the effect of 58297 expression on the expression of other genes). This provides, for example, for a selection of alternate molecular targets for therapeutic intervention if the ultimate or downstream target cannot be regulated.

The array is also useful for ascertaining differential expression patterns of one or more genes in normal and abnormal cells. This provides a battery of genes (*e.g*., including 58297) that could serve as a molecular target for diagnosis or therapeutic intervention.

As used herein, a "target sequence" can be any DNA or amino acid sequence of six or more nucleotides or two or more amino acids. A skilled artisan can readily recognize that the longer a target sequence is, the less likely a target sequence will be present as a random occurrence in the database. Typical sequence lengths of a target sequence are from about 10 to 100 amino acids or from about 30 to 300 nucleotide residues. However, it is well recognized that commercially important fragments, such as sequence fragments involved in gene expression and protein processing, may be of shorter length.

Computer software is publicly available which allows a skilled artisan to access sequence information provided in a computer readable medium for analysis and comparison to other sequences. A variety of known algorithms are disclosed publicly and a variety of commercially available software for conducting search means are and can be used in the computer-based systems of the present invention. Examples of such software include, but are not limited to, MacPattern (EMBL), BLASTN and BLASTX (NCBI).

Thus, the invention features a method of making a computer readable record of a sequence of a 58297 sequence which includes recording the sequence on a computer readable matrix. In a preferred embodiment the record includes one or more of the following: identification of an ORF; identification of a domain, region, or site; identification of the start of transcription; identification of the transcription terminator; the full length amino acid sequence of the protein, or a mature form thereof; the 5' end of the translated region.

In another aspect, the invention features, a method of analyzing a sequence. The method includes: providing a 58297 sequence, or record, in computer readable form; comparing a second sequence to the 58297 sequence; thereby analyzing a sequence. Comparison can include comparing to sequences for sequence identity or determining if one sequence is included within the other, e.g., determining if the 58297 sequence includes a sequence being compared. In a preferred embodiment the 58297 or second sequence is stored on a first computer, e.g., at a first site and the comparison is performed, read, or recorded on a second computer, e.g., at a second site. E.g., the 58297 or second sequence can be stored in a public or proprietary database in one computer, and the results of the comparison performed, read, or recorded on a second computer. In a preferred embodiment the record includes one or more of the following: identification of an ORF; identification of a domain, region, or site; identification of the start of transcription; identification of the transcription terminator; the full length amino acid sequence of the protein, or a mature form thereof; the 5' end of the translated region.

The contents of all references, patents and published patent applications cited throughout this application are incorporated herein by reference.

### Equivalents

Those skilled in the art will recognize, or be able to ascertain using no more than routine experimentation, many equivalents to the specific embodiments of the invention described herein. Such equivalents are intended to be encompassed by the following claims.

## Claims

1. An isolated nucleic acid molecule selected from the group consisting of:
a. a nucleic acid molecule comprising a nucleotide sequence which is at least 80% identical to the nucleotide sequence of SEQ ID NO:1 or SEQ ID NO:3;
b. a nucleic acid molecule comprising a fragment of at least 520 nucleotides of the nucleotide sequence of SEQ ID NO:1 or SEQ ID NO:3;
c. a nucleic acid molecule which encodes a polypeptide comprising the amino acid sequence of SEQ ID NO:2;
d. a nucleic acid molecule which encodes a fragment of a polypeptide comprising the amino acid sequence of SEQ ID NO:2, wherein the fragment comprises at least 100 contiguous amino acids of SEQ ID NO:2; and
e. a nucleic acid molecule which encodes a naturally occurring allelic variant of a polypeptide comprising the amino acid sequence of SEQ ID NO:2, wherein the nucleic acid molecule hybridizes to a nucleic acid molecule comprising SEQ ID NO:1, 3, or a complement thereof, under stringent conditions.

2. The isolated nucleic acid molecule of claim 1, which is at least 90% identical to the nucleotide sequence of SEQ ID NO:1 or SEQ ID NO:3, the isolated nucleic acid molecule of claim 1, which is at least 95% identical to the nucleotide sequence of SEQ ID NO:1 or SEQ ID NO:3.

3. The isolated nucleic acid molecule of claim 1, which encodes a fragment of a polypeptide comprising the amino acid sequence of SEQ ID NO:2, wherein the fragment comprises at least 200 contiguous amino acids of SEQ ID NO:2, or the isolated nucleic acid molecule of claim 1, which encodes a fragment of a polypeptide comprising the amino acid sequence of SEQ ID NO:2, wherein the fragment comprises at least 300 contiguous amino acids of SEQ ID NO:2.

4. The isolated nucleic acid molecule of claim 1, which is selected from the group consisting of:
a. a nucleic acid comprising the nucleotide sequence of SEQ ID NO:1 or SEQ ID NO:3; and
b. a nucleic acid molecule which encodes a polypeptide comprising the amino acid sequence of SEQ ID NO:2.

5. The nucleic acid molecule of claim 1 further comprising vector nucleic acid sequences, or the nucleic acid molecule of claim 1 further comprising nucleic acid sequences encoding a heterologous polypeptide.

6. A host cell which contains the nucleic acid molecule of claim 1.

7. The host cell of claim 6 which is a mammalian host cell.

8. A non-human mammalian host cell containing the nucleic acid molecule of claim 1.

9. An isolated polypeptide selected from the group consisting of:
a. a polypeptide which is encoded by a nucleic acid molecule comprising a nucleotide sequence which is at least 80% identical to a nucleic acid comprising the nucleotide sequence of SEQ ID NO:1 or SEQ ID NO:3, or a complement thereof;
b. a naturally occurring allelic variant of a polypeptide comprising the amino acid sequence of SEQ ID NO:2, wherein the polypeptide is encoded by a nucleic acid molecule which hybridizes to a nucleic acid molecule comprising SEQ ID NO:1 or SEQ ID NO:3; and
c. a fragment of a polypeptide comprising the amino acid sequence of SEQ ID NO:2, wherein the fragment comprises at least 100 contiguous amino acids of SEQ ID NO:2.

10. The isolated polypeptide of claim 9, comprising a fragment which comprises at least 200 contiguous amino acids of SEQ ID NO:2, or the isolated polypeptide of claim 9, comprising a fragment which comprises at least 300 contiguous amino acids of SEQ ID NO:2.

11. The isolated polypeptide of claim 9 comprising a polypeptide which is encoded by a nucleic acid molecule comprising a nucleotide sequence which is at least 90% identical to a nucleic acid comprising the nucleotide sequence of SEQ ID NO:1 or SEQ ID NO:3, or a complement thereof, or the isolated polypeptide of claim 9, comprising a polypeptide which is encoded by a nucleic acid molecule comprising a nucleotide sequence which is at least 95% identical to a nucleic acid comprising the nucleotide sequence of SEQ ID NO:1 or SEQ ID NO:3, or a complement thereof.

12. The isolated polypeptide of claim 9 comprising the amino acid sequence of SEQ ID NO:2, or the polypeptide of claim 9 further comprising heterologous amino acid sequences.

13. An antibody which selectively binds to a polypeptide of claim 9.

14. The antibody of claim 13, which is a monoclonal antibody.

15. The antibody of claim 14, comprising an immunologically active portion selected from the group consisting of:
a. an scFV fragment;
b. a dcFV fragment;
c. an Fab fragment; and
d. an F(ab')₂ fragment.

16. The antibody of claim 14, wherein the antibody is selected from the group consisting of:
a. a chimeric antibody;
b. a humanized antibody;
c. a human antibody;
d. a non-human antibody; and
e. a single chain antibody.

17. A method for producing a polypeptide selected from the group consisting of:
a. a polypeptide comprising the amino acid sequence of SEQ ID NO:2;
b. a polypeptide comprising a fragment of the amino acid sequence of SEQ ID NO:2, wherein the fragment comprises at least 100 contiguous amino acids of SEQ ID NO:2; and
c. a naturally occurring allelic variant of a polypeptide comprising the amino acid sequence of SEQ ID NO:2, wherein the polypeptide is encoded by a nucleic acid molecule which hybridizes to a nucleic acid molecule comprising SEQ ID NO: SEQ ID NO:3, or a complement thereof under stringent conditions;
comprising culturing the host cell of claim 6 under conditions in which the nucleic acid molecule is expressed.

18. A method for detecting the presence of a polypeptide of claim 9 in a sample, comprising:
contacting the sample with a compound which selectively binds to a polypeptide of claim 9; and
determining whether the compound binds to the polypeptide in the sample.

19. The method of claim 18, wherein the compound which binds to the polypeptide is an antibody.

20. A kit comprising a compound which selectively binds to a polypeptide of claim 9 and instructions for use, or a kit comprising a compound which selectively hybridizes to a nucleic acid molecule of claim 1 and instructions for use.

21. A method for detecting the presence of a nucleic acid molecule of claim 1 in a sample, comprising the steps of:
contacting the sample with a nucleic acid probe or primer which selectively hybridizes to the nucleic acid molecule; and
determining whether the nucleic acid probe or primer binds to a nucleic acid molecule in the sample.

22. The method of claim 21, wherein the sample comprises mRNA molecules and is contacted with a nucleic acid probe.

23. A method for identifying a compound which binds to a polypeptide of claim 9 comprising the steps of:
contacting a polypeptide, or a cell expressing a polypeptide of claim 9 with a test compound; and
determining whether the polypeptide binds to the test compound.

24. The method of claim 23, wherein the binding of the test compound to the polypeptide is detected by a method selected from the group consisting of:
a. detection of binding by direct detecting of test compound/polypeptide binding;
b. detection of binding using a competition binding assay; and
c. detection of binding using an assay for 58297-mediated signal transduction.

25. A method for modulating the activity of a polypeptide of claim 9 comprising contacting a polypeptide or a cell expressing a polypeptide of claim 9 with a compound which binds to the polypeptide in a sufficient concentration to modulate the activity of the polypeptide.

26. A method for identifying a compound which modulates the activity of a polypeptide of claim 9, comprising:
contacting a polypeptide of claim 9 with a test compound; and
determining the effect of the test compound on the activity of the polypeptide to thereby identify a compound which modulates the activity of the polypeptide.
